# EUROPEAN PATENT APPLICATION

(11) **EP 1 426 050 A1**
(43) Date of publication of application: **09.06.2004**
(21) Application number: 02765344.3
(22) Date of filing: 22.08.2002
(51) Int. Cl.: A61K 31/5025, A61K 45/00, C07D 487/04, A61P 1/00, A61P 1/04, A61P 1/18, A61P 5/14, A61P 7/00, A61P 7/02, A61P 9/00, A61P 9/10, A61P 11/00, A61P 11/02, A61P 11/06, A61P 13/12, A61P 15/00, A61P 17/00, A61P 17/02, A61P 17/04, A61P 17/06, A61P 19/02

(54) **JNK INHIBITORS**

(30) Priority: 23.08.2001 JP 2001253602
(71) Applicant: Takeda Chemical Industries, Ltd., Osaka-shi, Osaka 541-0045 (JP)
(72) Inventor: NAGAYA, Hideaki, Toyonaka-shi, Osaka 561-0801 (JP); KAWANO, Yasuhiko, Suita-shi, Osaka 565-0806 (JP); KAMEI, Takayuki, Mino-shi, Osaka 562-0044 (JP)
(74) Representative: von Kreisler, Alek, Dipl.-Chem.
(86) International application number: PCT/JP2002/008465
(87) International publication number: WO 2003/018020

(57) **Abstract**

A c-Jun N-terminal kinase activation inhibitor which comprises a compound represented by the formula: 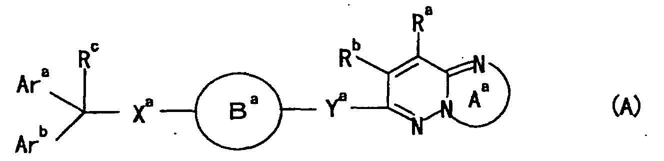 wherein each of Ar^{a} and Ar^{b} is an aromatic group optionally having substituents, Ar^{a} and Ar^{b} optionally form a condensed cyclic group together with the adjacent carbon atom; ring B^{a} is a nitrogen-containing heterocycle optionally having substituents; X^{a} and Y^{a} are the same or different and each is (1) a bond, (2) an oxygen atom, (3) S(O)p (wherein p is an integer of 0 to 2) , (4) NR^{d} (wherein R^{d} is a hydrogen atom or a lower alkyl group) or (5) a divalent linear lower hydrocarbon group optionally having substituents and containing 1 to 3 hetero atom(s); ring A^{a} is a 5-membered ring optionally having substituents; R^{a} and R^{b} are the same or different and each is (1) a hydrogen atom, (2) a halogen atom, (3) a hydrocarbon group optionally having substituents, (4) an acyl group or (5) a hydroxy group optionally having a substituent; R^{c} is (1) a hydrogen atom, (2) a hydroxy group optionally substituted by a lower alkyl group or (3) a carboxyl group or a salt thereof, or a prodrug thereof.

## Description

### TECHNICAL FIELD

The present invention relates to an excellent c-Jun N-terminal kinase (hereinafter abbreviated as JNK) activation inhibitor and TNF (tumor necrosis factor)-α inhibitor, etc.

### BACKGROUND ART

JNK is a member of Mitogen Activated Protein Kinase (MAPK) existing in the signal transduction system in which outer stimulation is transmitted into nuclei. It is known that JNK increases transcription activity of AP-1 lower gene by phosphorylating N-terminal of c-Jun as AP-1 transcription regulator (EMBO J. 15, 2760-2770(1996); Biochemica et Biophysica Acta, 1333, F85 1997). JNK activation inhibitor is supposed to inhibit the inflammation and expression of immune factor depending on AP-1, therefore, it can be used as a therapeutic drug for inflammatory disease such as rheumatoid arthritis and nervous degenerative disease (Mol. Cell. Biol. 1997, 17, 6274; J. Neurosci., 1998, 18, 104).

And, it is reported that c-Jun is concerned in muscle cellular apoptosis in ischemic and/or reperfusion condition and that JNK can be used as a drug for treating cardiovascular disease such as myocardial infarction and heart failure (Circ. Res., 82, 166 (1998)).

Examples of the compound having the above-mentioned JNK inhibitory activity are hydroxyindole derivatives described in WO 00/64872 and uracil derivatives described in WO 00/75118.

TNF-α is supposed to play an important role in several diseases. For example, the production of TNF-α is promoted in rheumatoid arthritis, which is an inflammatory disease, and this is considered to destroy the joint tissues.

On the other hand, many condensed pyridazine derivatives are disclosed as follows. USP 3,915,968 discloses a compound represented by the formula: 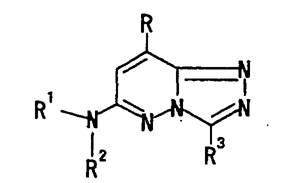 wherein R and R³ independently represent a hydrogen atom or a lower alkyl group (at least one of R and R³ is a lower alkyl group); R¹ and R² represent, taken together with the adjacent nitrogen atom, a heterocyclic group selected from the group consisting of pyrrolidine, piperidine, piperazine and morpholine; or a salt thereof. USP 4,136,182 discloses that a compound represented by the formula: 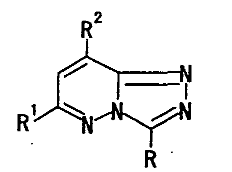 wherein R represents a hydrogen atom, a phenyl group or a lower alkylcarbonylamino group; R¹ represents morpholino or piperidino; R² represents a hydrogen atom or a lower alkyl group (at least one of R and R² is a group other than a hydrogen atom; when R is a phenyl group, R¹ is morpholino and R² is a lower alkyl group); or a salt thereof, is useful as a bronchodilator for mitigating bronchial spasms.

Also, Japanese Patent Unexamined Publication JP-A-H06-279447 discloses that a compound represented by the formula: 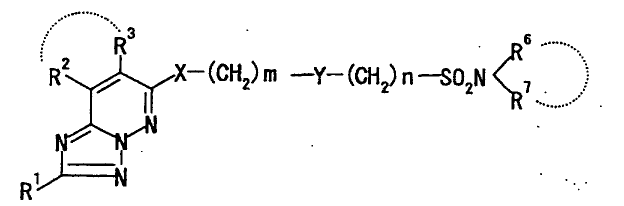 wherein R¹ represents a hydrogen atom, a lower alkyl group that may be substituted, or a halogen atom; R² and R³ independently represent a hydrogen atom or a lower alkyl group which may be substituted, or may form a 5- to 7-membered ring with the adjacent -C=C-; X represents an oxygen atom or S(O)ₚ (p represents an integer of 0 to 2); Y represents a group represented by the formula: 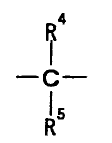 (R⁴ and R⁵ independently represent a hydrogen atom or a lower alkyl group which may be substituted) or a divalent group derived from a 3- to 7-membered homocycle or heterocycle which may be substituted; R⁶ and R⁷ independently represent a hydrogen atom, a lower alkyl group which may be substituted, a Cycloalkyl group which may be substituted or an aryl group that may be substituted, or may form a nitrogen-containing heterocyclic group which may be substituted, with the adjacent nitrogen atom; m represents an integer from 0 to 4, and n represents an integer from 0 to 4; or a salt thereof; and, as an example synthetic product, a compound of the formula: 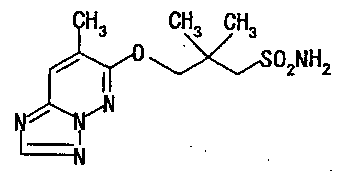 exhibits anti-asthmatic, anti-PAF, anti-inflammatory and anti-allergic activities.

Furthermore, Japanese Patent Unexamined Publication No.. JP-A-H06-279446 describes a compound represented by the formula: 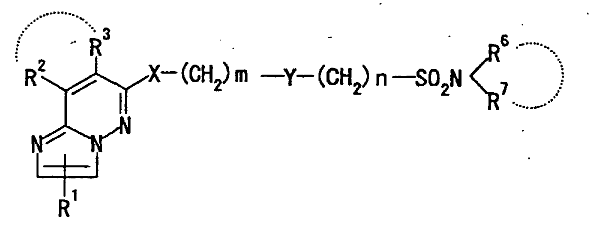 wherein R¹ represents a hydrogen atom, a lower alkyl group which may be substituted, or a halogen atom; R² and R³ independently represent a hydrogen atom or a lower alkyl group which may be substituted (provided that either of R² and R³ is a hydrogen atom, the other represents a lower alkyl group which may be substituted), or may form a 5- to 7-membered ring taken together with the adjacent -C=C-; X represents an oxygen atom or S(O)ₚ (p represents an integer of 0 to 2); Y represents a group represented by the formula: 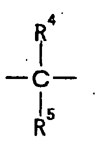 (R⁴ and R⁵ independently represent a hydrogen atom or a lower alkyl group which may be substituted) or a divalent group derived from a 3- to 7-membered homocycle or heterocycle which may be substituted; R⁶ and R⁷ independently represent a hydrogen atom, a lower alkyl group which may be substituted, a cycloalkyl group which may be substituted, or an aryl group which may be substituted, or may form, taken together with the adjacent nitrogen atom, a nitrogen-containing heterocyclic group which may be substituted; m represents an integer from 0 to 4, and n represents an integer from 0 to 4; or a salt thereof; and discloses that these compounds possess anti-allergic, anti-inflammatory and anti-PAF (platelet activating factor) activities to suppress bronchial spasms and bronchial contraction, therefore could be utilized as effective anti-asthmatic agents.

And, EP 128536 discloses anti-bacterial compounds represented by the formula: 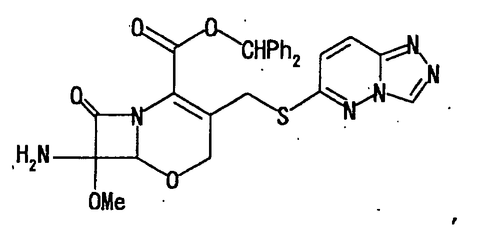 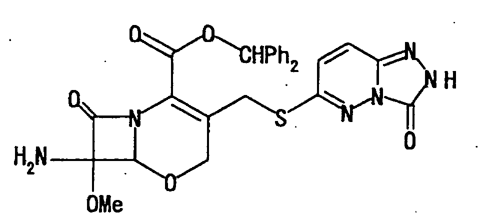 and so on, and USP 4,499,088 discloses anti-bacterial compounds represented by the formula: 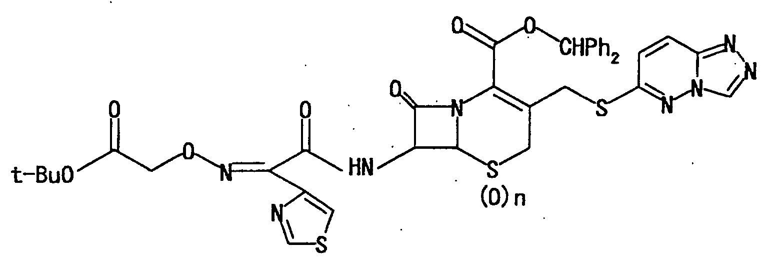 and so on.

JNK shows several physiological activites by phosphorylating c-Jun. And the development of JNK activation inhibitor having excellent condition in effect, duration and safety is desired for a drug for preventing and treating several diseases derived from excess activation of c-Jun (e.g., rheumatoid arthritis, cardiac ischemia, cerebral ischemia)

And the development of TNF-α inhibitor having excellent property as a drug which is excellent in prophylaxis and treatment effect for inflammatory diseases without side-effect is desired.

### DISCLOSURE OF THE INVENTION

In order to solve the aforementioned problems, the present inventors intensively studied, found that a compound, owing to its unique chemical structure characterized by the presence of nitrogen-containing heterocycle via a spacer from the condensed pyridazine skeleton represented by the formula 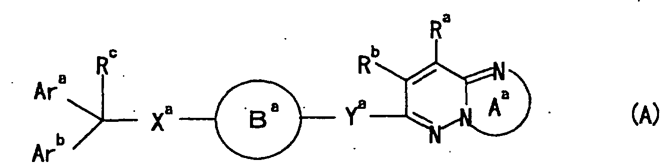 wherein each of Ar^{a} and Ar^{b} is an aromatic group optionally having substituents, Ar^{a} and Ar^{b} optionally form a condensed cyclic group together with the adjacent carbon atom; ring B^{a} is a nitrogen-containing heterocycle optionally having substituents; X^{a} and Y^{a} are the same or different and each is (1) a bond, (2) an oxygen atom, (3) S(O)ₚ (wherein p is an integer of 0 to 2), (4) NR^{d} (wherein R^{d} is a hydrogen atom or a lower alkyl group) or (5) a divalent linear lower hydrocarbon group optionally having substituents and containing 1 to 3 hetero atom(s); ring A^{a} is a 5-membered ring optionally having substituents; R^{a} and R^{b} are the same or different and each is (1) a hydrogen atom, (2) a halogen atom, (3) a hydrocarbon group optionally having substituents, (4) an acyl group or (5) a hydroxy group optionally having a substituent; R^{c} is (1) a hydrogen atom, (2) a hydroxy group optionally substituted by a lower alkyl group or (3) a carboxyl group or a salt thereof, or a prodrug thereof;
especially a compound, owing to its unique chemical structure characterized by the presence of nitrogen-containing heterocycle via a spacer from 6-position of [1,2,4]triazolo[1,5-b]pyridazine skeleton or imidazo[1,2-b]pyridazine skeleton represented by the formula: 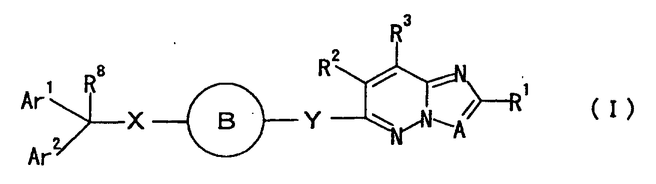 wherein each of Ar¹ and Ar² is an aromatic group optionally having substituents, Ar¹ and Ar² optionally form a condensed cyclic group together with the adjacent carbon atom; ring B is a nitrogen-containing heterocycle optionally having substituents; X and Y are the same or different and each is (1) a bond, (2) an oxygen atom, (3) S(O)ₚ (wherein p is an integer of 0 to 2), (4) NR⁴ (wherein R⁴ is a hydrogen atom or a lower alkyl group) or (5) a divalent linear lower hydrocarbon group optionally having substituents and containing 1 to 3 hetero atom(s); A is a nitrogen atom or CR⁷ (wherein R⁷ is a hydrogen atom, a halogen atom, a hydrocarbon group optionally having substituents, an acyl group or a hydroxy group optionally having a substituent); R¹, R² and R³ are the same or different and each is a hydrogen atom, a halogen atom, a hydrocarbon group optionally having substituents, an acyl group or a hydroxy group optionally having a substituent, R⁸ is a hydrogen atom, a hydroxy group optionally substituted by a lower alkyl group or a carboxyl group or a salt thereof, or a prodrug thereof exhibits unexpectedly excellent JNK activation inhibitory activity, TNF-α inhibitory activity, etc., and the drug comprising the compound is excellent in the stability as a pharmaceutical and enough to be used as a pharmaceutical. The inventors conducted further investigations based on these findings, and developed the present invention.

That is, the present invention relates to:
[1] a c-Jun N-terminal kinase activation inhibitor which comprises a compound represented by the formula: 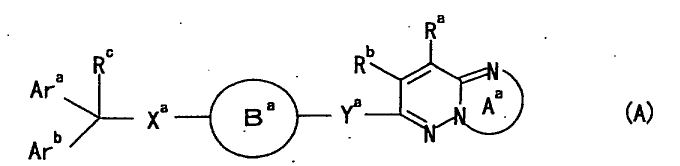 wherein each of Ar^{a} and Ar^{b} is an aromatic group optionally having substituents, Ar^{a} and Ar^{b} optionally form a condensed cyclic group together with the adjacent carbon atom; ring B^{a} is a nitrogen-containing heterocycle optionally having substituents; X^{a} and Y^{a} are the same or different and each is (1) a bond, (2) an oxygen atom, (3) S(O)ₚ (wherein p is an integer of 0 to 2), (4) NR^{d} (wherein R^{d} is a hydrogen atom or a lower alkyl group) or (5) a divalent linear lower hydrocarbon group optionally having substituents and containing 1 to 3 hetero atom(s); ring A^{a} is a 5-membered ring optionally having substituents; R^{a} and R^{b} are the same or different and each is (1) a hydrogen atom, (2) a halogen atom, (3) a hydrocarbon group optionally having substituents, (4) an acyl group or (5) a hydroxy group optionally having a substituent; R^{c} is (1) a hydrogen atom, (2) a hydroxy group optionally substituted by a lower alkyl group or (3) a carboxyl group or a salt thereof, or a prodrug thereof;
[2] a TNF-α inhibitor which comprises a compound represented by the formula: 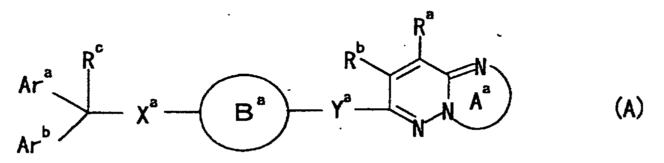 wherein each of Ar^{a} and Ar^{b} is an aromatic group optionally having substituents, Ar^{a} and Ar^{b} optionally form a condensed cyclic group together with the adjacent carbon atom; ring B^{a} is a nitrogen-containing heterocycle optionally having substituents; X^{a} and Y^{a} are the same or different and each is (1) a bond, (2) an oxygen atom, (3) S(O)ₚ (wherein p is an integer of 0 to 2), (4) NR^{d} (wherein R^{d} is a hydrogen atom or a lower alkyl group) or (5) a divalent linear lower hydrocarbon group optionally having substituents and containing 1 to 3 hetero atom(s); ring A^{a} is a 5-membered ring optionally having substituents; R^{a} and R^{b} are the same or different and each is (1) a hydrogen atom, (2) a halogen atom, (3) a hydrocarbon group optionally having substituents, (4) an acyl group or (5) a hydroxy group optionally having a substituent; R^{c} is (1) a hydrogen atom, (2) a hydroxy group optionally substituted by a lower alkyl group or (3) a carboxyl group or a salt thereof, or a prodrug thereof;
[3] a c-Jun N-terminal kinase activation inhibitor which comprises a compound represented by the formula: 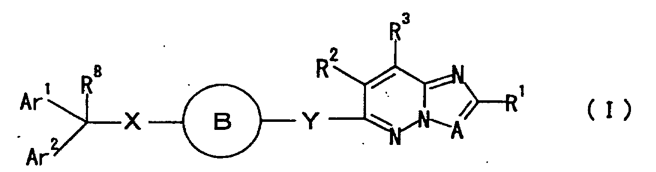 wherein each of Ar¹ and Ar² is an aromatic group optionally having substituents, Ar¹ and Ar² optionally form a condensed cyclic group together with the adjacent carbon atom; ring B is a nitrogen-containing heterocycle optionally having substituents; X and Y are the same or different and each is (1) a bond, (2) an oxygen atom, (3) S(O)ₚ (wherein p is an integer of 0 to 2), (4) NR⁴ (wherein R⁴ is a hydrogen atom or a lower alkyl group) or (5) a divalent linear lower hydrocarbon group optionally having substituents and containing 1 to 3 hetero atom(s); A is (1) a nitrogen atom or (2) CR⁷ (wherein R⁷ is a hydrogen atom, a halogen atom, a hydrocarbon group optionally having substituents, an acyl group or a hydroxy group optionally having a substituent); R¹, R² and R³ are the same or different and each is (1) a hydrogen atom, (2) a halogen atom, (3) a hydrocarbon group optionally having substituents, (4) an acyl group or (5) a hydroxy group optionally having a substituent, R⁸ is (1) a hydrogen atom, (2) a hydroxy group optionally substituted by a lower alkyl group or (3) a carboxyl group or a salt thereof, or a prodrug thereof;
[4] a TNF-α inhibitor which comprises a compound represented by the formula: 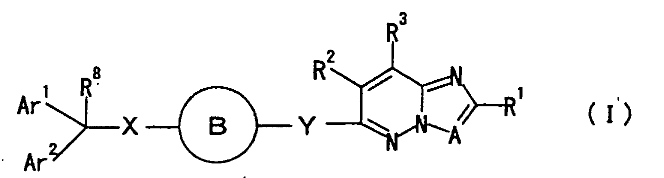 wherein each of Ar¹ and Ar² is an aromatic group optionally having substituents, Ar¹ and Ar² optionally form a condensed cyclic group together with the adjacent carbon atom; ring B is a nitrogen-containing heterocycle optionally having substituents; X and Y are the same or different and each is (1) a bond, (2) an oxygen atom, (3) S(O)ₚ (wherein p is an integer of 0 to 2), (4) NR⁴ (wherein R⁴ is a hydrogen atom or a lower alkyl group) or (5) a divalent linear lower hydrocarbon group optionally having substituents and containing 1 to 3 hetero atom(s); A is (1) a nitrogen atom or (2) CR⁷ (wherein R⁷ is a hydrogen atom, a halogen atom, a hydrocarbon group optionally having substituents, an acyl group or a hydroxy group optionally having a substituent); R¹, R² and R³ are the same or different and each is (1) a hydrogen atom, (2) a halogen atom, (3) a hydrocarbon group optionally having substituents, (4) an acyl group or (5) a hydroxy group optionally having a substituent, R⁸ is (1) a hydrogen atom, (2) a hydroxy group optionally substituted by a lower alkyl group or (3) a carboxyl group or a salt thereof, or a prodrug thereof;
[5] the inhibitor described in the above [3] or [4], wherein each of Ar¹ and Ar² is (1) a C₆₋₁₄ aromatic hydrocarbon group, (2) a 5 to 8 membered aromatic heterocyclic group containing 1 to 4 hetero atoms selected from a nitrogen atom, a sulfur atom and an oxygen atom other than carbon atoms or (3) a monovalent group obtained by removing one optional hydrogen atom from a ring formed by condensation of said aromatic heterocyclic ring with the C₆₋₁₄ aromatic hydrocarbon ring, wherein the C₆₋₁₄ aromatic hydrocarbon group, the 5 to 8 membered aromatic heterocyclic group and the monovalent group may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy and (xxviii) C₇₋₁₆ aralkyloxy; and Ar¹ and Ar² may form a condensed cyclic group with the adjacent carbon atom represented by the formula: 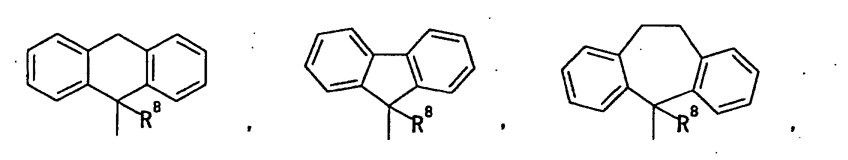 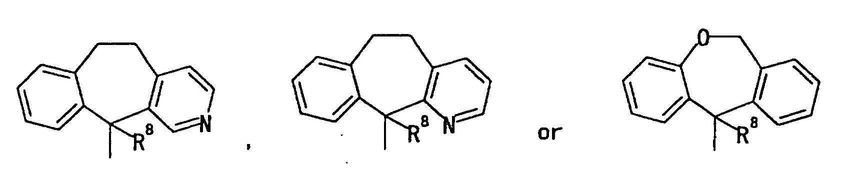 wherein R⁸ is a hydrogen atom, a hydroxy group which may be substituted by C₁₋₆ alkyl or a carboxyl group, and the condensed cyclic group may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo;
the ring B is a 3 to 13 membered nitrogen-containing heterocycle containing at least one nitrogen atom which may contain 1 to 3 hetero atoms selected from a nitrogen atom, an oxygen atom and a sulfur atom, and the 3 to 13 membered nitrogen-containing heterocycle may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo;
X and Y are same or different (1) a bond, (2) an oxygen atom, (3) S(O)ₚ wherein p is an integer of 0 to 2, (4) NR⁴ wherein R⁴ is a hydrogen atom or a linear or branched C₁₋₆ alkyl group or (5) a bivalent linear C₁₋₆ hydrocarbon group which may contain 1 to 3 hetero atoms selected from an oxygen atom, NR^{4'} (wherein R^{4'} is a hydrogen atom or a linear or branched C₁₋₆ alkyl group) and a sulfur atom, and the bivalent linear C₁₋₆ hydrocarbon group may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo;
A is a nitrogen atom or CR⁷ wherein R⁷ is
(1) a hydrogen atom,
(2) a halogen atom,
(3) a C₁₋₆ alkyl group, a C₂₋₆ alkenyl group, a C₂₋₆ alkynyl group, a C₃₋₆ cycloalkyl group, a condensed group formed by a C₃₋₆ cycloalkyl group and a benzene ring optionally having 1 to 3 C₁₋₆ alkoxy, a C₆₋₁₄ aryl group or a C₇₋₁₆ aralkyl group, which may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo,
(4) an acyl group represented by the formula: -(C=O)-R⁹, -SO₂-R⁹, -SO-R⁹, -(C=O)NR¹⁰R⁹, -(C=O)O-R⁹, -(C=S)O-R⁹ or -(C=S)NR¹⁰R⁹ wherein R⁹ is (a) a hydrogen atom, (b) a C₁₋₆ alkyl group, a C₂₋₆ alkenyl group, a C₂₋₆ alkynyl group, a C₃₋₆ cycloalkyl group, a condensed group formed by a C₃₋₆ cycloalkyl group and a benzene ring optionally having 1 to 3 C₁₋₆ alkoxy, a C₆₋₁₄ aryl group or a C₇₋₁₆ aralkyl group, which may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo or (c) -OR¹¹ wherein R¹¹ is a hydrogen atom or a C₁₋₆ alkyl group, a C₂₋₆ alkenyl group, a C₂₋₆ alkynyl group, a C₃₋₆ cycloalkyl group, a condensed group formed by a C₃₋₆ cycloalkyl group and a benzene ring optionally having 1 to 3 C₁₋₆ alkoxy, a C₆₋₁₄ aryl group or a C₇₋₁₆ aralkyl group, which may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo, R¹⁰ is a hydrogen atom or a C₁₋₆ alkyl group, or
(5) -OR¹² wherein R¹² is a hydrogen atom, or a C₁₋₆ alkyl group, a C₂₋₆ alkenyl group, a C₂₋₆ alkynyl group, a C₃₋₆ cycloalkyl group, a condensed group formed by a C₃₋₆ cycloalkyl group and a benzene ring optionally having 1 to 3 C₁₋₆ alkoxy, a C₆₋₁₄ aryl group or a C₇₋₁₆ aralkyl group, which may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo;
R¹, R² and R³ are the same or different and are independently
(1) a hydrogen atom,
(2) a halogen atom,
(3) a C₁₋₆ alkyl group, a C₂₋₆ alkenyl group, a C₂₋₆ alkynyl group, a C₃₋₆ cycloalkyl group, a condensed group formed by a C₃₋₆ cycloalkyl group and a benzene ring optionally having 1 to 3 C₁₋₆ alkoxy, a C₆₋₁₄ aryl group or a C₇₋₁₆ aralkyl group, which may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo,
(4) an acyl group represented by the formula: -(C=O)-R¹³, -SO₂-R¹³, -SO-R¹³, -(C=O)NR¹⁴R¹³, -(C=O)O-R¹³, -(C=S)O-R¹³ or -(C=S)NR¹⁴R¹³ wherein R¹³ is (a) a hydrogen atom, (b) a C₁₋₆ alkyl group, a C₂₋₆ alkenyl group, a C₂₋₆ alkynyl group, a C₃₋₆ cycloalkyl group, a condensed group formed by a C₃₋₆ cycloalkyl group and a benzene ring optionally having 1 to 3 C₁₋₆ alkoxy, a C₆₋₁₄ aryl group or a C₇₋₁₆ aralkyl group, which may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo or (c) -OR¹⁵ wherein R¹⁵ is a hydrogen atom, or a C₁₋₆ alkyl group, a C₂₋₆ alkenyl group, a C₂₋₆ alkynyl group, a C₃₋₆ cycloalkyl group, a condensed group formed by a C₃₋₆ cycloalkyl group and a benzene ring optionally having 1 to 3 C₁₋₆ alkoxy, a C₆₋₁₄ aryl group or a C₇₋₁₆ aralkyl group, which may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl , (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo, R¹⁴ is a hydrogen atom or a C₁₋₆ alkyl group; or
(5) -OR¹⁶ wherein R¹⁶ is a hydrogen atom, or a C₁₋₆ alkyl group, a C₂₋₆ alkenyl group, a C₂₋₆ alkynyl group, a C₃₋₆ cycloalkyl group, a condensed group formed by a C₃₋₆ cycloalkyl group and a benzene ring optionally having 1 to 3 C₁₋₆ alkoxy, a C₆₋₁₄ aryl group or a C₇₋₁₆ aralkyl group, which may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo;
   R⁸ is a hydrogen atom, a hydroxy group which may be substituted by C₁₋₆ alkyl or a carboxyl group;
[6] the inhibitor described in the above [3] or [4], wherein Ar¹ and Ar² are a phenyl group; the ring B is a ring represented by the formula: 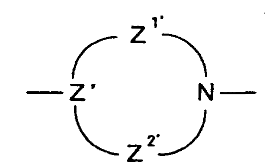 wherein Z' is a methine group; Z^{1'} and Z^{2'} are a methylene group or an ethylene group; X is a bond or an oxygen atom; Y is -(CH₂)ₚ₁NH- wherein ₚ₁ is an integer of 1 to 6; A is CR^{7"} wherein R^{7"} is a hydrogen atom or a C₁₋₆ alkyl group; R¹ is (1) a hydrogen atom, (2) a C₁₋₆ alkyl group which may be substituted by carboxyl or C₁₋₆ alkoxy-carbonyl or (3) a carbamoyl group which may be substituted by a C₁₋₆ alkyl group optionally having C₁₋₆ alkoxy-carbonyl; R² is a hydrogen atom; R³ is a hydrogen atom; R⁸ is a hydrogen atom;
[7] the inhibitor described in the above [3] or [4], wherein the compound is 2-[6-[3-[4-(diphenylmethoxy)piperidino]propylamino]imidazo[1,2-b]pyridazin-2-yl]-2-methylpropionic acid or a salt thereof;
[8] the inhibitor described in the above [3] or [4], wherein the compound is 2-[6-[3-[4-(diphenylmethoxy)piperidino]propylamino]imidazo[1,2-b]pyridazin-2-yl]-2-methylpropionic acid dihydrate;
[9] the inhibitor described in the above [1], which is an agent for preventing and/or treating c-Jun derived disease;
[10] the inhibitor described in the above [1], which is an agent for preventing and/or treating c-Jun N-terminal kinase derived disease;
[11] the inhibitor described in the above [1], which is an agent for preventing and/or treating acute pancreatitis, chronic pancreatitis, adult dyspnea syndrome, pachyderma, lupus erythematosus profundus, chronic thyroid gland, Graves' disease, autoimmune gastritis, autoimmune neutropenia, thrombocytopenia, myasthenia gravis, multiple myeloma, acute myeloblastic leukemia, chronic sarcoma, chronic myelocytic leukemia, metastatic melanoma, Kaposi's sarcoma, marasmic disease, Huntington's chorea, disease derived from ischemia and/or reperfusion of cerebral apoplexy, myocardial ischemia, ischemic heart disease, kidney ischemia, neovascular glaucoma, infantile angiosarcoma, vascularization, hypercardia, abnormal immune response, fever, cellular aging or apoptosis derived disease;
[12] the inhibitor described in the above [1], which is an agent for improving snuff;
[13] a method for inhibiting c-Jun N-terminal kinase activation, which comprises administering an effective amount' of a compound represented by the formula: 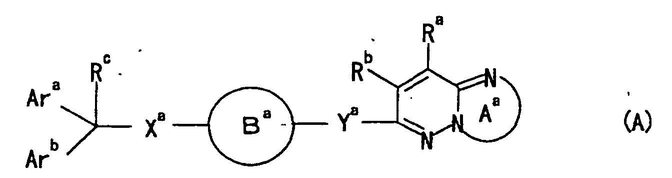 wherein each of Ar^{a} and Ar^{b} is an aromatic group optionally having substituents, Ar^{a} and Ar^{b} optionally form a condensed cyclic group together with the adjacent carbon atom; ring B^{a} is a nitrogen-containing heterocycle optionally having substituents; X^{a} and Y^{a} are the same or different and each is (1) a bond, (2) an oxygen atom, (3) S(O)ₚ (wherein p is an integer of 0 to 2), (4) NR^{d} (wherein R^{d} is a hydrogen atom or a lower alkyl group) or (5) a divalent linear lower hydrocarbon group optionally having substituents and containing 1 to 3 hetero atom(s); ring A^{a} is a 5-membered ring optionally having substituents; R^{a} and R^{b} are the same or different and each is (1) a hydrogen atom, (2) a halogen atom, (3) a hydrocarbon group optionally having substituents, (4) an acyl group or (5) a hydroxy group optionally having a substituent; R^{c} is (1) a hydrogen atom, (2) a hydroxy group optionally substituted by a lower alkyl group or (3) a carboxyl group or a salt thereof, or a prodrug thereof to a mammal;
[14] a method for preventing and/or treating acute pancreatitis, chronic pancreatitis, adult dyspnea syndrome, pachyderma, lupus erythematosus profundus, chronic thyroid gland, Graves' disease, autoimmune gastritis, autoimmune neutropenia, thrombocytopenia, myasthenia gravis, multiple myeloma, acute myeloblastic leukemia, chronic sarcoma, chronic myelocytic leukemia, metastatic melanoma, Kaposi's sarcoma, marasmic disease, Huntington's chorea, disease derived from ischemia and/or reperfusion of cerebral apoplexy, myocardial ischemia, ischemic heart disease, kidney ischemia, neovascular glaucoma, infantile angiosarcoma, vascularization, hypercardia, abnormal immune response, fever, cellular aging or apoptosis derived disease and improving snuff, which comprises administering an effective amount of a compound represented by the formula: 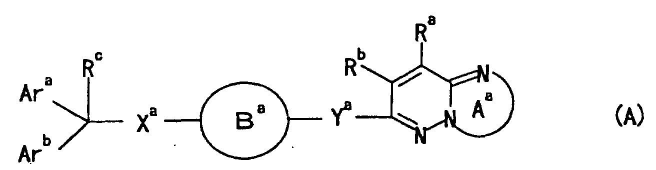 wherein each of Ar^{a} and Ar^{b} is an aromatic group optionally having substituents, Ar^{a} and Ar^{b} optionally form a condensed cyclic group together with the adjacent carbon atom; ring B^{a} is a nitrogen-containing heterocycle optionally having substituents; X^{a} and Y^{a} are the same or different and each is (1) a bond, (2) an oxygen atom, (3) S(O)ₚ (wherein p is an integer of 0 to 2), (4) NR^{d} (wherein R^{d} is a hydrogen atom or a lower alkyl group) or (5) a divalent linear lower hydrocarbon group optionally having substituents and containing 1 to 3 hetero atom(s); ring A^{a} is a 5-membered ring optionally having substituents; R^{a} and R^{b} are the same or different and each is (1) a hydrogen atom, (2) a halogen atom, (3) a hydrocarbon group optionally having substituents, (4) an acyl group or (5) a hydroxy group optionally having a substituent; R^{c} is (1) a hydrogen atom, (2) a hydroxy group optionally substituted by a lower alkyl group or (3) a carboxyl group or a salt thereof, or a prodrug thereof to a mammal;
[15] use of a compound represented by the formula: 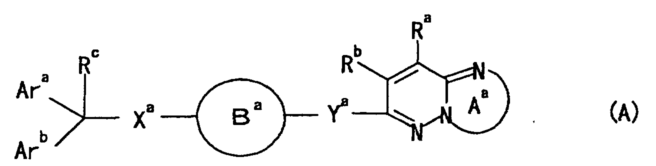 wherein each of Ar^{a} and Ar^{b} is an aromatic group optionally having substituents, Ar^{a} and Ar^{b} optionally form a condensed cyclic group together with the adjacent carbon atom; ring B^{a} is a nitrogen-containing heterocycle optionally having substituents; X^{a} and Y^{a} are the same or different and each is (1) a bond, (2) an oxygen atom, (3) S(O)ₚ (wherein p is an integer of 0 to 2), (4) NR^{d} (wherein R^{d} is a hydrogen atom or a lower alkyl group) or (5) a divalent linear lower hydrocarbon group optionally having substituents and containing 1 to 3 hetero atom(s); ring A^{a} is a 5-membered ring optionally having substituents; R^{a} and R^{b} are the same or different and each is (1) a hydrogen atom, (2) a halogen atom, (3) a hydrocarbon group optionally having substituents, (4) an acyl group or (5) a hydroxy group optionally having a substituent; R^{c} is (1) a hydrogen atom, (2) a hydroxy group optionally substituted by a lower alkyl group or (3) a carboxyl group or a salt thereof, or a prodrug thereof for producing a c-Jun N-terminal kinase activation inhibitor;
[16] use of a compound represented by the formula: 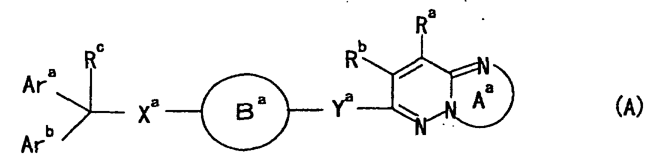 wherein each of Ar^{a} and Ar^{b} is an aromatic group optionally having substituents, Ar^{a} and Ar^{b} optionally form a condensed cyclic group together with the adjacent carbon atom; ring B^{a} is a nitrogen-containing heterocycle optionally having substituents; X^{a} and Y^{a} are the same or different and each is (1) a bond, (2) an oxygen atom, (3) S(O)ₚ (wherein p is an integer of 0 to 2), (4) NR^{d} (wherein R^{d} is a hydrogen atom or a lower alkyl group) or (5) a divalent linear lower hydrocarbon group optionally having substituents and containing 1 to 3 hetero atom(s); ring A^{a} is a 5-membered ring optionally having substituents; R^{a} and R^{b} are the same or different and each is (1) a hydrogen atom, (2) a halogen atom, (3) a hydrocarbon group optionally having substituents, (4) an acyl group or (5) a hydroxy group optionally having a substituent; R^{c} is (1) a hydrogen atom, (2) a hydroxy group optionally substituted by a lower alkyl group or (3) a carboxyl group or a salt thereof, or a prodrug thereof for producing an agent for preventing and/or treating acute pancreatitis, chronic pancreatitis, adult dyspnea syndrome, pachyderma, lupus erythematosus profundus, chronic thyroid gland, Graves' disease, autoimmune gastritis, autoimmune neutropenia, thrombocytopenia, myasthenia gravis, multiple myeloma, acute myeloblastic leukemia, chronic sarcoma, chronic myelocytic leukemia, metastatic melanoma, Kaposi's sarcoma, marasmic disease, Huntington's chorea, disease derived from ischemia and/or reperfusion of cerebral apoplexy, myocardial ischemia, ischemic heart disease, kidney ischemia, neovascular glaucoma, infantile angiosarcoma, vascularization, hypercardia, abnormal immune response, fever, cellular aging or apoptosis derived disease and improving snuff;
[17] a c-Jun N-terminal kinase activation inhibitor, which comprises a compound having (1) anti-histamine activity and/or eosinophile chemotaxis inhibiting activity and (2) c-Jun N-terminal kinase activation inhibiting activity or a prodrug thereof;
[18] a TNF-α inhibitor, which comprises a compound having (1) anti-histamine activity and/or eosinophile chemotaxis inhibiting activity and (2) c-Jun N-terminal kinase activation inhibiting activity or a prodrug thereof;
[19] an agent for preventing and/or treating acute pancreatitis, chronic pancreatitis, adult dyspnea syndrome, pachyderma, lupus erythematosus profundus, chronic thyroid gland, Graves' disease, autoimmune gastritis, autoimmune neutropenia, thrombocytopenia, myasthenia gravis, multiple myeloma, acute myeloblastic leukemia, chronic sarcoma, chronic myelocytic leukemia, metastatic melanoma, Kaposi's sarcoma, marasmic disease, Huntington's chorea, disease derived from ischemia and/or reperfusion of cerebral apoplexy, myocardial ischemia, ischemic heart disease, kidney ischemia, neovascular glaucoma, infantile angiosarcoma, vascularization, hypercardia, abnormal immune response, fever, cellular aging or apoptosis derived disease and improving snuff, which comprises a compound having (1) anti-histamine activity and/or eosinophile chemotaxis inhibiting activity and (2) c-Jun N-terminal kinase activation inhibiting activity or a prodrug thereof;
[20] an agent for preventing and/or treating acute pancreatitis, chronic pancreatitis, adult dyspnea syndrome, pachyderma, lupus erythematosus profundus, chronic thyroid gland, Graves' disease, autoimmune gastritis, autoimmune neutropenia, thrombocytopenia, myasthenia gravis, multiple myeloma, acute myeloblastic leukemia, chronic sarcoma, chronic myelocytic leukemia, metastatic melanoma, Kaposi's sarcoma, marasmic disease, Huntington's chorea, disease derived from ischemia and/or reperfusion of cerebral apoplexy, myocardial ischemia, ischemic heart disease, kidney ischemia, neovascular glaucoma, infantile angiosarcoma, vascularization, hypercardia, abnormal immune response, fever, cellular aging or apoptosis derived disease and improving snuff, which comprises a compound having (1) anti-histamine activity and/or eosinophile chemotaxis inhibiting activity, (2) c-Jun N-terminal kinase activation inhibiting activity and (3) TNF-α inhibiting activity or a prodrug thereof;
[21] a method for inhibiting c-Jun N-terminal kinase activation, which comprises administering an effective amount of a compound having (1) anti-histamine activity and/or eosinophile chemotaxis inhibiting activity and (2) c-Jun N-terminal kinase activation inhibiting activity or a prodrug thereof to a mammal;
[22] a method for preventing and/or treating acute pancreatitis, chronic pancreatitis, adult dyspnea syndrome, pachyderma, lupus erythematosus profundus, chronic thyroid gland, Graves' disease, autoimmune gastritis, autoimmune neutropenia, thrombocytopenia, myasthenia gravis, multiple myeloma, acute myeloblastic leukemia, chronic sarcoma, chronic myelocytic leukemia, metastatic melanoma, Kaposi's sarcoma, marasmic disease, Huntington's chorea, disease derived from ischemia and/or reperfusion of cerebral apoplexy, myocardial ischemia, ischemic heart disease, kidney ischemia, neovascular glaucoma, infantile angiosarcoma, vascularization, hypercardia, abnormal immune response, fever, cellular aging or apoptosis derived disease and improving snuff, which comprises administering an effective amount of a compound having (1) anti-histamine activity and/or eosinophile chemotaxis inhibiting activity and (2) c-Jun N-terminal kinase activation inhibiting activity or a prodrug thereof to a mammal;
[23] use of a compound having (1) anti-histamine activity and/or eosinophile chemotaxis inhibiting activity and (2) c-Jun N-terminal kinase activation inhibiting activity or a prodrug thereof for producing an agent for c-Jun N-terminal kinase activation inhibitor;
[24] use of a compound having (1) anti-histamine activity and/or eosinophile chemotaxis inhibiting activity and (2) c-Jun N-terminal kinase activation inhibiting activity or a prodrug thereof for producing an agent for preventing and/or treating acute pancreatitis, chronic pancreatitis, adult dyspnea syndrome, pachyderma, lupus erythematosus profundus, chronic thyroid gland, Graves' disease, autoimmune gastritis, autoimmune neutropenia, thrombocytopenia, myasthenia gravis, multiple myeloma, acute myeloblastic leukemia, chronic sarcoma, chronic myelocytic leukemia, metastatic melanoma, Kaposi's sarcoma, marasmic disease, Huntington's chorea, disease derived from ischemia and/or reperfusion of cerebral apoplexy, myocardial ischemia, ischemic heart disease, kidney ischemia, neovascular glaucoma, infantile angiosarcoma, vascularization, hypercardia, abnormal immune response, fever, cellular aging or apoptosis derived disease and improving snuff;
[25] a pharmaceutical, which comprises combining anti-histamic agent and c-Jun N-terminal kinase activation inhibitor and/or TNF-α inhibitor;
[26] an anti-allergy pharmaceutical, which comprises combining anti-histamic agent and c-Jun N-terminal kinase activation inhibitor and/or TNF-α inhibitor;
[27] a pharmaceutical for preventing and/or treating chronic urticaria, atopic dermatitis, allergic dermatitis, allergic conjunctivitis, hypersensitivity pneumonitis, eczema, dermatitis herpetiformis, psoriasis, eosinophilic pneumonia (PIE syndrome), chronic obstructive pulmonary disease (COPD) or asthma and improving snuff, which comprises combining anti-histamic agent and c-Jun N-terminal kinase activation inhibitor;
[28] a method for preventing and/or treating allergy, which comprises administering the combination of an effective amount of anti-histamic agent and an effective amount of c-Jun N-terminal kinase activation inhibitor to a mammal;
[29] a method for preventing and/or treating chronic urticaria, atopic dermatitis, allergic dermatitis, allergic conjunctivitis, hypersensitivity pneumonitis, eczema, dermatitis herpetiformis, psoriasis, eosinophilic pneumonia (PIE syndrome), chronic obstructive pulmonary disease (COPD) or asthma and improving snuff, which comprising administering the combination of an effective amount of anti-histamic agent and an effective amount of c-Jun N-terminal kinase activation inhibitor to a mammal;
[30] use of anti-histamic agent and c-Jun N-terminal kinase activation inhibitor for producing anti-allergy agent;
[31] use of anti-histamic agent and c-Jun N-terminal kinase activation inhibitor for producing an agent for preventing and/or treating chronic urticaria, atopic dermatitis, allergic dermatitis, allergic conjunctivitis, hypersensitivity pneumonitis, eczema, dermatitis herpetiformis, psoriasis, eosinophilic pneumonia (PIE syndrome), chronic obstructive pulmonary disease (COPD) or asthma and improving snuff; etc.

### BRIEF DESCRIPTION OF THE DRAWINGS

Fig. 1 shows a time course of the amount of auricle TNF-α in a passively sensitized mouse after application of DNFB (mean ± standard error (n=10)).
Fig. 2 shows the effect of Compound A on the amount of auricle TNF-α in a passively sensitized mouse eight hours after application of DNFB (mean ± standard error (n=10), **p<0.01 (vs. the control group) Dunnett).
Fig. 3 shows the effect of Compound A on the amount of TNF-α released from peritoneal mast cells of a passively sensitized rat after antigen stimulation (mean ± standard error (n=3)).
Fig. 4 shows the effect of Compound A on the activation of JNKs, PLC-γ, ERKs, and p38MAPK in passively sensitized RBL-2H3 cells after antigen stimulation.
Fig. 5 shows the effect of Compound A on the JNK kinase activity on a substrate of c-Jun in passively sensitized RBL-2H3 cells after antigen stimulation.

In the above-mentioned formula (A), Ar^{a} and Ar^{b} are each an "aromatic group optionally having substituents", and Ar^{a} and Ar^{b} may form a condensed cyclic group together with the adjacent carbon atom.

As the "aromatic group optionally having substituents" represented by Ar^{a} and Ar^{b}, for example, the "aromatic group optionally having substituents" represented by Ar¹ and Ar² mentioned below are used.

As the condensed cyclic group formed by Ar^{a} and Ar^{b} together with the adjacent carbon atom, for example, the condensed cyclic group formed by Ar¹ and Ar² together with the adjacent carbon atom mentioned below are used.

In the above-mentioned formula (A), ring B^{a} is "nitrogen containing heterocyclic group optionally having substituents".

As the "nitrogen containing heterocyclic group optionally having substituents" represented by ring B^{a}, for example, the "nitrogen containing heterocyclic group optionally having substituents" represented by ring B mentioned below are used.

In the above-mentioned formula (A), X^{a} and Y^{a} are the same or different and each is (1) a bond, (2) an oxygen atom, (3) S(O)ₚ (wherein p is an integer of 0 to 2), (4) NR^{d} (wherein R^{d} is a hydrogen atom or a lower alkyl group) or (5) a divalent linear lower hydrocarbon group optionally having substituents and containing 1 to 3 hetero atom(s).

As the lower alkyl group represented by R^{d}, for example, linear or branched C₁₋₆ alkyl group such as methyl, ethyl, propyl, isopropyl, butyl, isobutyl, sec-butyl, tert-butyl, pentyl, hexyl and the like, and the like are used.

As the "divalent linear lower hydrocarbon group optionally having substituents and containing 1 to 3 hetero atom(s)" represented by X^{a} and Y^{a}, for example, the "divalent linear lower hydrocarbon group optionally having substituents and containing 1 to 3 hetero atom(s)" represented by X and Y mentioned below are used.

In the above-mentioned formula (A), ring A^{a} is "5-membered ring optionally having substituents".

As the "5-membered ring optionally having substituents" represented by ring A^{a}, for example, the ring represented by the formula: 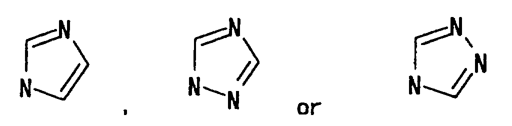 wherein the resonance structures are included if they exist are used. These rings may have substituents where the substitution can be achieved. Examples of the substituents are (1) a hydrogen atom, (2) a halogen atom (e.g., fluorine, chlorine, bromine, iodine), (3) a hydrocarbon group optionally having substituents, (4) an acyl group, (5) a hydroxy group optionally having a substituent and (6) an oxo group, etc.

Examples of the "hydrocarbon group optionally having substituents", the "acyl group" and the "hydroxy group optionally having a substituent" are similar to the "hydrocarbon group optionally having substituents", the "acyl group" and the "hydroxy group optionally having a substituent" represented by R¹ described below.

In the above-mentioned formula (A), R^{a} and R^{b} are the same or different and each is (1) a hydrogen atom, (2) a halogen atom (e.g., fluorine, chlorine, bromine, iodine), (3) a hydrocarbon group optionally having substituents, (4) an acyl group, (5) a hydroxy group optionally having a substituent.

Examples of the "hydrocarbon group optionally having substituents", the "acyl group" and the "hydroxy group optionally having a substituent" represented by R^{a} and R^{b} are similar to the "hydrocarbon group optionally having substituents", the "acyl group" and the "hydroxy group optionally having a substituent" represented by R¹, R² and R³ described below.

In the above-mentioned formula (A), R^{c} is (1) a hydrogen atom, (2) a hydroxy group which may be substituted by a lower alkyl group or (3) a carboxyl group.

The "hydroxy group which may be substituted by a lower alkyl group" represented by R^{c} is similar to the "hydroxy group which may be substituted by a lower alkyl group" represented by R⁸ described below.

Concrete examples of the compound represented by formula (A) 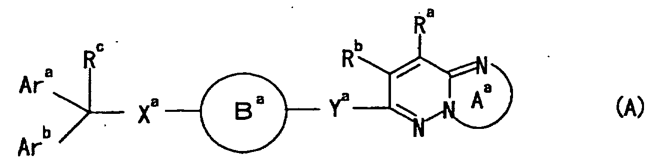 wherein each symbol has the same meaning described above or a salt thereof are a compound represented by formula (I) 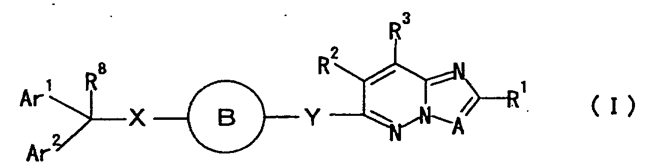 wherein each of Ar¹ and Ar² is an aromatic group'optionally having substituents, Ar¹ and Ar² optionally form a condensed cyclic group together with the adjacent carbon atom; ring B is a nitrogen-containing heterocycle optionally having substituents; X and Y are the same or different and each is (1) a bond, (2) an oxygen atom, (3) S(O)ₚ (wherein p is an integer of 0 to 2), (4) NR⁴ (wherein R⁴ is a hydrogen atom or a lower alkyl group) or (5) a divalent linear lower hydrocarbon group optionally having substituents and containing 1 to 3 hetero atom(s); A is (1) nitrogen atom or (2) CR⁷ wherein R⁷ is a hydrogen atom, a halogen atom, a hydrocarbon group optionally having substituents, an acyl group or hydroxy group optionally having a substituent; R¹, R² and R³ are the same or different and each is (1) a hydrogen atom, (2) a halogen atom, (3) a hydrocarbon group optionally having substituents, (4) an acyl group or (5) a hydroxy group optionally having a substituent; R⁸ is (1) a hydrogen atom, (2) a hydroxy group optionally substituted by a lower alkyl group or (3) a carboxyl group or a salt thereof; and a compound of formula (II) 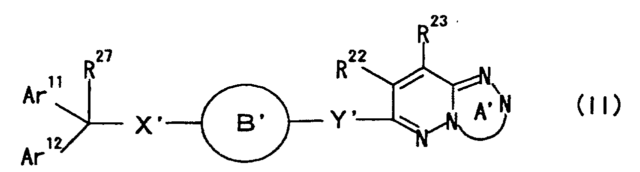 wherein ring A' is the formula. 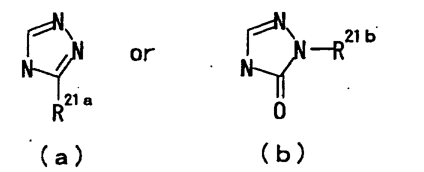 wherein R^{21a} is a hydrogen atom, a halogen atom, a hydrocarbon group optionally having substituents, an acyl group or a hydroxy group optionally having a substituent, R^{21b} is a hydrogen atom, a halogen atom, a hydrocarbon group optionally having substituents, an acyl group or a hydroxy group optionally having a substituent; each of Ar¹¹ and Ar¹² is an aromatic group optionally having substituents, Ar¹¹ and Ar¹² optionally form a condensed cyclic group together with the adjacent carbon atom; ring B' is a nitrogen-containing heterocycle optionally having substituents; X' and Y' are the same or different and each is (1) a bond, (2) an oxygen atom, (3) S(O)_{q} (wherein q is an integer of 0 to 2), (4) NR²⁴ (wherein R²⁴ is a hydrogen atom or a lower alkyl group) or (5) a divalent linear lower hydrocarbon group optionally having substituents and containing 1 to 3 hetero atom(s); R²² and R²³ are the same or different and each is (1) a hydrogen atom, (2) a halogen atom, (3) a hydrocarbon group optionally having substituents, (4) an acyl group or (5) a hydroxy group optionally having a substituent; R²⁷ is (1) a hydrogen atom, (2) a hydroxy group optionally substituted by a lower alkyl group or (3) a carboxyl group or a salt thereof.

Hereinafter the compound represented by formula (I) and formula (II) or a salt thereof will be explained in detail.

In the above-mentioned formula (I), Ar¹ and Ar² are each an "aromatic group optionally having substituents", and Ar¹ and Ar² may form a condensed cyclic group together with the adjacent carbon atom.

As the' "aromatic group" represented by Ar¹ and Ar², for example,
(1) a monocyclic or condensed polycyclic aromatic hydrocarbon group, more specifically a 6 to 14-membered monocyclic or condensed polycyclic aromatic hydrocarbon group exemplified by C₆₋₁₄ aryl group such as phenyl, tolyl, xylyl, biphenyl, 1-naphthyl, 2-naphthyl, 2-indenyl, 1-anthryl, 2-anthryl, 9-anthryl, 1-phenanthryl, 2-phenanthryl, 3-phenanthryl, 4-phenanthryl, 9-phenanthryl and the like, and the like (preferably phenyl, tolyl, xylyl, biphenyl, 1-naphthyl, 2-naphthyl and the like, particularly preferably phenyl and the like), and the like, and
(2) a monocyclic group (preferably 5 to 8-membered) containing, other than carbon atom, preferably 1 or 2 kinds of 1 or more (e.g., 1 to 4, preferably 1 to 3) heteroatoms selected from nitrogen atom, sulfur atom and oxygen atom, or a condensed aromatic heterocyclic group thereof, more specifically aromatic heterocycle such as thiophene, benzo[b]thiophene, benzo[b]furan, benzimidazole, benzoxazole, benzothiazole, benzisothiazole, naphtho[2,3-b]thiophene, thianthrene, furan, isoindolizine, xanthrene, phenoxathiin, pyrrole, imidazole, triazole, thiazole, oxazole, pyrazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, isothiazole, phenothiazine, isoxazole, furazane, phenoxazine or isochroman and the like (preferably pyridine, thiophene or furan and the like, more preferably pyridine and the like), or a monovalent group obtained by removing an optional hydrogen atom from a ring formed by condensing these rings (preferably the aforementioned monocyclic heterocycle) with 1 or plural (preferably 1 or 2, more preferably 1) aromatic rings (e.g., the above-mentioned aromatic hydrocarbon group and the like, preferably benzene ring and the like).

As the "aromatic group" of the "aromatic group optionally having substituents" represented by Ar¹ and Ar², for example, phenyl group and the like are preferable.

As the "substituent" of the aromatic group represented by Ar¹ and Ar², for example, (i) halogen atom (e.g., fluorine, chlorine, bromine, iodine), (ii) lower alkylenedioxy group (e.g., C₁₋₆ alkylenedioxy group such as methylenedioxy, ethylenedioxy and the like, preferably C₁₋₃ alkylenedioxy group and the like), (iii) nitro group, (iv) cyano group, (v) optionally halogenated lower alkyl group, (vi) optionally halogenated lower alkenyl group, (vii) optionally halogenated lower alkynyl group, (viii) lower cycloalkyl group (e.g., C₃₋₆ cycloalkyl group such as cyclopropyl, cyclobutyl, cyclopentyl, cyclohexyl and the like, and the like), (ix) optionally substituted lower alkoxy group, (x) optionally halogenated lower alkylthio group, (xi) hydroxy group, (xii) amino group, (xiii) mono-lower alkylamino group (e.g., mono-C₁₋₆ alkylamino group such as methylamino, ethylamino, propylamino, isopropylamino, butylamino and the like, and the like), (xiv) di-lower alkylamino group (e.g., di-C₁₋₆ alkylamino group such as dimethylamino, diethylamino, dipropylamino, dibutylamino and the like, and the like), (xv) 5 or 6-membered cyclic amino group (e.g., morpholino, piperazin-1-yl, piperidino, pyrrolidin-1-yl and the like), (xvi) lower alkyl-carbonyl' group (e.g., C₁₋₆ alkyl-carbonyl group such as acetyl, propionyl and the like, and the like), (xvii) carboxyl group, (xviii) lower alkoxy-carbonyl group (e.g., C₁₋₆ alkoxy-carbonyl group such as methoxycarbonyl, ethoxycarbonyl, propoxycarbonyl, butoxycarbonyl and the like, and the like), (xix) carbamoyl group, (xx) thiocarbamoyl, (xxi) mono-lower alkyl-carbamoyl group (e.g., mono-C₁₋₆ alkyl-carbamoyl group such as methylcarbamoyl, ethylcarbamoyl and the like, and the like), (xxii) di-lower alkyl-carbamoyl group (e.g., di-C₁₋₆ alkylcarbamoyl group such as dimethylcarbamoyl, diethylcarbamoyl and the like, and the like), (xxiii) aryl-carbamoyl (e.g., C₆₋₁₀ aryl-carbamoyl such as phenylcarbamoyl, naphthylcarbamoyl and the like, and the like), (xxiv) sulfo group, (xxv) lower alkylsulfonyl group (e.g., C₁₋₆ alkylsulfonyl group such as methylsulfonyl, ethylsulfonyl and the like, and the like), (xxvi) aryl group (e.g., C₆₋₁₀ aryl group such as phenyl, naphthyl and the like, and the like), (xxvii) aryloxy group (e.g., C₆₋₁₀ aryloxy group such as phenoxy, naphthyloxy and the like, and the like), (xxviii) aralkyloxy group (e.g., C₇₋₁₆ aralkyloxy group such as benzyloxy and the like, and the like) and the like are used.

As the above-mentioned "optionally halogenated lower alkyl group", for example, lower alkyl group such as C₁₋₆ alkyl group (e.g., methyl, ethyl, propyl, isopropyl, butyl, isobutyl, sec-butyl, tert-butyl, pentyl, hexyl and the like, and the like) optionally having 1 to 3 halogen atoms (e.g., fluorine, chlorine, bromine, iodine), and the like are mentioned. Specific examples thereof include methyl, fluoromethyl, chloromethyl, difluoromethyl, trichloromethyl, trifluoromethyl, ethyl, 2-bromoethyl, 2,2,2-trifluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl and the like.

As the above-mentioned "optionally halogenated lower alkenyl group" and "optionally halogenated lower alkynyl group", for example, lower alkenyl group (e.g., C₂₋₆ alkenyl group such as vinyl, propenyl, isopropenyl, 2-buten-1-yl, 4-penten-1-yl, 5-hexen-1-yl, and the like, and the like) optionally having 1 to 3 halogen atoms (e.g., fluorine, chlorine, bromine, iodine), and lower alkynyl group (e.g., C₂₋₆ alkynyl group such as 2-butyn-1-yl, 4-pentyn-1-yl, 5-hexyn-1-yl, and the like, and the like), optionally having 1 to 3 halogen atoms (e.g., fluorine, chlorine, bromine, iodine), and the like are used.

As the above-mentioned "optionally substituted lower alkoxy group", for example, lower alkoxy group (e.g., C₁₋₆ alkoxy group such as methoxy, ethoxy, propoxy, isopropoxy, n-butoxy, isobutoxy, sec-butoxy, tert-butoxy and the like, and the like) optionally having 1 to 3 halogen atoms (e.g., fluorine, chlorine, bromine, iodine), mono- or di-lower alkylamino group (e.g., mono- or di-C₁₋₆ alkylamino group such as methylamino, dimethylamino, ethylamino, diethylamino and the like, and the like) or lower alkoxy-carbonyl group (e.g., C₁₋₆ alkoxy-carbonyl group such as methoxycarbonyl, ethoxycarbonyl and the like, and the like), and the like are used.

As the above-mentioned "optionally halogenated lower alkylthio group", for example, lower alkylthio group (e.g., C₁₋₆ alkylthio group such as methylthio, ethylthio, n-propylthio, isopropylthio, n-butylthio, isobutylthio, sec-butylthio, tert-butylthio and the like, and the like) optionally having 1 to 3 halogen atoms (e.g., fluorine, chlorine, bromine, iodine), and the like are mentioned. Specific examples thereof include methylthio, difluoromethylthio, trifluoromethylthio, ethylthio, propylthio, isopropylthio, butylthio, 4,4,4-trifluorobutylthio, pentylthio, hexylthio and the like.

Specific examples of the condensed cyclic group formed by Ar¹ and Ar² together with the adjacent carbon atom include condensed cyclic group represented by 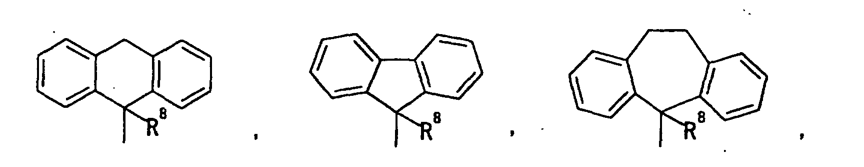 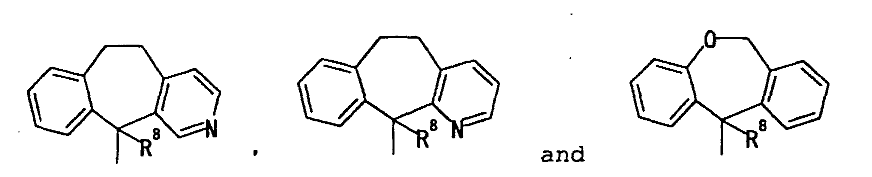 wherein R⁸ is as defined above, and the like.

Ar¹ and Ar² are the same or different and each is preferably an aromatic hydrocarbon group optionally having substituents, such as a C₆₋₁₄ aromatic hydrocarbon group, more preferably a phenyl group optionally having substituents. More specifically, Ar¹ and Ar² are each preferably (1) a phenyl group optionally substituted by halogen atom or C₁₋₆ alkyl, (2) a 5 to 8-membered aromatic heterocyclic group containing, other than carbon atom, 1 to 4 heteroatoms selected from nitrogen atom, sulfur atom and oxygen atom, and the like.

In the above-mentioned formula (I), ring B is a "nitrogen-containing heterocycle optionally having substituents".

As the "nitrogen-containing heterocycle" represented by ring B, for example, a 3 to 13-membered nitrogen-containing heterocycle containing 1 nitrogen atom, and optionally containing 1 to 3 heteroatoms selected from, for example, nitrogen atom, oxygen atom, sulfur atom and the like, and the like are used. In the above-mentioned formula, it is preferable to form a divalent group by removing one hydrogen atom each from nitrogen atom and the other atom of ring B. Specifically, for example, 3 to 9-membered (more preferably 3 to 6-membered) nitrogen-containing heterocyclic group of 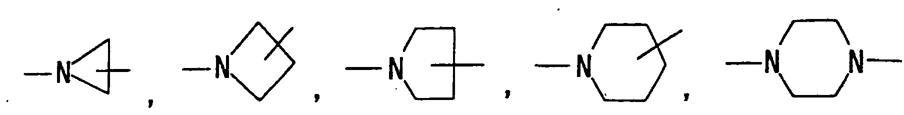 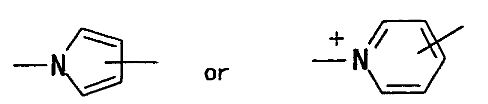 and the like, are preferable.

As the substituent of the nitrogen-containing heterocycle represented by ring B, for example, the "substituent" of the above-mentioned "aromatic group optionally having substituents" represented by Ar¹ and Ar², oxo group and the like are used.

Preferable examples of ring B include, for example, a ring represented by the formula 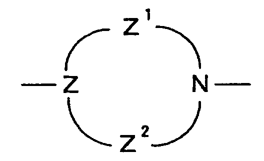 wherein Z is a nitrogen atom or a methine group, and Z¹ and Z² are each a linear C₁₋₄ alkylene group optionally substituted by hydroxy group, oxo group or C₁₋₆ alkyl group, and the like.

As the "C₁₋₆ alkyl group", for example, linear or branched C₁₋₆ alkyl group such as methyl, ethyl, propyl, isopropyl, butyl, isobutyl, sec-butyl, tert-butyl, pentyl, hexyl and the like, and the like are used.

As the "linear C₁₋₄ alkylene group", for example, linear C₁₋₄ alkylene group represented by methylene, ethylene, propylene and butylene are used.

As the "linear C₁₋₄ alkylene group optionally substituted by hydroxy group, oxo group or C₁₋₆ alkyl group" represented by Z¹ and Z², an unsubstituted linear C₁₋₄ alkylene group and the like are preferably used, and an unsubstituted linear C₁₋₂ alkylene group is particularly preferable.

As ring B, piperidine, piperazine and the like are more preferably used.

In the above-mentioned formula (I), X and Y are the same or different and each is (1) a bond, (2) an oxygen atom, (3) S(O)ₚ (p is an integer of 0 to 2), (4) NR⁴ (R⁴ is a hydrogen atom or a lower alkyl group) or (5) a divalent linear lower hydrocarbon group optionally having substituents and containing 1 to 3 heteroatoms.

As the lower alkyl group represented by R⁴, for example, linear or branched C₁₋₆ alkyl group such as methyl, ethyl, propyl, isopropyl, butyl, isobutyl, sec-butyl, tert-butyl, pentyl, hexyl and the like, and the like are used.

The "divalent linear lower hydrocarbon group optionally containing 1 to 3 heteroatoms" represented by X and Y, is a group obtained by removing one hydrogen atom bonded to each of the same or different carbon atoms of lower (C₁₋₆) hydrocarbon, namely 2 hydrogen atoms, which is, for example, a group optionally having a heteroatom selected from oxygen atom, NR^{4'} (R^{4'} is a hydrogen atom or a lower alkyl group), sulfur atom and the like, in a hydrocarbon chain.

As the lower alkyl group represented by R^{4'}, for example, linear or branched C₁₋₆ alkyl group such as methyl, ethyl, propyl, isopropyl, butyl, isobutyl, sec-butyl, tert-butyl, pentyl, hexyl and the like, and the like are used.

Specific examples of the "divalent linear lower hydrocarbon group" include
(i) C₁₋₆ alkylene group (e.g., -CH₂-, -(CH₂)₂-, -(CH₂)₃-, -(CH₂)₄-, -(CH₂)₅-, -(CH₂)₆- and the like),
(ii) C₂₋₆ alkenylene group (e.g., -CH=CH-, -CH=CH-CH₂-, -CH₂-CH=CH-CH₂- , -(CH₂)₂-CH=CH-CH₂- , -(CH₂)₂-CH=CH-(CH₂)₂-, -(CH₂)₃-CH=CH-CH₂- and the like),
(iii) C₂₋₆ alkynylene group (e.g., -C≡C-, -C≡C-CH₂-, -CH₂-C≡C-CH₂-, -(CH₂)₂-C≡C-CH₂-, -(CH₂)₂-C≡C-(CH₂)₂-, -(CH₂)₃-C≡C-CH₂- and the like) and the like.

As the "substituent" of the "divalent linear lower hydrocarbon group optionally containing 1 to 3 heteroatoms" represented by X and Y, for example, the "substituent" of the above-mentioned "aromatic group optionally having substituents" represented by Ar¹ and Ar², oxo group and the like are used. Particularly, hydroxy group and oxo group are preferable.

As X, a bond, an oxygen atom or NH is preferable, and particularly, a bond or an oxygen atom is preferable.

As Y, preferred is, for example, a group of the formula -(CH₂)ₘ-Y¹-(CH₂)ₙ-Y²-
wherein Y¹ and Y² are the same or different and each is a bond, an oxygen atom, S(O)ₚ (p is as defined above), NR⁴' (R^{4'} is as defined above), a carbonyl group, a carbonyloxy group or a group of the formula 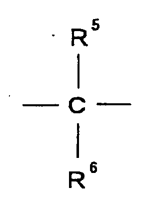 wherein R⁵ and R⁶ are the same or different and each is a hydroxy group or a C₁₋₄ alkyl group, and m and n are each an integer of 0 to 4 (provided that the sum of m and n is not more than 6, and the like.

As the "C₁₋₄ alkyl group" represented by R⁵ and R⁶, for example, linear or branched C₁₋₄ alkyl group such as methyl, ethyl, propyl, isopropyl, butyl, isobutyl, sec-butyl, tert-butyl and the like, and the like are used.

As Y, for example, a group represented by (i) C₁₋₆ alkylene group, (ii) -(CH₂)ₚ₁O-, (iii) -(CH₂)ₚ₁NH-, (iv) -(CH₂)ₚ₁S-, (v) -(CH₂)_{q1}CH(OH)(CH₂)_{q2}O-, (vi) -(CH₂)_{q1}CH(OH)(CH₂)_{q2}NH-, (vii) -(CH₂)_{q1}CH(OH)(CH₂)_{q2}S-, (viii) -(CH₂)ₚ₁CONH-, (ix) -COO(CH₂)ₚ₁O-, (x) -COO(CH₂)ₚ₁NH-, (xi) -COO(CH₂)ₚ₁S-, (xii) -(CH₂)_{q1}O(CH₂)_{q2}O-, (xiii) -(CH₂)_{q1}O(CH₂)_{q2}NH- or (xiv) -(CH₂)_{q1}O(CH₂)_{q2}S- (p¹ is an integer of 1 to 6 and q¹ and q² are each an integer of 1 to 3) is preferable.

Of these, for example, Y is preferably a bond, -(CH₂)₂-O-, -(CH₂)₃-O-, -(CH₂)₄-O-, -(CH₂)₆-O-, -(CH₂)₂-NH-, -(CH₂)₃-NH-, -(CH₂)₄-NH-, -(CH₂)₃-S-, -CH₂-CH(OH)-CH₂-O-, -(CH₂)₂-CO-NH-, -CH₂-CO-NH-, -CO-O-(CH₂)₂-O-, -CO-O-(CH₂)₃-O-, -(CH₂)₆-NH-, -(CH₂)₆-S-, -(CH₂)₂-O-(CH₂)₂-O-, -(CH₂)₂-O-(CH₂)₂-S- and the like.

In the above-mentioned formula, A is a nitrogen atom or CR⁷ (R⁷ is a hydrogen atom, a halogen atom, a hydrocarbon group optionally having substituents, an acyl group or a hydroxy group optionally having a substituent).

As the "halogen atom" represented by R⁷, fluorine, chlorine, bromine and iodine are exemplified.

The "hydrocarbon group" represented by R⁷ is, for example, a group obtained by removing one hydrogen atom from a hydrocarbon compound. Examples thereof include chain or cyclic hydrocarbon group such as alkyl group, alkenyl group, alkynyl group, cycloalkyl group, aryl group, aralkyl group and the like. Of these, chain (linear or branched) or cyclic hydrocarbon group having 1 to 16 carbon atoms and the like are preferable, and
a) alkyl group [preferably lower alkyl group (e.g., C₁₋₆ alkyl group such as methyl, ethyl, propyl, isopropyl, butyl, isobutyl, sec-butyl, tert-butyl, pentyl, hexyl and the like, and the like)],
b) alkenyl group [preferably lower alkenyl group (e.g., C₂₋₆ alkenyl group such as vinyl, allyl, isopropenyl, butenyl, isobutenyl, sec-butenyl and the like, and the like)],
c) alkynyl group [preferably lower alkynyl group (e.g., C₂₋₆ alkynyl group such as propargyl, ethynyl, butynyl, 1-hexynyl and the like, and the like)],
d) cycloalkyl group [preferably lower cycloalkyl group (e.g., C₃₋₆ cycloalkyl group such as cyclopropyl, cyclobutyl, cyclopentyl, cyclohexyl optionally condensed with benzene ring optionally having 1 to 3 lower alkoxy groups (e.g., C₁₋₆ alkoxy group such as methoxy and the like, and the like), and the like, and the like)],
e) aryl group (e.g., C₆₋₁₄ aryl group such as phenyl, tolyl, xylyl, biphenyl, 1-naphthyl, 2-naphthyl, 2-indenyl, 1-anthryl, 2-anthryl, 9-anthryl, 1-phenanthryl, 2-phenanthryl, 3-phenanthryl, 4-phenanthryl, 9-phenanthryl and the like, and the like, preferably phenyl group),
f) aralkyl group [preferably lower aralkyl group (e.g., C₇₋₁₆ aralkyl group such as benzyl, phenethyl, diphenylmethyl, 1-naphthylmethyl, 2-naphthylmethyl, 2-phenylethyl, 2-diphenylethyl, 1-phenylpropyl, 2-phenylpropyl, 3-phenylpropyl, 4-phenylbutyl, 5-phenylpentyl and the like, and the like, more preferably benzyl group)] and the like are preferable.

As the "substituent" of the "hydrocarbon group" represented by R⁷, for example, the "substituent" of the above-mentioned "aromatic group optionally having substituents" represented by Ar¹ and Ar², oxo group (with the proviso that the acyl group formed by the hydrocarbon group and the oxo group is excluded) and the like are used.

As the "acyl group" represented by R⁷, for example, -(C=O)-R⁹, -SO₂-R⁹, -SO-R⁹, -(C=O)NR¹⁰R⁹, -(C=O)O-R⁹, -(C=S)O-R⁹, -(C=S)NR¹⁰R⁹, (R⁹ is a hydrogen atom, a hydrocarbon group optionally having substituents or a hydroxy group optionally having a substituent, and R¹⁰ is a hydrogen atom or a lower alkyl group (e.g., C₁₋₆ alkyl group such as methyl, ethyl, propyl, isopropyl, butyl, isobutyl, sec-butyl, tert-butyl, pentyl, hexyl and the like, and the like, particularly C₁₋₃ alkyl group such as methyl, ethyl, propyl, isopropyl and the like, and the like are preferable)) and the like are mentioned.

Of these, preferred are -(C=O)-R⁹, -SO₂-R⁹, -SO-R⁹, -(C=O)NR¹⁰R⁹ and -(C=O)O-R⁹, and -(C=O)-R⁹ is more preferable.

The "hydrocarbon group" represented by R⁹ is a group obtained by removing one hydrogen atom from a hydrocarbon compound. Examples thereof include chain (linear or branched) or cyclic hydrocarbon group such as alkyl group, alkenyl group, alkynyl group, cycloalkyl group, aryl group, aralkyl group and the like. Specific example thereof is the above-mentioned "hydrocarbon group" represented by R⁷ and the like, and of these, chain or cyclic hydrocarbon group having 1 to 16 carbon atoms and the like are preferable, and lower (C₁₋₆) alkyl group is particularly preferable.

As the "substituent" that the "hydrocarbon group" represented by R⁹ may have, for example, the "substituent" of the above-mentioned "aromatic group optionally having substituents" represented by Ar¹ and Ar², oxo group and the like are used.

As the "hydroxy group optionally having a substituent" represented by R⁹, for example, those similar to the "hydroxy group optionally having a substituent" represented by R⁷ to be mentioned below and the like are used.

As the "hydroxy group optionally having a substituent" represented by R⁷, for example, (1) a hydroxy group or (2) a hydroxy group having, for example, one aforementioned "hydrocarbon group optionally having substituents" and the like, instead of hydrogen atom of hydroxy group is used.

As R⁷, (1) a hydrogen atom, (2) a halogen atom, (3) a C₁₋₆ alkyl group optionally substituted by carboxyl group or C₁₋₆ alkoxy-carboxyl, (4) a C₁₋₆ alkoxy group, (5) a C₁₋₆ alkoxy-carbonyl group or (6) a carboxyl group is preferable, and particularly, a hydrogen atom, a halogen atom, a C₁₋₆ alkyl group, a C₁₋₆ alkoxy-carbonyl group and a carboxyl group are preferable.

As A, a nitrogen atom or CR⁷' (R⁷' is a hydrogen atom, a halogen atom, a C₁₋₆ alkyl group, a C₁₋₆ alkoxy-carbonyl group or a carboxyl group) is preferable, and of these, nitrogen atom, CH and C-CH₃ are preferable, and nitrogen atom and CH are particularly preferable.

In the above-mentioned formula (I), R¹, R² and R³ are the same or different and each is (1) a hydrogen atom, (2) a halogen atom, (3) a hydrocarbon group optionally having substituents, (4) an acyl group or (5) a hydroxy group optionally having a substituent.

As the "halogen atom" represented by R¹, R² and R³, fluorine, chlorine, bromine and iodine are exemplified.

As the "hydrocarbon group optionally having substituents" represented by R¹, R² and R³, for example, the above-mentioned "hydrocarbon group optionally having substituents" represented by R⁷, and the like are used.

As the "acyl group" represented by R¹, R² and R³, for example, the above-mentioned "acyl group" represented by R⁷, and the like are used.

As the "hydroxy group optionally having a substituent" represented by R¹, R² and R³, for example, the above-mentioned "hydroxy group optionally having a substituent" represented by R⁷, and the like are used.

R¹, R² and R³ are the same or different and each is (1) a hydrogen atom, (2) a C₁₋₆ alkyl group optionally substituted by carboxyl group or C₁₋₆ alkoxy-carbonyl, (3) a C₁₋₆ alkoxy group, (4) a C₁₋₆ alkoxy-carbonyl group, (5) a carboxyl group or (6) a C₆₋₁₄ aryl group (particularly phenyl) is preferable, and (1) a hydrogen atom, (2) a C₁₋₆ alkyl group optionally substituted by carboxyl group and C₁₋₆ alkoxy-carbonyl, (3) a C₁₋₆ alkoxy group, (4) a C₁₋₆ alkoxy-carbonyl group or (5) a carboxyl group are more preferable.

As R¹, (1) a hydrogen atom, (2) a C₁₋₆ alkyl group optionally substituted by a group selected from the group consisting of (i) carboxyl, (ii) C₁₋₆ alkoxy-carbonyl, (iii) hydroxy or (iv) carbamoyl optionally having mono- or di-C₁₋₆ alkyl, (3) a C₆₋₁₄ aryl group, (4) a C₁₋₆ alkoxy group, (5) a C₁₋₆ alkoxy-carbonyl group, (6) a carboxyl group, (7) a carbamoyl group optionally having C₁₋₆ alkyl optionally substituted by carboxyl or C₁₋₆ alkoxy-carbonyl, or (8) a C₃₋₆ cycloalkyl group optionally substituted by C₁₋₆ alkoxy-carbonyl and the like are also preferable.

As R², a hydrogen atom, a C₁₋₆ alkyl group, a C₁₋₆ alkoxy-carbonyl group or a carboxyl group and the like are also preferable.

As R³, a hydrogen atom is preferable.

In the above-mentioned formulas, R⁸ is (1) a hydrogen atom, (2) hydroxy group optionally substituted by lower alkyl group, or (3) carboxyl group.

In the above-mentioned the formula, the "lower alkyl group" of "hydroxy group optionally substituted by lower alkyl group", which is represented by R⁸ is, for example, C₁₋₆ alkyl group such as methyl, ethyl, propyl, isopropyl, butyl, isobutyl, sec-butyl, tert-butyl, pentyl,' hexyl and the like, and the like.

As R⁸, a hydrogen atom or a hydroxy group is preferable, and a hydrogen atom is particularly preferable.

As the compound represented by the formula (I) (hereinafter abbreviated as the compound (I)), a compound wherein Ar¹ and Ar² are each (1) a phenyl group optionally substituted by halogen atom or C₁₋₆ alkyl or (2) a 5 to 8-membered aromatic heterocyclic group containing, other than carbon atom, 1 to 4 heteroatoms selected from nitrogen atom, sulfur atom and oxygen atom, ring B is a ring represented by the formula 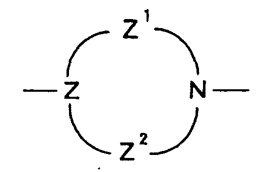 wherein Z is a nitrogen atom or a methine group, and Z¹ and Z² are each a linear C₁₋₄ alkylene group optionally substituted by hydroxy group, oxo group or C₁₋₆ alkyl group, X is a bond, an oxygen atom or NH, Y is a group represented by (i) C₁₋₆ alkylene group, (ii) -(CH₂)ₚ₁O-, (iii) -(CH₂)ₚ₁NH-, (iv) -(CH₂)ₚ₁S-, (v) -(CH₂)_{q1}CH(OH)(CH₂)_{q2}O-, (vi) -(CH₂)_{q1}CH(OH)(CH₂)_{q2}NH-, (vii) -(CH₂)_{q1}CH(OH)(CH₂)_{q2}S-, (viii) -(CH₂)ₚ₁CONH-, (ix) -COO(CH₂)ₚ₁O-, (x) -COO(CH₂)ₚ₁NH-, (xi) -COO(CH₂)ₚ₁S-, (xii) -(CH₂)_{q1}O(CH₂)_{q2}O-, (xiii) -(CH₂)_{q1}O(CH₂)_{q2}NH- or (xiv) -(CH₂)_{q1}O(CH₂)_{q2}S- (p¹ is an integer of 1 to 6, and q¹ and q² are each an integer of 1 to 3), A is a nitrogen atom or CR^{7'} (R^{7'} is a hydrogen atom, a halogen atom, a C₁₋₆ alkyl group, a C₁₋₆ alkoxy-carbonyl group or a carboxyl group) , R¹ is (1) a hydrogen atom, (2) a C₁₋₆ alkyl group optionally substituted by a group selected from the group consisting of (i) carboxyl, (ii) C₁₋₆ alkoxy-carbonyl, (iii) hydroxy and (iv) carbamoyl optionally having mono- or di-C₁₋₆ alkyl, (3) a C₆₋₁₄ aryl group, (4) a C₁₋₆ alkoxy group, (5) a C₁₋₆ alkoxy-carbonyl group, (6) a carboxyl group, (7) a carbamoyl group optionally having C₁₋₆ alkyl optionally substituted by carboxyl or C₁₋₆ alkoxy-carbonyl, or (8) a C₃₋₆ cycloalkyl group optionally substituted by C₁₋₆ alkoxy-carbonyl, R² is a hydrogen atom, a C₁₋₆ alkyl group, a C₁₋₆ alkoxy-carbonyl group or a carboxyl group, R³ is a hydrogen atom, R⁸ is a hydrogen atom or a hydroxy group is preferable.

Particularly, a compound wherein Ar¹ and Ar² are each a phenyl group, ring B is a ring represented by the formula 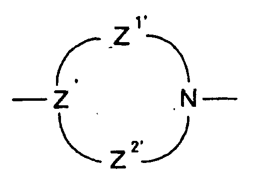 wherein Z' is a methine group, and Z^{1'} and Z^{2'} are methylene group or ethylene group (preferably ethylene group), X is a bond, an oxygen atom or NH (preferably a bond or an oxygen atom), Y is a group represented by -(CH₂)ₚ₁NH- (p¹ is an integer of 1 to 6), A is CR^{7"} (R^{7"} is a hydrogen atom or a C₁₋₆ alkyl group), R¹ is (1) a hydrogen atom, (2) a C₁₋₆ alkyl group optionally substituted by carboxyl or C₁₋₆ alkoxy-carbonyl or (3) a carbamoyl group optionally having C₁₋₆ alkyl optionally substituted by C₁₋₆ alkoxy-carbonyl, R² is a hydrogen atom, R³ is a hydrogen atom, and R⁸ is a hydrogen atom, is preferable.

More specifically, (1) ethyl 2-[6-[3-[4-(diphenylmethoxy)piperidino]propylamino]imidazo[1,2-b]pyridazin-2-yl]-2-methylpropionate or a salt thereof (particularly, difumarate, disuccinate, citrate and the like), (2) 2-[6-[3-[4-(diphenylmethoxy)piperidino]propylamino]-imidazo[1,2-b]pyridazin-2-yl]-2-methylpropionic acid or a salt thereof (particularly dihydrate), (3) ethyl N-[6-[3-[4-(diphenylmethoxy)piperidino]propylamino]imidazo[1,2-b]pyridazine-2-carbonyl]glycinate or a salt thereof, (4) ethyl 2-[6-[3-[4-(diphenylmethoxy)piperidino]propylamino]-3-methylimidazo[1,2-b]pyridazin-2-yl]-2-methylpropionate or a salt thereof (particularly dihydrochloride), (5) ethyl 2-[6-[3-[4-(diphenylmethylamino)piperidino]propylamino]imidazo[1,2-b]pyridazin-2-yl]-2-methylpropionate or a salt thereof, (6) 2-[6-[3-[4-(diphenylmethoxy)piperidino]propylamino]-3-methylimidazo[1,2-b]pyridazin-2-yl]-2-methylpropionic acid or a salt thereof, and (7) N-[6-[3-[4-(diphenylmethoxy)piperidino]propylamino]-3-methylimidazo[1,2-b]pyridazine-2-carbonyl]glycine or a salt thereof and the like are preferable.

And the compound represented by formula (II): 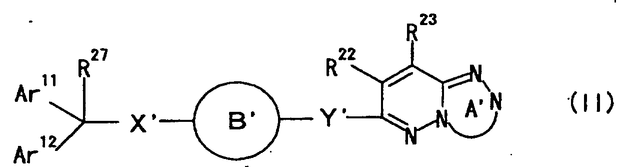 wherein ring A' is a ring represented by the formula: 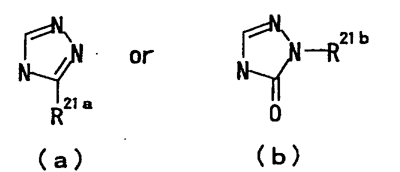 wherein R^{21a} is a hydrogen atom, a halogen atom, a hydrocarbon group optionally having substituents, an acyl group or a hydroxy group having a substituent; R^{21b} is a hydrogen atom, a halogen atom, a hydrocarbon group optionally having substituents, an acyl group or a hydroxy group optionally having a substituent; Ar¹¹ and Ar¹² are independently an aromatic group optionally having substituents, and may form a condensed ring group with an adjacent carbon atom; ring B' is a nitrogen-containing heterocycle optionally having a substituent; X' and Y', whether identical or not, are (1) a bond, (2) an oxygen atom, (3) S(O)_{q} (q is an integer of 0 to 2), (4) NR²⁴ wherein R²⁴ is a hydrogen atom or a lower alkyl group, or (5) a divalent linear lower hydrocarbon group which may have a substituent, and which may contain 1 to 3 hetero atoms; R²² and R²³, whether identical or not, are (1) a hydrogen atom, (2) a halogen atom, (3) a hydrocarbon group optionally having substituents, (4) an acyl group or (5) a hydroxy group optionally having a substituent; R²⁷ is (1) a hydrogen atom, (2) a hydroxy group which may be substituted by lower alkyl or (3) a carboxyl group; or a salt thereof or a prodrug thereof is included the content of compound (A) described above and exhibits excellent JNK activation inhibitory activity and TNF-α inhibitory activity. Therefore the compound can be used in the same way as compound (I) or its salt or its prodrug.

With respect to formula (II) above, compound (II) wherein ring A' is type (a) and compound (II) wherein ring A' is type (b) are hereinafter referred to as compound (IIa) and compound (IIb), respectively.

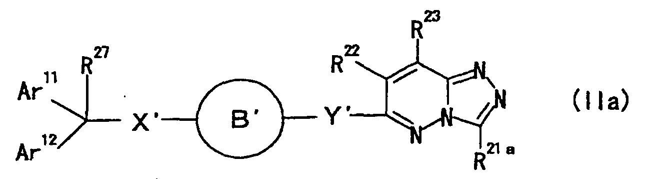

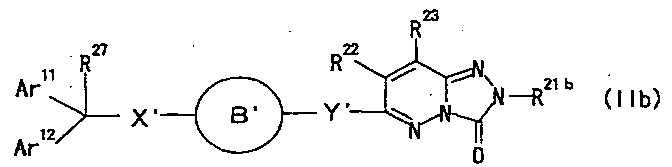

In formula (II) above, Ar¹¹ and Ar¹² are an "aromatic group optionally having substituents", and may form a condensed ring group with an adjacent carbon atom.

Examples of the "aromatic group" represented by Ar¹¹ and Ar¹² include (1) monocyclic or condensed polycyclic aromatic hydrocarbon group, specifically 6- to 14-membered monocyclic or condensed polycyclic aromatic hydrocarbon group such as C₆₋₁₄ aryl group such as phenyl, tolyl, xylyl, biphenyl, 1-naphthyl, 2-naphthyl, 2-indenyl, 1-anthryl, 2-anthryl, 9-anthryl, 1-phenanthryl, 2-phenanthryl, 3-phenanthryl, 4-phenanthryl, 9-phenanthryl, etc. (preferably phenyl, tolyl, xylyl, biphenyl, 1-naphthyl, 2-naphthyl, etc., particularly preferably phenyl, etc.), or (2) monocyclic group (preferably 5- to 8-membered) containing 1 or more (e.g., 1 to 4, preferably 1 to 3) of one or two kinds of hetero atoms selected from among a nitrogen atom, a sulfur atom and an oxygen atom, in addition to carbon atoms, or condensed aromatic heterocyclic group thereof, specifically aromatic heterocycle such as thiophene, benzo[b]thiophene, benzo[b]furan, benzimidazole, benzoxazole, benzothiazole, benzisothiazole, naphtho[2,3-b]thiophene, thianthrene, furan, isoindolylzine, xanthrene, phenoxathiin, pyrrole, imidazole, triazole, thiazole, oxazole, pyrazole, pyridine, pyrazine, pyrimidine, pyridazine, indole, isoindole, 1H-indazole, purine, 4H-quinolizine, isoquinoline, quinoline, phthalazine, naphthylidine, quinoxaline, quinazoline, cinnoline, carbazole, β-carboline, phenanthridine, acridine, phenazine, isothiazole, phenothiazine, isoxazole, furazane, phenoxazine and isochroman (preferably pyridine, thiophene, furan, etc., more preferably pyridine etc.), or monovalent group resulting from removal of an optionally selected hydrogen atom from a condensed ring formed by one of these rings (preferably monocyclic heterocycles mentioned above) and one or more than one (preferably 1 or 2, more preferably 1) aromatic ring (e.g., aromatic hydrocarbon groups mentioned above, preferably benzene ring, etc.).

The "aromatic group" of the "aromatic group optionally having a substituent" represented by Ar¹¹ and Ar¹² is preferably phenyl or the like.

Examples of the "substituent" for the aromatic group represented by Ar¹¹ and Ar¹² include: (i) halogen atom (e.g., fluorine, chlorine, bromine, iodine), (ii) lower alkylenedioxy group (e.g., C₁₋₃ alkylenedioxy group such as methylenedioxy and ethylenedioxy, and the like), (iii) nitro group, (iv) cyano group, (v) optionally halogenated lower alkyl group, (vi) optionally halogenated lower alkenyl group, (vii) optionally halogenated lower alkynyl group, (viii) lower cycloalkyl group (e.g., C₃₋₆ cycloalkyl group such as cyclopropyl, cyclobutyl, cyclopentyl and cyclohexyl, and the like), (ix) lower alkoxy group which may be substituted, (x) optionally halogenated lower alkylthio group, (xi) hydroxy group, (xii) amino group, (xiii) mono-lower alkylamino group (e.g., mono-C₁₋₆ alkylamino' group such as methylamino, ethylamino, propylamino, isopropylamino and butylamino, and the like), (xiv) di-lower alkylamino group (e.g., di-C₁₋₆ alkylamino group such as dimethylamino, diethylamino, dipropylamino and dibutylamino, and the like), (xv) 5- or 6-membered cyclic amino group (e.g., morpholino, piperazin-1-yl, piperidino, pyrroridin-1-yl, and the like), (xvi) lower alkyl-carbonyl group (e.g., C₁₋₆ alkyl-carbonyl group such as acetyl, propionyl, and the like), (xvii) carboxyl group, (xviii) lower alkoxy-carbonyl group (e.g., C₁₋₆ alkoxy-carbonyl group such as methoxycarbonyl, ethoxycarbonyl, propoxycarbonyl, butoxycarbonyl, and the like), (xix) carbamoyl group, (xx) thiocarbamoyl group, (xxi) mono-lower alkyl-carbamoyl group (e.g., mono-C₁₋₆ alkyl-carbamoyl group such as methylcarbamoyl, ethylcarbamoyl, and the like) or mono-lower alkyl-thiocarbamoyl group (e.g., mono-C₁₋₆ alkyl-thiocarbamoyl group such as methylthiocarbamoyl, ethylthiocarbamoyl, and the like), (xxii) di-lower alkyl-carbamoyl group (e.g., di-C₁₋₆ alkylcarbamoyl group such as dimethylcarbamoyl, diethylcarbamoyl, and the like) or di-lower alkyl-thiocarbamoyl group (e.g., di-C₁₋₆ alkylthiocarbamoyl group such as dimethylthiocarbamoyl, diethylthiocarbamoyl, and the like), (xxiii) aryl-carbamoyl (e.g., C₆₋₁₀ aryl-carbamoyl such as phenylcarbamoyl, naphthylcarbamoyl, and the like) or aryl-thiocarbamoyl (e.g., C₆₋₁₀ aryl-thiocarbamoyl such as phenylthiocarbamoyl, naphthylthiocarbamoyl, and the like), (xxiv) sulfo group, (xxv) lower alkylsulfonyl group (e.g., C₁₋₆ alkylsulfonyl group such as methylsulfonyl, ethylsulfonyl, and the like), (xxvi) aryl group (e.g., C₆₋₁₀ aryl group such as phenyl, naphthyl and the like), (xxvii) aryloxy group (e.g., C₆₋₁₀ aryloxy group such as phenyloxy, naphthyloxy, and the like), (xxviii) aralkyloxy group (e.g., C₇₋₁₆ aralkyloxy group such as benzyloxy and the like), and the like.

Examples of the "optionally halogenated lower alkyl group" include lower alkyl group (e.g., C₁₋₆ alkyl group such as methyl, ethyl, propyl, isopropyl, butyl, isobutyl, sec-butyl, tert-butyl, pentyl, hexyl and the like) optionally having 1 to 3 halogen atoms (e.g., fluorine, chlorine, bromine, iodine), specifically methyl, chloromethyl, difluoromethyl, trichloromethyl, trifluoromethyl, ethyl, 2-bromoethyl, 2,2,2-trifluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl, and the like.

Examples of the "optionally halogenated lower alkenyl group" and "optionally halogenated lower alkynyl group" include lower alkenyl group (e.g., C₂₋₆ alkenyl group such as vinyl, propenyl, isopropenyl, 2-buten-1-yl, 4-penten-1-yl, 5-hexen-1-yl, and the like) optionally having 1 to 3 halogen atoms (e.g., fluorine, chlorine, bromine, iodine) and lower alkynyl group (e.g., C₂₋₆ alkynyl group such as 2-butyn-1-yl, 4-pentyn-1-yl, 5-hexyn-1-yl, and the like) optionally having 1 to 3 halogen atoms (e.g., fluorine, chlorine, bromine, iodine).

Examples of the "lower alkoxy group which may be substituted" include lower alkoxy group (e.g., C₁₋₆ alkoxy group such as methoxy, ethoxy, propoxy, isopropoxy, n-butoxy, isobutoxy, sec-butoxy, tert-butoxy, and the like) optionally having 1 to 3 halogen atoms (e.g., fluorine, chlorine, bromine, iodine), mono- or di-lower alkylamino group (e.g., mono- or di-C₁₋₆ alkylamino group such as methylamino, dimethylamino, ethylamino and diethylamino) or lower alkoxy-carbonyl group (e.g., C₁₋₆ alkoxy-carbonyl group such as methoxycarbonyl, ethoxycarbonyl, and the like).

Examples of the "optionally halogenated lower alkylthio group" include lower alkylthio group (e.g., C₁₋₆ alkylthio group such as methylthio, ethylthio, n-propylthio, isopropylthio, n-butylthio, isobutylthio, sec-butylthio, tert-butylthio, and the like) optionally having 1 to 3 halogen atoms (e.g., fluorine, chlorine, bromine, iodine), specifically methylthio, difluoromethylthio, trifluoromethylthio, ethylthio, propylthio, isopropylthio, butylthio, 4,4,4-trifluorobutylthio, pentylthio, hexylthio, and the like.

Specific examples of the condensed ring formed by Ar¹¹ and Ar¹², along with the adjacent carbon atom, include condensed ring groups represented by the formula: 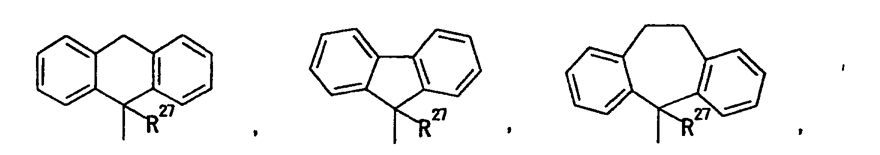 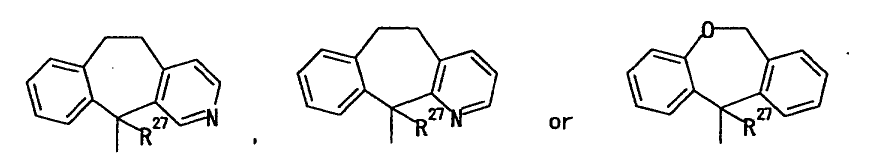 wherein R²⁷ has the same definition as that shown above.

It is preferable that Ar¹¹ and Ar¹², whether identical or not, are an aromatic hydrocarbon group (e.g., C₆₋₁₄ aromatic hydrocarbon group) optionally having substituents, and a benzene ring optionally having substituents is more preferred: More preferably, Ar¹¹ and Ar¹² are independently (1) phenyl group which may be substituted by a halogen atom or C₁₋₆ alkyl, or (2) a 5- to 8-membered aromatic heterocyclic group containing 1 to 4 hetero atoms selected from nitrogen atom, sulfur atom and oxygen atom, in addition to carbon atoms.

In formula (II) above, ring B' represents a "nitrogen-containing heterocycle optionally having a substituent".

Examples of the "nitrogen-containing heterocycle" represented by ring B' include 3- to 13-membered nitrogen-containing heterocycle which contains one nitrogen atom, which may further contain 1 to 3 hetero atoms selected from nitrogen atom, oxygen atom, sulfur atom, and the like. In formula (II) above, it is preferable that ring B' forms a divalent group resulting from removal of one hydrogen atom from the nitrogen atom and another atom of ring B', respectively. Specific examples include 3- to 9-membered (more preferably 3- to 6-membered) nitrogen atom-containing heterocyclic groups such as

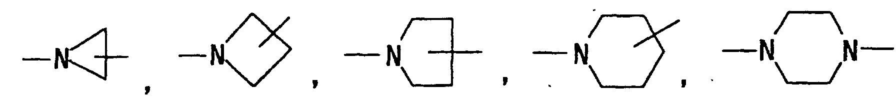

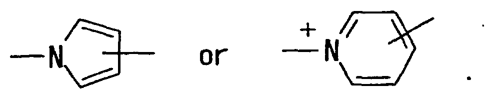

Examples of the substituent for the nitrogen-containing heterocycle represented by ring B' include the same as the substituent for the "aromatic group optionally having a substituent" represented by Ar¹¹ and Ar¹² above and oxo group and the like.

Specific preferable examples of ring B' include a ring represented by the formula: 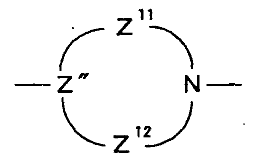 wherein Z" is a nitrogen atom or a methine group, Z¹¹ and Z¹² are independently a linear C₁₋₄ alkylene group which may be substituted by a hydroxy group, an oxo group or a C₁₋₆ alkyl group.

Examples of said "C₁₋₆ alkyl group" include linear or branched C₁₋₆ alkyl group such as methyl, ethyl, propyl, isopropyl, butyl, isobutyl, sec-butyl, tert-butyl, pentyl, hexyl, and the like.

Examples of said "linear C₁₋₄ alkylene group" include linear C₁₋₄ alkylene group such as methylene, ethylene, propylene and butylene.

Preferable examples of the "linear C₁₋₄ alkylene group which may be substituted by a hydroxy group, an oxo group or a C₁₋₆ alkyl group" represented by Z¹¹ and Z¹² include unsubstituted linear C₁₋₄ alkylene group and the like, and unsubstituted linear C₁₋₂ alkylene groups are more preferred.

Ring B' is more preferably piperidine, piperazine, and the like.

In formula (II) above, X' and Y', whether identical or not, are (1) a bond, (2) oxygen atom, (3) S(O)_{q} (q is an integer of 0 to 2), (4) NR²⁴ wherein R²⁴ is a hydrogen atom or a lower alkyl group, or (5) a divalent linear lower hydrocarbon group which may contain a substituent, and which may further contain 1 to 3 hetero atoms.

Examples of the lower alkyl group represented by R²⁴ include linear or branched C₁₋₆ alkyl group such as methyl, ethyl, propyl, isopropyl, butyl, isobutyl, sec-butyl, tert-butyl, pentyl, hexyl, and the like.

Examples of the "divalent linear lower hydrocarbon group which may further contain 1 to 3 hetero atoms" represented by X' and Y' include groups resulting from removal of each of hydrogen atoms (2 in total) bound to the same or different carbon atom from a lower (C₁₋₆) hydrocarbon, and which may optionally contain hetero atoms selected from oxygen atom, NR^{24'} wherein R^{24'} is a hydrogen atom or a lower alkyl group, sulfur atom, and the like, in the hydrocarbon chain.

Examples of the lower alkyl group represented by R^{24'} include linear or branched C₁₋₆ alkyl group such as methyl, ethyl, propyl, isopropyl, butyl, isobutyl, sec-butyl, tert-butyl, pentyl, hexyl, and the like.

Specific examples of the "divalent linear lower hydrocarbon group" include (i) C₁₋₆ alkylene group (e.g. , -CH₂-, -(CH₂)₂-, -(CH₂)₃-, -(CH₂)₄-, -(CH₂)₅-, -(CH₂)₆-, and the like), (ii) C₂₋₆ alkenylene group (e.g., -CH=CH-, -CH=CH-CH₂-, -CH₂-CH=CH-CH₂-, -(CH₂)₂-CH=CH-CH₂-, -(CH₂)₂-CH=CH-(CH₂)₂-, -(CH₂)₃-CH=CH-CH₂-, and the like) and (iii) C₂₋₆ alkynylene group (e.g., -C≡C-, -C≡C-CH₂-, -CH₂-C≡C-CH₂-, -(CH₂)₂-C≡C-CH₂-, -(CH₂)₂-C≡ C-(CH₂)₂-, -(CH₂)₃-C≡C-CH₂-, and the like).

Examples of the "substituent" for the "divalent linear lower hydrocarbon group which may further contain 1 to 3 hetero atoms" represented by X' and Y' include the same as the "substituent" for the "aromatic group optionally having a substituent" represented by Ar¹¹ and Ar¹² above and oxo group and the like, and is preferably a hydroxy group or an oxo group.

X' is preferably a bond, an oxygen atom or NH, and a bond or an oxygen atom is particularly preferred.

Preferable examples of Y' include a group represented by the formula:

-(CH₂)ₛ-Y¹¹-(CH₂)ₜ-Y¹²-

wherein Y¹¹ and Y¹², whether identical or not, are a bond, an oxygen atom, S(O)_{q} wherein q has the same definition as that shown above, NR^{24'} wherein R^{24'} has the same definition as that shown above, a carbonyl group, a carbonyloxy group or a group represented by the formula: 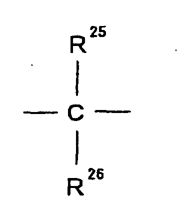 wherein R²⁵ and R²⁶, whether identical or not, are a hydroxy group or a C₁₋₄ alkyl group; s and t are independently an integer of 0 to 4 (sum of s and t is not more than 6).

Examples of the "C₁₋₄ alkyl group" represented by R²⁵ and R²⁶ include linear or branched C₁₋₄ alkyl group such as methyl, ethyl, propyl, isopropyl, butyl, isobutyl, sec-butyl, tert-butyl and the like.

Preferable examples of Y' include (i) C₁₋₆ alkylene groups, (ii) -(CH₂)ₚ₂O-, (iii) -(CH₂)ₚ₂NH-, (iv) -(CH₂)ₚ₂S-, (v) -(CH₂)_{q3}CH(OH)(CH₂)_{q4}O-, (vi) -(CH₂)_{q3}CH(OH)(CH₂)_{q4}NH-, (vii) -(CH₂)_{q3}CH(OH)(CH₂)_{q4}S-, (viii) -(CH₂)ₚ₂CONH-, (ix) -COO(CH₂)ₚ₂O-, (x) -COO (CH₂)ₚ₂NH-, (xi) -COO(CH₂)ₚ₂S-, (xii) -(CH₂)_{q3}O(CH₂)_{q4}O-, (xiii) -(CH₂)_{q3}O(CH₂)_{q4}NH- or (xiv) -(CH₂)_{q3}O(CH₂)_{q4}S- wherein p² is an integer of 1 to 6, q³ and q⁴ are an integer of 1 to 3.

In particular, Y' is preferably a bond, -(CH₂)₂-O-, -(CH₂)₃-O-, -(CH₂)₄-O-, -(CH₂)₆-O-, -(CH₂)₂-NH-, -(CH₂)₃-NH-, -(CH₂)₄-NH-, -(CH₂)₃-S-, -CH₂-CH(OH)-CH₂-O-, -(CH₂)₂-CO-NH-, -CH₂-CO-NH-, -CO-O-(CH₂)₂-O-, -CO-O- (CH₂)₃-O-, -(CH₂)₆-NH-, -(CH₂)₆-S-, -(CH₂)₂-O-(CH₂)₂-O-, -(CH₂)₂-O-(CH₂)₂-S-, and the like.

In the case of compound (IIa), Y' is preferably a group represented by the formula:

-(CH₂)ₛ-Y¹³-(CH₂)ₜ-Y¹⁴-

wherein Y¹³ is a bond or -CH(OH)-, Y¹⁴ is an oxygen atom, S or NH, and s and t independently are an integer of 0 to 4 (sum of s and t is not more than 6). In particular, s and t are preferably an integer of 1 to 3, and 3 is more preferred. When Y¹³ is -CH(OH)-, s and t are preferably 1.

In the case of Compound (IIb), Y' is preferably a group represented by the formula:

-(CH₂)_{w}-Y¹⁵-

wherein w is an integer of 1 to 6, and Y¹⁵ is an oxygen atom or NH. In particular, w is preferably an integer of 1 to 3, and 3 is more preferred.

In formula (II) above, R^{21a} is (1) a hydrogen atom, (2) a halogen atom, (3) a hydrocarbon group optionally having substituents, (4) an acyl group or (5) a hydroxy group having a substituent.

In formula (II) above, R^{21b} is (1) a hydrogen atom, (2) a halogen atom, (3) a hydrocarbon group optionally having substituents, (4) an acyl group or (5) a hydroxy group optionally having a substituent.

R²² and R²³, whether identical or not, are (1) a hydrogen atom, (2) a halogen atom, (3) a hydrocarbon group optionally having substituents, (4) an acyl group or (5) a hydroxy group optionally having a substituent.

Examples of the "halogen atom" represented by R^{21a}, R^{21b}, R²² and R²³ include a fluorine atom, a chlorine atom, a bromine atom and an iodine atom.

Examples of the "hydrocarbon group" of the "hydrocarbon group optionally having substituents" represented by R^{21a}, R^{21b}, R²² and R²³ include groups resulting from removal of one hydrogen atom from a hydrocarbon compound, specifically linear or cyclic hydrocarbon group such as alkyl group, alkenyl group, alkynyl group, cycloalkyl group, aryl group, aralkyl group, and the like. In particular, chain (linear or branched) or cyclic hydrocarbon groups, etc. having 1 to 16 carbon atoms are preferred, with greater preference given to
(a) alkyl group, preferably lower alkyl group (e.g., C₁₋₆ alkyl group such as methyl, ethyl, propyl, isopropyl, butyl, isobutyl, sec-butyl, tert-butyl, pentyl, hexyl, and the like),
(b) alkenyl group, preferably lower alkenyl group (e.g., C₂₋₆ alkenyl group such as vinyl, allyl, isopropenyl, butenyl, isobutenyl, sec-butenyl, and the like),
(c) alkynyl group, preferably lower alkynyl group (e.g., C₂₋₆ alkynyl group such as propargyl, ethynyl, butynyl, 1-hexynyl, and the like),
(d) cycloalkyl group, preferably lower cycloalkyl group (e.g., C₃₋₆ cycloalkyl group such as cyclopropyl, cyclobutyl, cyclopentyl, and cyclohexyl which may condense with a benzene ring optionally having 1 to 3 lower alkoxy groups (e.g., C₁₋₆ alkoxy groups such as methoxy and the like),
(e) aryl group (e.g., C₆₋₁₄ aryl group such as phenyl, tolyl, xylyl, biphenyl, 1-naphthyl, 2-naphthyl, 2-indenyl, 1-anthryl, 2-anthryl, 9-anthryl, 1-phenanthryl, 2-phenanthryl, 3-phenanthryl, 4-phenanthryl or 9-phenanthryl, preferably phenyl group), and
(f) aralkyl group (preferably lower aralkyl group (e.g., C₇₋₁₆ aralkyl group such as benzyl, phenethyl, diphenylmethyl, 1-naphthylmethyl, 2-naphthylmethyl, 2-phenylethyl, 2-diphenylethyl, 1-phenylpropyl, 2-phenylpropyl, 3-phenylpropyl, 4-phenylbutyl and 5-phenylpentyl and the like, more preferably benzyl group).

Examples of the "substituent" for said "hydrocarbon group" include the same as the "substituent" for the "aromatic group optionally having a substituent" represented by Ar¹¹ and Ar¹² above and the like.

In particular, examples of preferred hydrocarbon include alkyl group such as C₁₋₆ alkyl group, and examples of substituents for hydrocarbon group include cyano, carboxyl, C₁₋₆ alkoxy-carbonyl, carbamoyl (or thiocarbamoyl), and the like.

Examples of the "acyl group" represented by R^{21a}, R^{21b}, R²² and R²³ include groups represented by the formula -(C=O)-R²⁸, -SO₂-R²⁸, -SO-R²⁸, -( C=O)NR²⁸R²⁹, -(C=O)O-R²⁸, -(C=S)O-R²⁸ or -(C=S)NR²⁸R²⁹ wherein R²⁸ is a hydrogen atom, a hydrocarbon group optionally having substituents or a hydroxy group optionally having a substituent; and R²⁹ is a hydrogen atom or a lower alkyl group (e.g., C₁₋₆ alkyl group such as methyl, ethyl,' propyl, isopropyl, butyl, isobutyl, sec-butyl, tert-butyl, pentyl, hexyl, and the like, preferably a C₁₋₃ alkyl group such as methyl, ethyl, propyl, isopropyl, and the like).

In particular, groups represented by the formula -(C=O)-R²⁸, -SO₂-R²⁸, -SO-R²⁸, -(C=O)NR²⁸R²⁹ or -(C=O)O-R²⁸ are preferred, and a group represented by the formula -(C=O)-R²⁸ is more preferred.

The "hydrocarbon group which may be substituted" represented by R²⁸ is the same as the "hydrocarbon group optionally having a substituent" represented by R^{21a}, R^{21b}, R²² and R²³ above. In particular, the hydrocarbon group represented by R²⁸ is preferably an alkyl group such as a C₁₋₆ alkyl group, and the substituent thereof is preferably carboxyl, C₁₋₆ alkoxy-carbonyl, and the like. R²⁹ is preferably a hydrogen atom or the like.

Examples of the "hydroxy group having a substituent" represented by R^{21a} include hydroxy group having one group such as a hydrocarbon group optionally having substituents, instead of a hydrogen atom of the hydroxy group.

Examples of the "hydroxy group optionally having a substituent" represented by R^{21b}, R²², R²³ and R²⁸ include (1) a hydroxy group or (2) a hydroxy group having one group such as a hydrocarbon group optionally having substituents, instead of a hydrogen atom of the hydroxy group.

The "hydrocarbon group optionally having a substituent" present in the hydroxy group is the same as the "hydrocarbon group optionally having a substituent" represented by R^{21a}, R^{21b}, R²², R²³ and R²⁸ above.

With respect to compound (IIa), the acyl group represented by R^{21a}, R^{21b}, R²² and R²³ above is preferably (1) a carboxyl group, (2) a C₁₋₆ alkoxy-carbonyl group, (3) a carbamoyl group (or thiocarbamoyl group) which may be substituted by a C₁₋₆ alkyl group optionally having carboxyl or C₁₋₆ alkoxy-carbonyl, and the like.

In particular, R^{21a} is preferably (1) a hydrogen atom, (2) a carboxyl group, (3) a C₁₋₆ alkoxy-carbonyl group, (4) a C₁₋₆ alkyl group which may be substituted by a group selected from the group consisting of (i) cyano, (ii) carboxyl, (iii) C₁₋₆ alkoxy-carbonyl and (iv) carbamoyl (or thiocarbamoyl) or (5) a carbamoyl group (or thiocarbamoyl group) which may be substituted by a C₁₋₆ alkyl group optionally having carboxyl or C₁₋₆ alkoxy-carbonyl, or the like.

With respect to compound (IIb), when R^{21b} is a hydrogen atom, the oxo group of the triazolo[4,3-b]pyridazine ring may be enolated, and the partial structural formula: 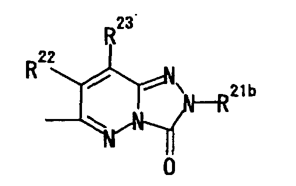 may represent any of the formula:

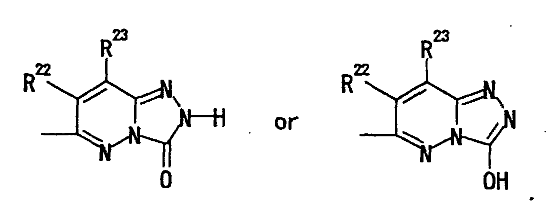

In particular, R^{21b} is preferably (1) a hydrogen atom, (2) a C₁₋₆ alkyl group which may be substituted by a group selected from the group consisting of (i) carboxyl, (ii) C₁₋₆ alkoxy-carbonyl, (iii) C₁₋₆ alkyl-carbonyloxy and (iv) C₁₋₆ alkyl-carbonyloxy-C₁₋₆ alkoxy-carbonyl, and the like.

With respect to formula (II) above, R²² and R²³ are preferably a hydrogen atom.

In formula (II) above, R²⁷ represents a hydrogen atom, a hydroxy group which may be substituted by a lower alkyl group or a carboxyl group.

Examples of the "lower alkyl group" of the "hydroxy group which may be substituted by a lower alkyl group" represented by R²⁷ include C₁₋₆ alkyl group such as methyl, ethyl, propyl, isopropyl, butyl, isobutyl, sec-butyl, tert-butyl, pentyl, hexyl, and the like.

R²⁷ is preferably a hydrogen atom or a hydroxy group, and a hydrogen atom is particularly preferred.

As compound (II) of the present invention, the following are preferred:

### Compound (II)-I:

Compound (II) wherein R^{21a} is (1) a hydrogen atom, (2) a carboxyl group, (3) a C₁₋₆ alkoxy-carbonyl group, (4) a C₁₋₆ alkyl group which may be substituted by a group selected from the group consisting of (i) cyano, (ii) carboxyl, (iii) C₁₋₆ alkoxy-carbonyl and (iv) carbamoyl or (5) a carbamoyl group which may be substituted by a C₁₋₆ alkyl group optionally having carboxyl or C₁₋₆ alkoxy-carbonyl; R^{21b} is (1) a hydrogen atom or (2) a C₁₋₆ alkyl group which may be substituted by a group selected from the group consisting of (i) carboxyl, (ii) C₁₋₆ alkoxy-carbonyl, (iii) C₁₋₆ alkyl-carbonyloxy and (iv) C₁₋₆ alkyl-carbonyloxy-C₁₋₆ alkoxy-carbonyl; R²² and R²³ are a hydrogen atom; R²⁷ is a hydrogen atom or a hydroxy group (particularly a hydrogen atom) ; Ar¹¹ and Ar¹² are independently a phenyl group which may be substituted; ring B' is a ring represented by the formula: 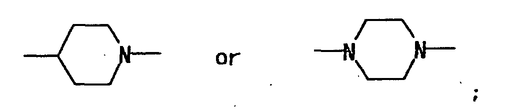 X' is a bond or an oxygen atom; Y' is a group represented by the formula:

-(CH₂)ₛ-Y¹³-(CH₂)ₜ-Y¹⁴-

wherein Y¹³ is a bond or -CH(OH)-, Y¹⁴ is an oxygen atom, S or NH, and s and t are independently an integer of 0 to 6 (sum of s and t is not more than 6).

### Compound (II)-II:

(1) 6-[6-[4-(diphenylmethoxy)piperidino]hexyloxy][1,2,4]triazolo[4,3-b]pyridazine or a salt thereof, (2) 6-[6-[4-(diphenylmethoxy)piperidino]hexylamino][1,2,4]triazolo(4,3-b] pyridazine or a salt thereof, (3) 3-tert-butyl-6-[3-[4-(diphenylmethoxy)piperidino]propoxy][1,2,4]triazolo[4,3-b]pyridazine or a salt thereof, (4) 6-[3-[4-(diphenylmethoxy)piperidino]propylamino][1,2,4]triazolo[4,3-b]pyridazine-3-carboxylic acid or a salt thereof, (5) 6-[3-[4-(diphenylmethoxy)piperidino]propylamino][1,2,4]triazolo[4,3-b]pyridazin-3(2H)-one or a salt thereof, (6) Ethyl 2-[6-[3-[4-(diphenylmethoxy)piperidino]propylamino]-3-oxo-[1,2,4]triazolo[4,3-b]pyridazin-2(3H)-yl]-2-methylpropionate or a salt thereof, (7) 2-[6-[3-[4-(diphenylmethoxy)piperidino]propylamino]-3-oxo-[1,2,4]triazolo[4,3-b]pyridazin-2(3H)-yl]-2-methylpropionic acid or a salt thereof, (8) Pivaloyloxymethyl 2-[6-[3-[4-(diphenylmethoxy)piperidino]propylamino]-3-oxo-[1,2,4]triazolo[4,3-b]pyridazin-2(3H)-yl]-2-methylpropionate or a salt thereof, (9) Pivaloyloxymethyl 2-[6-[3-[4-(diphenylmethoxy)piperidino]propoxy]-3-oxo-[1,2,4]triazolo[4,3-b]pyridazin-2(3H)-yl]-2-methylpropionate or a salt thereof.

As Compound (IIa) of the present invention, the following are preferred:

### Compound (IIa)-I:

Compound (IIa) wherein Ar¹¹ and Ar¹² are independently a phenyl group which may be substituted; ring B' is a ring represented by the formula: 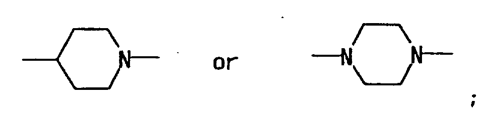 X' is a bond or an oxygen atom; Y' is a group represented by the formula:

-(CH₂)ₛ-Y¹³-(CH₂)ₜ-Y¹⁴-

wherein Y¹³ is a bond or -CH(OH)-, Y¹⁴ is an oxygen atom, S or NH, and s and t are independently an integer of 0 to 4 (sum of s and t is not more than 6);
R^{21a} is (1) a hydrogen atom, (2) a carboxyl group, (3) a C₁₋₆ alkoxy-carbonyl group, (4) a C₁₋₆ alkyl group which may be substituted by a group selected from the group consisting of (i) cyano, (ii) carboxyl, (iii) C₁₋₆ alkoxy-carbonyl and (iv) carbamoyl (or thiocarbamoyl), or (5) a carbamoyl group (or thiocarbamoyl group) which may be substituted by a C₁₋₆ alkyl group optionally having carboxyl or C₁₋₆ alkoxy-carbonyl; and R²² , R²³ and R²⁷ are a hydrogen atom.

### Compound (IIa)-II:

(1) 6-[6-[4-(diphenylmethoxy)piperidino]hexyloxy][1,2,4]triazolo[4,3-b]pyridazine or a salt thereof, (2) 6-[6-[4-(diphenylmethoxy)piperidino]hexylamino][1,2,4]triazolo[4,3-b]pyridazine or a salt thereof, (3) 3-tert-butyl-6-[3-[4-(diphenylmethoxy)piperidino]propoxy][1,2,4]triazolo[4,3-b]pyridazine or a salt thereof, (4) 6-[3-[4-(diphenylmethoxy)piperidino]propylamino][1,2,4]triazolo[4,3-b]pyridazine-3-carboxylic acid or a salt thereof.

As Compound (IIb) of the present invention, the following are preferred:

### Compound (IIb)-I:

Compound (IIb) wherein Ar¹¹ and Ar¹² are independently a phenyl group which may be substituted; ring B' is a ring represented by the formula: 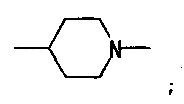 X' is an oxygen atom; Y' is a group represented by the formula:

-(CH₂)_{w}-Y¹⁵-

wherein w is an integer of 1 to 6, and Y¹⁵ is an oxygen atom or NH; R^{21b} is (1) a hydrogen atom or (2) a C₁₋₆ alkyl group which may be substituted by a group selected from the group consisting of (i) carboxyl, (ii) C₁₋₆ alkoxy-carbonyl, (iii) C₁₋₆ alkyl-carbonyloxy and (iv) C₁₋₆ alkyl-carbanyloxy-C₁₋₆ alkoxy-carbonyl; and R²², R²³ and R²⁷ are a hydrogen atom.

### Compound (IIb)-II:

(1) 6-[3-[4-(diphenylmethoxy)piperidino]propylamino][1,2,4]triazolo[4,3-b]pyridazin-3(2H)-one or a salt thereof, (2) Ethyl 2-[6-[3-[4-(diphenylmethoxy)piperidino]propylamino]-3-oxo-[1,2,4]triazolo[4,3-b]pyridazin-2(3H)-yl]-2-methylpropionate or a salt thereof, (3) 2-[6-[3-[4-(diphenylmethoxy)piperidino]propylamino]-3-oxo-[1,2,4]triazolo[4,3-b]pyridazin-2(3H)-yl]-2-methylpropionic acid or a salt thereof, (4) Pivaloyloxymethyl 2-[6-[3-[4-(diphenylmethoxy)piperidino]propylamino]-3-oxo-[1,2,4]triazolo[4,3-b]pyridazin-2(3H)-yl]-2-methylpropionate or a salt thereof, (5) Pivaloyloxymethyl 2-[6-[3-[4-(diphenylmethoxy)piperidino]propoxy]-3-oxo-[1,2,4]triazolo[4,3-b]pyridazin-2(3H)-yl]-2-methylpropionate or a salt thereof.

A prodrug for the above-mentioned compound (A), (I) or (II) or a salt thereof (hereinafter abbreviated as compound (A), (I) or (II)) may be a compound which is converted into compound (A), (I) or (II) under physiological conditions in vivo as a result of a reaction with an enzyme or gastric acid, thus a compound undergoing an enzymatic oxidation, reduction or hydrolysis to form compound (A), (I) or (II) and a compound hydrolyzed by gastric acid to form compound (A), (I) or (II).

A prodrug for compound (A), (I) or (II) is, for example, a compound obtained by subjecting an amino group in compound (A), (I) or (II) to an acylation, alkylation or phosphorylation (e.g., a compound obtained by subjecting an amino group in compound (A), (I) or (II) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation, etc.); a compound obtained by subjecting a hydroxy group in compound (A), (I) or (II) to an acylation, alkylation, phosphorylation or boration (e.g., a compound obtained by subjecting a hydroxy group in compound (A), (I) or (II) to an acetylation, palmitoylation, propanoylation, pivaloylation, succinylation, fumarylation, alanylation, dimethylaminomethylcarbonylation, etc.); a compound obtained by subjecting a carboxy group in compound (A), (I) or (II) to an esterification or amidation (e.g., a compound obtained by subjecting a carboxy group in compound (A) , (I) or (II) to an ethylesterification, phenylesterification, carboxymethylesterification, dimethylaminomethylesterification, pivaloyloxymethylesterification, ethoxycarbonyloxyethylesterification, phthalidylesterification, (5-methyl-2-oxo-1,3-dioxolen-4-yl)methylesterification, cyclohexyloxycarbonylethylesterification and methylamidation, etc.) and the like. Any of these compounds can be produced from compound (A), (I) or (II) by a method known per se.

A prodrug for compound (A), (I) or (II) may also be one which is converted into compound (A), (I) or (II) under a physiological condition, such as those described in "IYAKUHIN no KAIHATSU (Development of Pharmaceuticals)", Vol.7, Design of Molecules, p. 163-198, Published by HIROKAWA SHOTEN (1990).

The compound (I) or salt thereof can be produced in accordance with the known methods. For example, the compound (I) or salt thereof can be produced by the method described in JP-A-2000-191663, JP-A-2000-191664, JP-A-2000-198735 and WO 00/23450 or the applied methods thereof.

The compound (II) or salt thereof can be produced in accordance with the known methods. For example, the compound (II) or salt thereof can be produced by the method described in JP-A-2000-178277 and WO 00/20417 or the applied methods thereof.

Salts of the compound (A), (I) or (II) include, for example, salt with inorganic acid (e.g., hydrochloric acid, phosphoric acid, hydrobromic acid, sulfuric acid, and the like) and salt with organic acid (e.g., acetic acid, formic acid, propionic acid, fumaric acid, maleic acid, succinic acid, tartaric acid, citric acid, malic acid, oxalic acid, methanesulfonic acid, benzenesulfonic acid, and the like). Provided that the compound (A), (I) or (II) has an acidic group such as carboxylic acid and the like as a substituent thereof, the acidic group may form a salt with an inorganic base (e.g., an alkali metal or alkaline earth metal such as sodium, potassium, calcium, magnesium, and the like, or ammonia) or an organic base (e.g., tri-C₁₋₃ alkylamine such as triethylamine, and the like).

The compound (A), (I) or (II) of the present invention or a salt thereof or a pro-drug thereof (hereinafter, sometimes abbreviated as this compound) can be used as a safe drug, for example, a drug having JNK activation inhibitory activity, because it exhibits excellent JNK activation inhibitory activity with low toxicity and low side-effect.

JNK activation inhibitor of this invention comprising this compound exhibits excellent JNK activation inhibitory activity in a mammal (e.g., mouse, rat, hamster, rabbit, cat, dog, bovine, sheep, monkey, human). And it is excellent in absorbability (in oral administration) and stability (in metabolism). Therefore, it can be used as a drug for preventing and/or treating JNK derived disease and c-Jun derived disease such as acute pancreatitis, chronic pancreatitis, adult dyspnea syndrome, pachyderma, lupus erythematosus profundus, chronic thyroid gland, Graves' disease, autoimmune gastritis, autoimmune neutropenia, thrombocytopenia, myasthenia gravis, multiple myeloma, acute myeloblastic leukemia, chronic sarcoma, chronic myelocytic. leukemia, metastatic melanoma, Kaposi's sarcoma, marasmic disease, Huntington's chorea, disease derived from ischemia and/or reperfusion of cerebral apoplexy, myocardial ischemia, ischemic heart disease, kidney ischemia, neovascular glaucoma, infantile angiosarcoma, vascularization, hypercardia, abnormal immune response, fever, cellular aging and apoptosis derived disease.

And this compound exhibits excellent TNF-α inhibitory activity with low toxicity and low side-effect. Therefore, it can be used as a safe drug, for example, a drug having TNF-α inhibitory activity.

TNF-α inhibitor of this invention comprising this compound exhibits excellent TNF-α inhibitory activity in mammals (e.g., mouse, rat, hamster, rabbit, cat, dog, bovine, sheep, monkey, human). And it is excellent in absorbability (in oral administration) and stability (in metabolism). Therefore, it can be used as a drug for preventing and/or treating TNF-α derived disease. The TNF-α derived (TNF-α induced) disease is the disease that is occurred in the presence of TNF-α and remedied by the TNF-α inhibitory effect. Examples of the disease are inflammatory disease (e.g., diabetic complication such as retinopathy, nephrosis, nerve disorder and great vessel disorder; arthritis such as rheumatoid arthritis, osteoarthritis, rheumatoid myelitis, gouty arthritis and osteomyelitis; lumbago; gout; inflammatory after operation and/or injury; remission of swelling; neuralgia; pharyngitis; cystitis; pneumonia; atopic dermatitis; inflammatory bowel disease such as Crohn's disease, ulcerative colitis; meningitis; inflammatory ophthalmic disease; inflammatory pulmonary disease such as pneumonia, silicosis, pulmonary sarcoidosis and pulmonary tuberculosis); circulatory disease (e.g., angina pectoris, cardiac infarction, congestive heart failure, disseminated intravascular coagulation, and the like); diabetic nephropathy; asthma; allergic disease; chronic obstructive pulmonary disease; systematic lupus erythematosus; Crohn's disease; autoimmune hemolytic anemia; psoriasis; nervous degenerative disease (e.g., Alzheimer disease, Parkinson's disease, amyotrophic lateral sclerosis and AIDS encephalopathy); central nervous disorder (e.g., cerebrovascular disorder such as cerebral hemorrhage and cerebral infarction; cephalic trauma; spine damage; cerebral edema and multiple scleroma); toxemia (e.g., sepsis; septic shock; endotoxin shock; gram negative sepsis and toxin shock syndrome); Addison's disease; Creutzfeldt-Jakob disease; virus infection (e.g., cytomegalovirus; influenza virus and herpesvirus), suppression of rejection in transplantation and hypotension in dialysis.

This compound provides eosinophilic infiltration inhibitory effect in accordance with JNK activation inhibitory effect and TNF-α inhibitory effect. And according to this effect, this compound can be used as a drug for improvement of snuff.

Furthermore, among this compound, the compound having anti-histamine effect and/or eosinophilic infiltration inhibitory effect, etc. in addition to JNK activation inhibitory effect and TNF-α inhibitory effect is preferable for the purpose of this invention.

Thus, the compound which may have anti-histamine effect and/or eosinophilic infiltration inhibitory effect, etc. in addition to JNK activation inhibitory effect and TNF-α inhibitory effect exhibits low toxicity and few side-effect. Therefore it can be used as a safe pharmaceutical such as JNK activation inhibitor, anti-histamic agent, eosinophilic infiltration inhibitor and anti-allergy agent and the like.

The pharmaceutical composition of this invention comprising the compound having anti-histamine effect and/or eosinophilic infiltration inhibitory effect, etc. in addition to JNK activation inhibitory effect and TNF-α inhibitory effect exhibits an excellent JNK activation inhibitory effect, anti-histamic effect, eosinophilic infiltration inhibitory effect and anti-allergy effect to a mammal (e.g., mouse, rat, hamster, rabbit, cat, dog, bovine, sheep, monkey and human, etc.), and it is superior in absorption (in oral administration) and stability (in metabolism). Therefore it can be used as a drug for preventing and/or treating chronic urticaria, atopic dermatitis, allergic rhinitis, allergic conjunctivitis, hypersensitivity pneumonitis, eczema, dermatitis herpetiformis, psoriasis, eosinophilic pneumonia (PIE syndrome), chronic obstructive pulmonary disease (COPD), asthma, contact dermatitis, itching, dry dermatitis, acute urticaria and prurigo; and a drug for improving snuff.

The preparation of this invention comprising this compound can be administered orally or parenterally (e.g., topical, rectal, intravenous administration, etc.) with safe as pharmaceutical preparation such as tablet (including sugar-coated tablet, film-coated tablet), powder, granule, capsule (including soft capsule), liquid preparation, injection, suppository, sustained release preparation, etc., which is produced in accordance with the known method per se generally used in the pharmaceutical preparation production by using this compound as it is or mixing with pharmacologically acceptable carriers. The amount of this compound in the pharmaceutical preparation of this invention is about 0.01 to about 100% by weight based on the entire preparation, preferably about 0.1 to about 50% by weight, more preferably about 0.5 to about 20% by weight. While the dose of such a preparation may vary depending on the objective animal, administration route, objective disease, symptom and the like, a daily dose in a patient (body weight: about 60 kg) is usually about 0.01 to about 30 mg/kg as an active ingredient (this compound), preferably about 0.1 to about 20 mg/kg, more preferably about 1 to about 20 mg/kg, which is given orally once or in some portions a day as a drug for preventing and/or treating c-Jun related disease.

The pharmacologically acceptable carrier optionally employed for producing the preparation of this invention may for example be the conventional organic or inorganic carrier as materials for preparation, such as excipient, lubricant, binder and disintegrator in solid preparation; solvent, solubilizer, suspending agent, isotonicity agent, buffer agent and painkiller in liquid preparation and the like. Furthermore, if necessary, additives such as ordinary antiseptic, antioxidant, colorant, edulcorant, adsorbent, madidans agent and the like can be used properly.

Examples of the excipient include lactose, sucrose, D-mannitol, starch, corn starch, crystalline cellulose, light anhydrous silicic acid, and the like.

Examples of the lubricant include magnesium stearate, calcium stearate, talc and colloidal silica, and the like.

Examples of the binder include crystalline cellulose, sucrose, D-mannitol, dextrin, hydroxypropylcellulose, hydroxypropylmethylcellulose, polyvinylpyrrolidone, starch, sucrose, gelatin, methylcellulose, sodium carboxymethylcellulose, and the like.

Examples of the disintegrator include starch, carboxymethylcellulose, calcium carboxymethylcellulose, sodium carboxymethylstarch, L-hydroxypropylcellulose, and the like.

Examples of the solvent include water for injection, alcohol, propyleneglycol, macrogol, sesame oil, corn oil, olive oil, and the like.

Examples of the solubilizer include polyethyleneglycol, propyleneglycol, D-mannitol, benzyl benzoate, ethanol, trisaminomethane, cholesterol, triethanolamine, sodium carbonate, sodium citrate, and the like.

Examples of suspending agent include surfactant such as stearyl triethanolamine, sodium lauryl sulfate, laurylaminopropionic acid, lecithin, benzalkonium chloride, benzethonium chloride, glyceryl monostearate and the like; hydrophilic polymer such as polyvinyl alcohol, polyvinyl pyrrolidone, sodium carboxymethylcellulose, methylcellulose, hydroxymethylcellulose, hydroxyethylcellulose, hydroxypropylcellulose and the like.

Examples of isotonicity agent include glucose, D-sorbitol, sodium chloride, glycerin, D-mannitol, and the like.

Examples of buffer agent include buffer such as phosphate, acetate, carbonate, citrate and the like.

Examples of painkiller include benzyl alcohol, and the like.

Examples of antiseptic include p-hydroxybenzoic acid esters, chlorobutanol, benzyl alcohol, phenethyl alcohol, dehydroacetic acid, sorbic acid, and the like.

Examples of antioxidant include sulfite, ascorbic acid, α-tocopherol, and the like.

Furthermore, this compound can be used as a drug for allergy in combination with other anti-histamic agent.

Examples of the anti-histamic agent include chlorpheniramine maleate, diphenhydramine hydrochloride, dimenhydrinate, cyproheptadine hydrochloride, promethazine hydrochloride, alimemazine tartrate, clemastine fumarate, hydroxyzine pamoate, homochlorcyclizine hydrochloride, terfenadine, mequitazine, and the like.

And this compound can be used in combination with other drugs. Examples of the drugs used in combination with this compound include immunomodulatory drug (immunosuppressant), steroid, and the like. Examples of immunomodulatory drug (immunosuppressant) include ethyl 6,7-dimethoxy-4-(3,4-dimethoxyphenyl)-2-(1,2,4-triazol-1-ylmethyl)quinoline-3-carboxylate (JP-H07-A-118266), methotrexate, cyclophosphamide, MX-68, atiprimode dihydrochloride, BMS-188667, CKD-461, remexolone, ciclosporin, tacrolimus, gusperimus, azathioprine, anti-lymph serum, freeze-dried sulfonated human normal immunoglobulin, erythropoietin, colony stimulating factor, interleukin, interferon and the like. Examples of steroid include dexamethasone, hexestrol, methimazole, betamethasone, triamcinolone, triamcinolone acetonide, fluocinonide, fluocinolone acetonide, prednisolone, methylprednisolone, cortisone acetate, hydrocortisone, fluorometholone, beclometasone dipropionate, estriol, and the like.

The following effects can be obtained by combining this compound and the other drug:
(1) The amount of dose can be reduced compared to the condition that this compound or the other drug is used alone;
(2) In accordance with the patient's condition (mild case or serious case), the other drug can be selected;
(3) By selecting the other drug having different functional mechanism to this compound, the remedy term can be extended;
(4) By selecting the other drug having different functional mechanism to this compound, the remedy effect can be sustained;
(5) By combining this compound with the other drug, synergistic effect can be obtained.

Especially, when the other drug is a steroid, the mild class steroid can be used compared to the condition that the steroid is used alone.

Hereinafter, using this compound in combination with the other drug is denoted "concomitant medicament of this invention".

When using the concomitant medicament of this invention, the administration time is not limited. This compound or pharmaceutical composition thereof and the other drug or pharmaceutical composition thereof may be administered to the target simultaneously or separately. The dosage of the other drug is selected depending on the target, administration route, disease, combination, etc. and according to the dosage clinically used.

The administration mode of concomitant medicament of this invention is not particularly limited, provided that this compound and the other drug are combined upon administration. Such an administration mode may for example be (1) an administration of a single preparation obtained by formulating this compound and the other drug simultaneously, (2) a simultaneous administration via an identical route of two preparations obtained by formulating this compound and the other drug separately, (3) a sequential and intermittent administration via an identical route of two preparations obtained by formulating this compound and the other drug separately, (4) a simultaneous administration via different routes of two preparations obtained by formulating this compound and the other drug separately, (5) a sequential and intermittent administration via different routes of two preparations obtained by formulating this compound and the other drug separately (for example, this compound followed by the other drug, and vice versa) and the like.

The concomitant medicament of this invention has a low toxicity, and thus this compound and/or the other drug described above are mixed with a pharmacologically acceptable carrier in accordance with a method known per se to form a pharmaceutical composition, for example, a tablet (including sugar-coated tablet and film-coated tablet), powder, granule, capsule (including softcapsule), solution, injection formulation, suppository, sustained release formulation and the like, which can safely be given orally or parenterally (e.g., topical, rectal, intravenous administration etc.). An injection formulation may be given intravenously, intramuscularly, subcutaneously, into an organ or directly into a lesion.

The pharmacologically acceptable carrier which may be employed for producing the concomitant medicament of this invention may for example be the conventional organic or inorganic carrier as materials for preparation, such as excipient, lubricant, binder and disintegrator in solid preparation; solvent, solubilizer, suspending agent, isotonicity agent, buffer agent and painkiller in liquid preparation and the like. Furthermore, if necessary, additives such as ordinary antiseptic, antioxidant, colorant, edulcorant, adsorbent, madidans agent and the like can be used properly.

Examples of the excipient include lactose, sucrose, D-mannitol, starch, corn starch, crystalline cellulose, light anhydrous silicic acid, and the like.

Examples of the lubricant include magnesium stearate, calcium stearate, talc and colloidal silica, and the like.

Examples of the binder include crystalline cellulose, sucrose, D-mannitol, dextrin, hydroxypropylcellulose, hydroxypropylmethylcellulose, polyvinylpyrrolidone, starch, sucrose, gelatin, methylcellulose, sodium carboxymethylcellulose, and the like.

Examples of the disintegrator include starch, carboxymethylcellulose, calcium carboxymethylcellulose, sodium carboxymethylstarch, L-hydroxypropylcellulose, and the like.

Examples of the solvent include water for injection, alcohol, propyleneglycol, macrogol, sesame oil, corn oil, olive oil, and the like.

Examples of the solubilizer include polyethyleneglycol, propyleneglycol, D-mannitol, benzyl benzoate, ethanol, trisaminomethane, cholesterol, triethanolamine, sodium carbonate, sodium citrate, and the like.

Examples of suspending agent include surfactant such as stearyl triethanolamine, sodium lauryl sulfate, laurylaminopropionic acid, lecithin, benzalkonium chloride, benzethonium chloride, glyceryl monostearate and the like; hydrophilic polymer such as polyvinyl alcohol, polyvinyl pyrrolidone, sodium carboxymethylcellulose, methylcellulose, hydroxymethylcellulose, hydroxyethylcellulose, hydroxypropylcellulose and the like.

Examples of isotonicity agent include glucose, D-sorbitol, sodium chloride, glycerin, D-mannitol, and the like.

Examples of buffer agent include buffer such as phosphate, acetate, carbonate, citrate and the like.

Examples of painkiller include benzyl alcohol, and the like.

Examples of antiseptic include p-hydroxybenzoic acid esters, chlorobutanol, benzyl alcohol, phenethyl alcohol, dehydroacetic acid, sorbic acid, and the like.

Examples of antioxidant include sulfite, ascorbic acid, α-tocopherol, and the like.

The ratio between this compound and the other drug in the concomitant medicament of this invention may be selected appropriately on the basis of the target, route and disease, etc.

For example, the amount of this compound contained in the concomitant medicament of this invention is usually about 0.01 to 100% by weight based on the entire preparation, preferably about 0.1 to about 50% by weight, more preferably about 0.5 to about 20% by weight, although it may vary depending on the dosage form.

The amount of the other drug contained in the concomitant medicament of this invention is usually about 0.01 to 100% by weight based on the entire preparation, preferably about 0.1 to about 50% by weight, more preferably about 0.5 to about 20% by weight, although it may vary depending on the dosage form.

The amount of the additive such as a carrier contained in the concomitant medicament of this invention is usually about 1 to 99.99% by weight based on the entire preparation, preferably about 10 to 90% by weight, although it may vary depending on the dosage form.

Similar amounts may be employed also when this compound and the other drug are formulated separately.

Such a formulation can be produced by a method known per se which is employed usually in a pharmaceutical process (e.g. the process described in Japanese Pharmacopoeia).

Specifically, tablets can be produced by granulating a pharmaceutical as it is, or in a uniform mixture with excipients, binders, disintegrators and other appropriate additives, by an appropriate method, then adding lubricants etc., and subjecting the mixture to compressive shaping, or by subjecting to direct compressive shaping a pharmaceutical as it is, or in a uniform mixture with excipients, binders, disintegrators and other appropriate additives, or subjecting to compressive shaping previously prepared granules as they are, or in a uniform mixture with appropriate additives. These tablets may incorporate colorants, correctives etc. as necessary, and may be coated with appropriate coating agents.

Injectable preparations can be produced by dissolving, suspending or emulsifying a given amount of a pharmaceutical in an aqueous solvent such as water for injection, physiological saline or Ringer's solution, or a non-aqueous solvent such as a vegetable oil, and diluting to a given amount, or transferring a given amount of a pharmaceutical into a container for injection and sealing the container.

Examples of the carriers for oral preparations are substances in common use in pharmaceutical production, such as starch, mannitol, crystalline cellulose and carboxymethyl cellulose sodium. Examples of the carriers for injectable preparations are distilled water, physiological saline, glucose solutions and transfusions. Other additives in common use for pharmaceutical production can also be added, as appropriate.

Depending on the kind of this compound, patient age, body weight, symptoms, dosage form, route and frequency of administration and other factors, the daily dose of the concomitant medicament of this invention is normally about 0.1 to about 100 mg/kg, preferably about 1 to about 50 mg/kg, and more preferably about 1 to about 10 mg/kg, based on daily dose of this compound and the other drug, once or in some portions daily for each asthmatic patient (adult, body weight: about 60 kg). Of course, the amount of the dose changes in accordance with several conditions as described above. Therefore in some situations, lower dose is sufficient, and the other situation, more than the amount of the dose described above is necessary.

Any amount of the other drug can be selected as far as the side effect is not observed and the purpose of this invention is achieved. The daily dose of the other drug is different according to the level of symptom, age, sex, body weight, sensitivity, administration time, administration interval, property of the formulation, preparation, kind, ingredient and the like. Therefore, it is not limited especially.

In administering the concomitant medicament of this invention, this compound and the other drug may be administered at the same time. And this compound may be administered after administering the other drug, and vice versa. When administering the one after administering another, the timelag depends on the ingredient, dosage form and administering method. For example, when this compound is administered after administering the other drug, this compound is administered 1 minute to 3 days, preferably 10 minutes to 1 day, more prefereably 15 minutes to 1 hour after administering the other drug. And when the other drug is administered after administering this compound, the other drug is administered 1 minute to 1 day, preferably 10 minutes to 6 hours, more prefereably 15 minutes to 1 hour after administering this compound.

The present invention is hereinafter described in more detail by means of the following reference examples, examples and experimental examples, which are not to be construed as limitative.

In the reference examples below, the fraction containing the desired product was detected by observation via TLC (Thin Layer Chromatography). In the TLC observation, 60F₂₅₄, produced by Merck, was used as a TLC plate, with a UV detector as a means of detection.

### EXAMPLES

### Reference Example 1

### Production of 6-[3-[4-(diphenylmethoxy)piperidino]propylamino][1,2,4]triazolo[1,5-b]pyridazine

6-(3-Hydroxypropylamino)[1,2,4]triazolo[1,5-b]pyridazine (290 mg) was suspended in tetrahydrofuran (10 mL). N-ethyldiisopropylamine (388 mg) and methanesulfonyl chloride (344 mg) were added to the suspension, and the mixture was stirred at room temperature for one hour. Ice-water and sodium chloride were added to the mixture, followed by extraction with ethyl acetate. The extract was washed with saturated brine, dried over magnesium sulfate and concentrated under reduced pressure. The residue was dissolved in N,N-dimethylformamide (5 mL), followed by addition of 4-(dipheylmethoxy)piperidine (352 mg), sodium iodide (208 mg) and potassium carbonate (193 mg). The mixture was stirred at room temperature for 15 hours and at 60°C for 3 hours. After the mixture was cooled, ice-water was added thereto, followed by extraction with ethyl acetate. The extract was washed with saturated brine, dried over magnesium sulfate and concentrated under reduced pressure. The residue was subjected to silica gel column chromatography and eluted with a mixture of ethyl acetate, methanol and triethylamine (90:10:1). Fractions containing the objective compound were concentrated. The resulting crystals were washed with ethyl ether and dried to yield the title compound (209 mg).
m.p. 136-138°C

| Elemental Analysis for C₂₆H₃₀N₆O | | | |
|---|---|---|---|
| Calculated (%) | C, 70.56; | H, 6.83; | N, 18.99 |
| Found (%) | C, 70.43; | H, 6.83; | N, 19.04 |

### Reference Example 2

### Production of ethyl 2-[6-[3-[4-(diphenylmethoxy)piperidino]propylamino]imidazo[1,2-b]pyridazin-2-yl]-2-methylpropionate difumarate

4-(Diphenylmethoxy)-1-piperidinepropanamine (4.2 g) and ethyl 2-(6-chloroimidazo[1,2-b]pyridazin-2-yl)-2-methylpropionate (1.76 g) were stirred at 190-200°C for 3.5 hours. After cooling, aqueous sodium bicarbonate was added, followed by extraction with ethyl acetate; the extract was washed with saturated brine, dried over magnesium sulfate and concentrated under reduced pressure. The residue was subjected to silica gel column chromatography and eluted with ethyl acetate:methanol:triethylamine (100:5:1). The desired fraction was collected, dissolved in 16 mL of ethyl acetate; a solution of 867 mg of fumaric acid in 16 mL of methanol was added, followed by concentration. Acetone was added to the residue; the crystal precipitated was collected by filtration, washed with acetone and dried to yield 2.30 g of the title compound.
Melting point : 126-128°C

| Elemental analysis for C₄₁H₄₉N₅O₁₁ | | | |
|---|---|---|---|
| Calculated (%) | C, 62.50; | H, 6.27; | N, 8.89 |
| Found (%) | C, 62.28; | H, 6.15; | N, 8.97 |

### Reference Example 3

### Production of 2-[6-[3-[4-(diphenylmethoxy)piperidino]propylamino]imidazo[1,2-b]pyridazin-2-yl]-2-methylpropionic acid

Ethyl 2-[6-[3-[4-(diphenylmethoxy)piperidino]propylamino]imidazo[1,2-b]pyridazin-2-yl]-2-methylpropionate (468 mg) was dissolved in ethanol (3 mL); 1 N aqueous solution of sodium hydroxide (2 mL) was added, followed by stirring at room temperature for 15 hours. The mixture was concentrated under reduced pressure. The residue was diluted with water and washed with ethyl acetate; the water layer was adjusted to pH 7 by the addition of 1 N hydrochloric acid, followed by extraction with ethyl acetate-tetrahydrofuran (1:1); the extract was washed with saturated brine, dried over magnesium sulfate and concentrated under reduced pressure. Ethyl acetate was added to the residue; the crystal precipitated was collected by filtration, washed with ethyl acetate and dried to yield the title compound (267 mg), which can be recrystallized from acetone.
Melting point : 205-206°C

| Elemental analysis for C₃₁H₃₇N₅O₃ | | | |
|---|---|---|---|
| Calculated (%) | C, 70.56; | H, 7.07; | N, 13.27 |
| Found (%) | C, 70.46; | H, 7.06; | N, 13.36 |

### Reference Example 4

### Production of N-[6-[3-[4-(diphenylmethoxy)piperidino]propylamino]imidazo[1,2-b]pyridazine-2-carbonyl]glycine ethyl ester dihydrochloride

N-[6-[3-[4-(diphenylmethoxy)piperidino]propylamino]imidazo[1,2-b]pyridazine-2-carbonyl]glycine ethyl ester (0.628 g) was dissolved in tetrahydrofuran (10 mL); 4 N hydrogen chloride solution in ethyl acetate (1.5 mL) was added, followed by concentration under reduced pressure. To the residue, methanol (10 mL) was added, followed by concentration under reduced pressure. The crystal obtained was collected and washed with ethyl acetate to yield the title compound (0.658 g).
Melting point : 205°C

| Elemental analysis for C₃₂H₄₀N₆O₄Cl₂ | | | |
|---|---|---|---|
| Calculated (%) | C, 59.72; | H, 6.26; | N, 13.06 |
| Found (%) | C, 59.74; | H, 6.41; | N, 12.63 |

### Reference Example 5

### Production of ethyl 2-[6-[3-[4-(diphenylmethoxy)piperidino]propylamino]-3-methylimidazo[1,2-b]pyridazin-2-yl]-2-methylpropionate dihydrochloride

4-(Diphenylmethoxy)-1-piperidinepropanamine (2.38 g) and ethyl 2-(6-chloro-3-methylimidazo[1,2-b]pyridazin-2-yl)-2-methylpropionate (1.03 g) were stirred at 160°C for 7.5 hours. After cooling, aqueous sodium bicarbonate was added, followed by extraction with ethyl acetate; the extract was washed with saturated brine, dried over magnesium sulfate and concentrated under reduced pressure. The residue was subjected to silica gel column chromatography and eluted with ethyl acetate : methanol : triethylamine (50:5:1). The desired fraction was collected, concentrated under reduced pressure and dissolved in ethyl acetate (5 mL); 4 N solution of hydrogen chloride in ethyl acetate (0.96 mL) was added, followed by concentration again. The residue was powdered by the addition of ethyl ether, collected by filtration and dried to yield the title compound (666 mg) .

### Amorphous

| Elemental analysis for C₃₄H₄₅N₅O₃Cl₂·1.5H₂O | | | |
|---|---|---|---|
| Calculated (%) | C, 60.98; | H, 7.22; | N, 10.46 |
| Found (%) | C, 60.70; | H, 6.95; | N, 10.34 |

### Reference Example 6

### Production of ethyl 2-[6-[3-[4-(diphenylmethylamino)piperidino]propylamino]imidazo[1,2-b]pyridazin-2-yl]-2-methylpropionate

4-(Diphenylmethylamino)-1-piperidinepropanamine (3.12 g) and ethyl 2-(6-chloroimidazo[1,2-b]pyridazin-2-yl)-2-methylpropionate (1.72 g) were stirred at 180°C for 3 hours. After cooling, aqueous sodium bicarbonate was added, followed by extraction with ethyl acetate; the extract was washed with saturated brine, dried over magnesium sulfate and concentrated under reduced pressure. The residue was subjected to silica gel column chromatography and eluted with ethyl acetate : methanol : triethylamine (50:5:1). The desired fraction was collected and concentrated under reduced pressure. The residue was crystallized by the addition of ethyl ether-hexane (1:3), collected by filtration, washed with hexane and dried to yield the title compound (1.83 g).
Melting point : 115-117°C

| Elemental analysis for C₃₃H₄₂N₆O₂ | | | |
|---|---|---|---|
| Calculated (%) | C, 71.45; | H, 7.63; | N, 15.15 |
| Found (%) | C, 71.40; | H, 7.70; | N, 14.94 |

### Reference Example 7

### Production of ethyl 2-[6-[3-[4-(diphenylmethoxy)piperidino]propylamino]imidazo[1,2-b]pyridazin-2-yl]-2-methylpropionate disuccinate

In ethanol (1 mL), ethyl 2-[6-[3-[4-(diphenylmethoxy)piperidino]propylamino]imidazo[1,2-b]pyridazin-2-yl]-2-methylpropionate (0.278 g) produced in Reference Example 2 was dissolved, and succinic acid (0.118 g) was added thereto and dissolved, followed by concentration under reduced pressure. To the residue was added tetrahydrofuran (0.5 mL) and the residue was dissolved. After addition of ethyl acetate (2 mL), the crystals formed were collected by filtration, washed with ethyl acetate and dried to yield the title compound (0.382 g).
Melting point 98-101°C (decomposed)

| Elemental analysis: for C₄₁H₅₃N₅O₁₁·1/3CH₃CO₂C₂H₅ | | | |
|---|---|---|---|
| Calculated (%) | C, 61.92 ; | H, 6.83 ; | N, 8.53 |
| Found (%) | C, 61.54 ; | H, 6.83 ; | N, 8.50 |

### Reference Example 8

### Production of ethyl 2-[6-[3-[4-(diphenylmethoxy)piperidino]propylamino]imidazo[1,2-b]pyridazin-2-yl]-2-methylpropionate citrate (1:1)

In ethanol (8 mL), ethyl 2-[6-[3-[4-(diphenylmethoxy)piperidino]propylamino]imidazo[1,2-b]pyridazin-2-yl]-2-methylpropionate (1.667 g) was dissolved, and citric acid monohydrate (0.631 g) was added thereto and dissolved under heating, followed by concentration under reduced pressure. To the residue was added ethyl acetate (23 mL), and the crystals formed were collected by filtration and washed with ethyl acetate (12 mL). To the crystals was added methanol (30 mL) and the crystals were dissolved under heating, followed by concentration under reduced pressure. To the residue was added ethanol (30 mL) and the residue was then dissolved. After standing, the crystals formed were collected by filtration, washed with ethanol (10 mL) and dried to yield the title compound (2.01 g).
Melting point: 176°C (decomposed)

| Elemental analysis for C₃₉H₄₉N₅O₁₀ | | | |
|---|---|---|---|
| Calculated (%) | C, 62.64 ; | H, 6.60 ; | N, 9.36 |
| Found (%) | C, 62.50 ; | H, 6.56 ; | N, 9.43 |

### Reference Example 9

### Production of 2-[6-[3-[4-(diphenylmethoxy)piperidino]propylamino]imidazo[1,2-b]pyridazin-2-yl]-2-methylpropionic acid dihydrate

In dimethyl sulfoxide (600 mL) were suspended 4-(diphenylmethoxy)-1-piperidinepropaneamine (363.6 g, 1120 mmol), ethyl 2-(6-chloroimidazo[1,2-b]pyridazin-2-yl)-2-methylpropionate (200.0 g, 747 mmol) and sodium carbonate (158.4 g, 1490 mmol), which were then heated in an oil bath (bath temperature 165-170°C) under a nitrogen gas stream and stirred for 3.5 hours. After cooling to room temperature, ethyl acetate (2000 mL) and water (2000 mL) were added, followed by separation into two layers. The organic layer was washed with water (1000 mL) twice and concentrated under reduced pressure. To the residue was added ethanol (1000 mL) and concentrated under reduced pressure to yield crude ethyl 2-[6-[3-[4-(diphenylmethoxy)piperidino]propylamino]imidazo[1,2-b]pyridazin-2-yl]-2-methylpropionate (588 g) as an oily material. This oily material was dissolved in ethanol (1400 mL), and sodium hydroxide (59.8 g, 1490 mmol) dissolved in water (600 mL) was added thereto. The reaction mixture was heated to 60°C (inner temperature) and stirred for 1 hour. The reaction solution was concentrated under reduced pressure. To the residue were added water (2000 mL) and ethyl acetate (2000 mL), followed by separation into two layers. The aqueous layer was washed with ethyl acetate (1000 mL) twice, and ethanol (2000 mL) was added to the aqueous layer. After the aqueous layer was adjusted to about pH 6 by the addition of 1N hydrochloric acid (1000 mL), the crystals formed were collected by filtration, washed with water (800 mL) and ethanol : water (1:1) (800 mL), and dried to yield the crude title compound (353.6 g). HPLC purity area percentage 97.7%, Yield 82.0%

To the crude title compound (353.6 g) thus obtained was added ethanol (1240 mL), and heated under reflux for 1 hour. The solution was stirred under ice-cooling. The crystals formed were collected by filtration, washed with cold ethanol (930 mL) and dried. 'The resulting crystals were suspended in water (2000 mL) and stirred for 1 hour while heating in a water bath (inner temperature 65-70°C). After cooling to room temperature, the crystals formed were collected by filtration, washed with water (1000 mL) and dried to yield the title compound (276 g).
Melting point 203-205°C (The crystals began to soften at 110-120 °C and solidified again.)

| Elemental analysis for C₃₁H₃₇N₅O₃·2H₂O | | | |
|---|---|---|---|
| Calculated (%) | C, 66.05 ; | H, 7.33 ; | N, 12.42 |
| Found (%) | C, 66.35 ; | H, 7.29 ; | N, 12.39 |

### Reference Example 10

### Production of 2-[6-[3-[4-(diphenylmethoxy)piperidino]propylamino]-3-methylimidazo[1,2-b]pyridazin-2-yl]-2-methylpropionic acid

In N-methyl-2-pyrrolidinone (25 mL) were dissolved 4-(diphenylmethoxy)-1-piperidinepropaneamine (17.0 g) and ethyl 2-(6-chloro-3-methylimidazo[1,2-b]pyridazin-2-yl)-2-methylpropionate (7.39 g), and stirred at 160°C for 8.5 hours. After cooling, water was added and extracted with ethyl acetate. The extract was washed with saturated brine and dried over magnesium sulfate. Following to concentration under reduced pressure, the residue was subjected to silica gel column chromatography to be eluted with ethyl acetate : methanol : triethylamine (50:5:1). The desired fraction was collected and concentrated to yield ethyl 2-[6-[3-[4-(diphenylmethoxy)piperidino]propylamino]-3-methylimidazo[1,2-b]pyridazin-2-yl]-2-methylpropionate (10.4 g) as an oily material. In ethanol (100 mL) was dissolved this oily material (10.4 g), and 5N aqueous sodium hydroxide solution (18.3 mL) was added thereto and heated under reflux for 2 hours. After cooling, the mixture was concentrated under reduced pressure. The residue was diluted with water and washed with diisopropyl ether. The aqueous layer was adjusted to pH 5 by the addition of 5N hydrochloric acid. The crystals formed were collected by filtration, recrystallized from methanol-water (10:1) and dried to yield the title compound (4.09 g).
Melting point 219-220°C

| Elemental analysis for C₃₂H₃₉N₅O₃ | | | |
|---|---|---|---|
| Calculated (%) | C, 70.95 ; | H, 7.26 ; | N, 12.93 |
| Found (%) | C, 70.85 ; | H, 7.00; | N, 13.20 |

### Reference Example 11

### Production of 6-[6-[4-(diphenylmethoxy)piperidino]hexyloxy][1,2,4]triazolo[4,3-b]pyridazine fumarate

4-(Diphenylmethoxy)-1-piperidinehexanol (825 mg) was dissolved in tetrahydrofuran (13 mL); sodium hydride (60% in oil, 108 mg) was added, followed by refluxing under heating for 1.5 hours. After cooling, 6-chloro[1,2,4]triazolo[4,3-b]pyridazine (347 mg) was added, followed by refluxing under heating for 2 hours. After cooling, water was added; the reaction mixture was extracted with ethyl acetate, washed with saturated brine, and'dried over magnesium sulfate. After concentration under reduced pressure, the residue was subjected to silica gel column chromatography and eluted with ethyl acetate-methanol-triethylamine (50:5:1). The desired fraction was collected and concentrated under reduced pressure; the residue (940 mg) was dissolved in ethanol; a solution of fumaric acid (225 mg) in ethanol was added, followed by concentration under reduced pressure. The residue was crystallized from ethanol-ethyl acetate (1:5), washed with ethyl acetate, and dried, to yield the title compound (939 mg). ¹H-NMR (CDCl₃) δ ppm : 1.30-1.55 (4H, m), 1.65-1.85 (4H, m), 1.85-2.20 (4H, m), 2.80-3.35 (5H, m), 3.73 (1H, brs), 4.27 (2H, t, J = 6.4 Hz), 5.44 (1H,s), 6.76(2H, S), 6.78 (1H, d, J = 9.6 Hz) , 7.25-7.35 (10H, m) , 7.94 (1H, d, J = 9. 8 Hz), 8.87 (1H, s).
Melting point : 144-146°C

| Elemental analysis for C₃₃H₃₉N₅O₆ | | | |
|---|---|---|---|
| Calculated (%) | C, 65.87; | H, 6.53; | N, 11.64 |
| Found (%) | C, 65.81; | H, 6.48; | N, 10.87. |

### Reference Example 12

### Production of 6-[6-[4-(diphenylmethoxy)piperidino]hexylamino][1,2,4]triazolo[4,3-b]pyridazine

### Process A: Production of 6-(6-hydroxyhexylamino)[1,2,4]triazolo[4,3-b]pyridazine

In ethanol (35 mL), 6-chloro[1,2,4]triazolo[4,3-b]pyridazine (3.55 g) was suspended; 6-aminohexanol (6.73 g) was added, followed by refluxing under heating for 16 hours. After cooling, the reaction mixture was concentrated under reduced pressure; the residue was subjected to silica gel column chromatography and eluted with ethyl acetate-methanol-triethylamine (50:5:1). The desired fraction was collected and concentrated under reduced pressure; the precipitated crystal was washed with diethyl ether and dried to yield the title compound (5.67 g).
¹H-NMR (CDCl₃) δ ppm : 1.38-1.80 (8H, m), 3.11 (1H, q, J = 6.4 Hz), 3.37 (2H, q, J = 6.4 Hz) , 3.67 (2H, t, J = 6.1 Hz), 5.12 (1H, brs), 6.61 (1H, d, J = 9.8 Hz), 7.78 (1H, d, J = 10.0 Hz), 8.74 (1H, s).

### Process B: Production of 6-[6-(methanesulfonyloxy)hexylamino][1,2,4]triazolo[4,3-b]pyridazine

6-(6-Hydroxyhexylamino)[1,2,4]triazolo[4,3-b]pyridazine (3.26 g) was suspended in tetrahydrofuran (60 mL); N-ethyldiisopropylamine (3.59 g) and methanesulfonyl chloride (3.17 g) were added, followed by stirring at room temperature for 4 hours. Brine was added; the reaction mixture was extracted with ethyl acetate-tetrahydrofuran (1:1) and dried over magnesium sulfate. After concentration under reduced pressure, the residue was subjected to silica gel column chromatography and eluted with ethyl acetate-methanol-triethylamine (50:5:1). The desired fraction was collected and concentrated under reduced pressure; the precipitated crystal was washed with diethyl ether, and dried, to yield the title compound (3.24 g).
Melting point : 103-105°C

| Elemental analysis for C₁₂H₁₉N₅O₃·0.3H₂O | | | |
|---|---|---|---|
| Calculated (%) | C, 45.21; | H, 6.20; | N, 21.97 |
| Found (%) | C, 45.15; | H, 6.24; | N, 21.60. |

### Process C:

6-[6-(Methanesulfonyloxy)hexylamino][1,2,4]triazolo[4,3-b]pyridazine (810 mg) was dissolved in N,N-dimethylformamide (15 mL); 4-(diphenylmethoxy)piperidine (829 mg), potassium carbonate (428 mg), and potassium iodide (515 mg) were added, followed by stirring at 60°C for 4 hours. After cooling, brine was added; the reaction mixture was extracted with ethyl acetate and dried over magnesium sulfate. After concentration under reduced pressure, the residue was subjected to silica gel column chromatography and eluted with ethyl acetate-methanol-triethylamine (50:5:1). The desired fraction was collected and concentrated under reduced pressure; the residue was crystallized from ethanol-ethyl acetate (1:5), washed with diethyl ether, recrystallized in the same manner, and dried to yield the title compound (599 mg).
Melting point : 124-127°C

| Elemental analysis for C₂₉H₃₆N₆O·0.5H₂O | | | |
|---|---|---|---|
| Calculated (%) | C, 70.56; | H, 7.55; | N, 17.02 |
| Found (%) | C, 70.68; | H, 7.29; | N, 17.38. |

### Reference Example 13

### Production of 3-tert-butyl-6-[3-[4-(diphenylmethoxy)piperidino]propoxy)[1,2,4]triazolo[4,3-b]pyridazine

4-(Diphenylmethoxy)-1-piperidinepropanol (991 mg) was dissolved in tetrahydrofuran (20 mL); sodium tert-butoxide (322 mg) was added, followed by stirring at an external temperature of 60°C for 2 hours. After cooling, 3-tert-butyl-6-chloro[1,2,4]triazolo[4,3-b]pyridazine (642 mg) was added, followed by refluxing under heating for 2 hours. After cooling, brine was added; the reaction mixture was extracted with ethyl acetate and dried over magnesium sulfate. After concentration under reduced pressure, the residue was subjected to silica. gel column chromatography and eluted with ethyl acetate-methanol-triethylamine (50:5:1). The desired fraction was collected and concentrated under reduced pressure; the residue (1.26 g) was dissolved in pyridine (3 mL); acetic anhydride (1.5 mL) was added, followed by stirring at room temperature for 6 hours. After the reaction mixture was concentrated under reduced pressure, the residue was subjected to silica gel column chromatography and eluted with ethyl acetate-methanol-triethylamine (50:5:1). The desired fraction was collected and concentrated under reduced pressure; the residue crystallized from ethyl acetate-diethyl ether (1:1), washed with diethyl ether and dried to yield the title compound (500 mg).
Melting point : 125-127°C

| Elemental analysis for C₃₀H₃₇N₅O₂ | | | |
|---|---|---|---|
| Calculated (%) | C, 72.12; | H, 7.46; | N, 14.02 |
| Found (%) | C, 71.93; | H, 7.47; | N, 14.18. |

### Reference Example 14

### Production of ethyl 6-[3-[4-(diphenylmethoxy)piperidino]propylamino][1,2,4]triazolo[4,3-b] pyridazine-3-carboxylate

4-(Diphenylmethoxy)-1-piperidinepropanamine (5.15 g) and ethyl 6-chloro[1,2,4]triazolo[4,3-b]pyridazine-3-carboxylate (3.6 g) were dissolved in N,N-dimethylformamide (70 mL); N-ethyldiisopropylamine (5.48 mL) was added, followed by stirring under heating at an external temperature of 70°C for 4 hours. After cooling, cold brine was added; the reaction mixture was extracted with tetrahydrofuran and dried over magnesium sulfate. After concentration under reduced pressure, the residue was subjected to silica gel column chromatography and eluted with ethyl acetate-methanol-triethylamine (50:5:1). The desired fraction was collected and concentrated under reduced pressure; half of the precipitated crystal was recrystallized from ethanol-ethyl acetate (1:1), collected by filtration, and dried, to yield the title compound (2.13 g).
Melting point : 129-133°C

| Elemental analysis for C₂₉H₃₄N₆O₃·0.3H₂O | | | |
|---|---|---|---|
| Calculated (%) | C, 66.98; | H, 6.71; | N, 16.16 |
| Found (%) | C, 67.09; | H, 6.88; | N, 16.02. |

### Reference Example 15

### Production of 6-[3-[4-(diphenylmethoxy)piperidino]propylamino][1,2,4]triazolo[4,3-b]pyridazine-3-carboxylic acid

Ethyl 6-[3-[4-(diphenylmethoxy)piperidino]propylamino] [1,2,4]triazolo[4,3-b]pyridazine-3-carboxylate (1.31 g) was dissolved in ethanol (8 mL); 1 N aqueous solution of sodium hydroxide (8 mL) was added, followed by refluxing under heating for 3 hours. After cooling, the ethanol was distilled off; the residue was washed with ethyl acetate; 1 N hydrochloric acid was added to reach pH 5. The precipitated crystal was collected by filtration, washed with water and ethyl acetate and dried to yield the title compound (1.19 g).
Melting point : 102-104°C

| Elemental analysis for C₂₇H₃₀N₆O₃·2H₂O | | | |
|---|---|---|---|
| Calculated (%) | C, 62.05; | H, 6.56; | N, 16.00 |
| Found (%) | C, 61.82; | H, 6.40; | N, 16.06. |

### Reference Example 16

### Production of 6-[3-[4-(diphenylmethoxy)piperidino]propylamino][1,2,4]triazolo[4,3-b]pyridazin-3(2H)-one

6-Chloro[1,2,4]triazolo[4,3-b]pyridazin-3(2H)-one (0.512 g) was dissolved in n-butanol (15 mL); 4-(diphenylmethoxy)-1-piperidinepropanamine (0.88 g) and N-ethyldiisopropylamine (1.04 mL) were added, followed by refluxing under heating 'in an oil bath for 5 hours. After cooling, the n-butanol was mostly distilled off under reduced pressure; to the residue, an aqueous solution of sodium hydrogen carbonate was added; the reaction mixture was extracted with ethyl acetate; the extract was washed with saturated brine and dried over magnesium sulfate. After concentration under reduced pressure, the residue was subjected to silica gel column chromatography and eluted with ethyl acetate-methanol-triethylamine (85:15:1). The desired fraction was collected and concentrated; the crystal obtained was filtered, washed with diethyl ether, and dried, to yield the title compound (0.35 g).
Melting point : 156-170°C

| Elemental analysis for C₂₆H₃₀N₆O₂·2H₂O | | | |
|---|---|---|---|
| Calculated (%) | C, 65.52; | H, 6.72; | N, 17.64 |
| Found (%) | C, 65.78; | H, 6.71; | N, 17.52 |

### Reference Example 17

### Production of ethyl 2-[6-[3-[4-(diphenylmethoxy)piperidino]propylamino]-3-oxo[1,2,4]triazolo[4,3-b]pyridazin-2(3H)-yl]-2-methylpropionate hydrochloride

6-[3-[4-(Diphenylmethoxy)piperidino]propylamino]-[1,2,4]triazolo[4,3-b]pyridazin-3(2H)-one (0.606 g) was suspended in N,N-dimethylformamide (3 mL); 60% oily sodium hydride (0.063 g) was added, followed by stirring at room temperature for 1 hour. Ethyl 2-bromoisobutyrate (0.253 mL) was added, followed by stirring in an oil bath (bath temperature 80°C) for 17 hours. After cooling, 60% oily sodium hydride (0.063 g) and ethyl 2-bromoisobutyrate (0.253 mL) were added, followed by stirring in an oil bath (bath temperature 80°C) for 3 hours. After cooling, ice water was added; the reaction mixture was extracted with ethyl acetate; the extract was washed with saturated brine and dried over magnesium sulfate. After concentration under reduced pressure, the residue was subjected to silica gel column chromatography and eluted with ethyl acetate-methanol-triethylamine (90:10:1). The desired fraction was collected and concentrated to yield ethyl 2-[6-[3-[4-(diphenylmethoxy)piperidino]propylamino]-3-oxo[1,2,4]triazolo[4,3-b]pyridazin-2(3H)-yl]-2-methylpropionate (0.38 g) as an oily substance. This oily substance was dissolved in ethyl acetate (5 mL); 4 N solution of hydrogen chloride in ethyl acetate (0.5 mL) was added, followed by concentration; the residue was powdered from diethyl ether, collected by filtration and dried to yield the title compound (0.342 g).
Melting point : 162°C

| Elemental analysis for C₃₂H₄₀N₆O₄·HCl·(C₂H₅)₂O | | | |
|---|---|---|---|
| Calculated (%) | C, 61.83; | H, 7.35; | N, 12.02 |
| Found (%) | C, 62.16; | H, 7.02; | N, 12.15 |

### Reference Example 18

### Production of 2-[6-[3-[4-(diphenylmethoxy)piperidino]propylamino]-3-oxo[1,2,4]triazolo[4,3-b]pyridazin-2(3H)-yl]-2-methylpropionic acid

Ethyl 2-[6-[3-[4-(diphenylmethoxy)piperidino]propylamino]-3-oxo[1,2,4]triazolo[4,3-b]pyridazin-2(3H)-yl]-2-methylpropionate (0.50 g) was dissolved in ethanol (3 mL); 1 N aqueous solution of sodium hydroxide (2.2 mL) was added, followed by stirring at room temperature for 24 hours. After the ethanol was distilled off under reduced pressure, the residue was diluted with water and washed with ethyl acetate. To the water layer, 1 N hydrochloric acid (2.2 mL) was added; the mixture was saturated with sodium chloride and extracted with ethyl acetate-tetrahydrofuran (2:1); the extract was washed with saturated brine and dried over magnesium sulfate. After concentration under reduced pressure, the residue was powdered by the addition of ethyl acetate, collected by filtration, washed with ethyl acetate and dried to yield the title compound (0.203 g).
Melting point : 181-185 °C

| Elemental analysis for C₃₀H₃₆N₆O₄·2.5H₂O | | | |
|---|---|---|---|
| Calculated (%) | C, 61.10; | H, 7.01; | N, 14.25 |
| Found (%) | C, 61.31; | H, 7.02; | N, 13.91 |

### Reference Example 19

### Production of pivaloyloxymethyl 2-[6-[3-[4-(diphenylmethoxy)piperidino]propylamino]-3-oxo[1,2,4]triazolo[4,3-b]pyridazin-2(3H)-yl]-2-methylpropionate

Ethyl 2-[6-[3-[4-(diphenylmethoxy)piperidino]propylamino]-3-oxo[1,2,4]triazolo[4,3-b]pyridazin-2(3H)-yl]-2-methylpropionate (0.750 g) was dissolved in ethanol (5 mL); 1 N aqueous solution of sodium hydroxide (3.28 mL) was added, followed by stirring at room temperature for 24 hours. After the ethanol was distilled off under reduced pressure, the residue was diluted with water and washed with ethyl acetate; to the water layer, 1 N hydrochloric acid (3.28 mL) was added; the reaction mixture was extracted with ethyl acetate-tetrahydrofuran (1:1); the extract was washed with saturated brine and dried over magnesium sulfate. After concentration under reduced pressure, the residue was dissolved in N,N-dimethylformamide (5 mL); potassium carbonate (0.273 g) and chloromethyl pivalate (0.285 mL) were added, followed by stirring at room temperature for 15 hours. After ice water was added, the reaction mixture was extracted with ethyl acetate; the extract was washed with brine and dried over magnesium sulfate. After concentration under reduced pressure, the residue was subjected to silica gel column chromatography and eluted with ethyl acetate-methanol-triethylamine (185:15:2). The desired fraction was collected and concentrated; the residue was crystallized from diethyl ether, collected by filtration, and dried, to yield the title compound (0.524 g).
Melting point : 162°C

| Elemental analysis for C₃₆H₄₆N₆O₆ | | | |
|---|---|---|---|
| Calculated (%) | C, 65.63; | H, 7.04; | N, 12.76 |
| Found (%) | C, 65.52; | H, 6.80; | N, 12.86 |

### Reference Example 20

### Production of pivaloyloxymethyl 2-[6-[3-[4-(diphenylmethoxy)piperidino]propoxy]-3-oxo[1,2,4]triazolo[4,3-b]pyridazin-2(3H)-yl]-2-methylpropionate fumarate

Ethyl 2-[6-[3-[4-(diphenylmethoxy)piperidino]propoxy]-3-oxo[1,2,4]triazolo[4,3-b]pyridazin-2(3H)-yl]-2-methylpropionate (0.750 g) was dissolved in ethanol (6 mL); 1 N aqueous solution of sodium hydroxide (3.9 mL) was added, followed by stirring at room temperature for 40 hours. After the ethanol was distilled off under reduced pressure, the residue was diluted with water and washed with ethyl acetate; to the water layer, 1 N hydrochloric acid (3.9 mL) was added; the reaction mixture was extracted with ethyl acetate-tetrahydrofuran (1:1); the extract was washed with saturated brine and dried over magnesium sulfate. After concentration under reduced pressure, the residue was dissolved in N,N-dimethylformamide (5 mL); potassium carbonate (0.324 g) and chloromethyl pivalate (0.339 mL) were added, followed by stirring at room temperature for 18 hours. After ice water was added, the reaction mixture was extracted with ethyl acetate; the extract was washed with brine and dried over magnesium sulfate. After concentration under reduced pressure, the residue was subjected to silica gel column chromatography and eluted with ethyl acetate-methanol (95:5). The desired fraction was collected and concentrated to yield pivaloyloxymethyl 2-[6-[3-[4-(diphenylmethoxy)piperidino]propoxy]-3-oxo[1,2,4]triazolo[4,3-b]pyridazin-2(3H)-yl]-2-methylpropionate (0.50 g) as an oily substance. This oily substance (0.50 g) was dissolved in ethyl acetate (5 mL); a solution of fumaric acid (87 mg) in methanol (3 mL) was added, followed by concentration under reduced pressure. The residue was crystallized from ethyl acetate, collected by filtration, and dried to yield the title compound (0.463 g).
Meltinq point : 160-162°C

| Elemental analysis for C₄₀H₄₉N₅O₁₁·0.5H₂O | | | |
|---|---|---|---|
| Calculated (%) | C, 61.21; | H, 6.42; | N, 8.92 |
| Found (%) | C, 61.37; | H, 6.50; | N, 8.88 |

### Example 1

| | | |
|---|---|---|
| (1) | Compound of Reference Example 1 | 10.0 mg |
| (2) | Lactose | 60.0 mg |
| (3) | Corn starch | 35.0 mg |
| (4) | Gelatin | 3.0 mg |
| (5) | Magnesium stearate | 2.0 mg |

A mixture of the compound obtained in Reference Example 1 (10.0 mg), lactose (60.0 mg) and corn starch (35.0 mg) is granulated through a sieve of 1 mm mesh, using 10% aqueous solution of gelatin (0.03 mL) (containing 3.0 mg of gelatin), after which it is dried at 40°C and again sieved. The resulting granules are mixed with magnesium stearate (2.0 mg), followed by compression. The resulting core tablets are coated with a sugar coat, using an aqueous suspension of sucrose, titanium dioxide, talc and gum arabic. The coated tablets are polished with beeswax to yield finished coated tablets.

### Example 2

| | | |
|---|---|---|
| (1) | Compound of Reference Example 1 | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

The compound obtained in Reference Example 1 (10.0 mg) and magnesium stearate (3.0 mg) are mixed and granulated, using aqueous solution of soluble starch (0.07 mL) (containing 7.0 mg of soluble starch). The resulting granules are dried and mixed with lactose (70.0 mg) and corn starch (50.0 mg), followed by compression, to yield tablets.

### Example 3

| | | |
|---|---|---|
| (1) | Compound of Reference Example 1 | 5.0 mg |
| (2) | Sodium chloride | 20.0 mg |
| (3) | Distilled water was added to reach a total quantity of | 2 mL. |

The compound obtained in Example 1 (5.0 mg) and sodium chloride (20.0 mg) were dissolved in distilled water, and diluted with water to reach a total quantity of 2.0 mL. The resulting solution was filtered and aseptically packed in a 2 mL ampule, which was sterilized and sealed to yield a solution for injection.

### Example 4

| | | |
|---|---|---|
| (1) | Compound of Reference Example 9 | 10.0 mg |
| (2) | Lactose | 60.0 mg |
| (3) | Corn starch | 35.0 mg |
| (4) | Gelatin | 3.0 mg |
| (5) | Magnesium stearate | 2.0 mg |

A mixture of the compound obtained in Reference Example 9 (10.0 mg), lactose (60.0 mg) and corn starch (35.0 mg) is granulated through a sieve of 1 mm mesh, using 10% aqueous solution of gelatin (0.03 mL) (containing 3.0 mg of gelatin), after which it is dried at 40°C and again sieved. The resulting granules are mixed with magnesium stearate (2.0 mg), followed by compression. The resulting core tablets are coated with a sugar coat, using an aqueous suspension of sucrose, titanium dioxide, talc and gum arabic. The coated tablets are polished with beeswax to yield finished coated tablets.

### Example 5

| | | |
|---|---|---|
| (1) | Compound of Reference Example 9 | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

The compound obtained in Reference Example 9 (10.0 mg) and magnesium stearate (3.0 mg) are mixed and granulated, using aqueous solution of soluble starch (0.07 mL) (containing 7.0 mg of soluble starch). The resulting granules are dried and mixed with lactose (70.0 mg) and corn starch (50.0 mg), followed by compression, to yield tablets.

### Example 6

| | | |
|---|---|---|
| (1) | Compound of Reference Example 10 | 10.0 mg |
| (2) | Lactose | 60.0 mg |
| (3) | Corn starch | 35.0 mg |
| (4) | Gelatin | 3.0 mg |
| (5) | Magnesium stearate | 2.0 mg |

A mixture of the compound obtained in Reference Example 10 (10.0 mg), lactose (60.0 mg) and corn starch (35.0 mg) is granulated through a sieve of 1 mm mesh, using 10% aqueous solution of gelatin (0.03 mL) (containing 3.0 mg of gelatin), after which it is dried at 40°C and again sieved. The resulting granules are mixed with magnesium stearate (2.0 mg), followed by compression. The resulting core tablets are coated with a sugar coat, using an aqueous suspension of sucrose, titanium dioxide, talc and gum arabic. The coated tablets are polished with beeswax to yield finished coated tablets.

### Example 7

| | | |
|---|---|---|
| (1) | Compound of Reference Example 10 | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

The compound obtained in Reference Example 10 (10.0 mg) and magnesium stearate (3.0 mg) are mixed and granulated, using aqueous solution of soluble starch (0.07 mL) (containing 7.0 mg of soluble starch). The resulting granules are dried and mixed with lactose (70.0 mg) and corn starch (50.0 mg), followed by compression, to yield tablets.

### Example 8

After uniformly mixing the compound disclosed in Reference Example 9 (1500 g), lactose (2025 g) and corn starch (556.5 g) in a fluidized bed granulator (FD-5 S, POWREX CO.), the mixture was granulated by spraying an aqueous solution of hydroxypropylcellulose (126 g) in the granulator, and then dried in the fluidized bed granulator. The dried granules were milled by using a power mill with a punching screen of 1.5 mm φ to form milled granules. The uniform granules (3927 g) are taken and croscarmellose sodium (210 g) and magnesium stearate (63 g) are added thereto. These were mixed in a Tumbler mixer to provide granules for tabletting. The granules were tabletted by using a tabletting machine equipped with a die 6.5 mm in diameter to provide core tablets each weighing 300 mg. The core tablets were sprayed, in a Doria coater coating machine, with a liquid prepared by dissolving hydroxypropylmethylcellulose 2910 (TC-5) and macrogol 6000 and dispersing titanium oxide and ferric oxide to provide about 13500 film coated tablets of the following formulation containing 100 mg per tablet.

### Tablet formulation:

| | Composition | Amount (mg) |
|---|---|---|
| (1) | Compound of Reference Example 9 | 100.0 |
| (2) | Lactose | 135.0 |
| (3) | Corn starch | 37.1 |
| (4) | Croscarmellose sodium | 15.0 |
| (5) | Hydroxypropylcellulose | 8.4 |
| (6) | Magnesium stearate | 4.5 |
| | Total (Core tablet) | 300.0 |

### Film coated tablet formulation:

| | | |
|---|---|---|
| (1) | Core tablet | 300.0 |
| (Film ingredients) | | |
| (2) | Hydroxypropylmethylcellulose 2910 | 7.485 |
| (3) | Macrogol 6000 | 1.5 |
| (4) | Titanium oxide | 1.0 |
| (5) | Ferric oxide | 0.015 |
| | Total | 310.0 |

### Example 9

About 13500 film coated tablets of the following formulation containing 25 mg of the compound disclosed in Reference Example 9 per tablet was prepared in accordance with the method disclosed in Example 8.

### Tablet formulation:

| | Composition | Amount (mg) |
|---|---|---|
| (1) | Compound of Reference Example 9 | 25.0 |
| (2) | Lactose | 210.0 |
| (3) | Corn starch | 37.1 |
| (4) | Croscarmellose sodium | 15.0 |
| (5) | Hydroxypropylcellulose | 8.4 |
| (6) | Magnesium stearate | 4.5 |
| | Total (Core tablet) | 300.0 |

### Film coated tablet formulation:

| | | |
|---|---|---|
| (1) | Core tablet | 300.0 |
| (Film ingredients) | | |
| (2) | Hydroxypropylmethylcellulose 2910 | 7.485 |
| (3) | Macrogol 6000 | 1.5 |
| (4) | Titanium oxide | 1.0 |
| (5) | Ferric oxide | 0.015 |
| | Total | 310.0 |

### Example 10

About 13500 film coated tablets of the following formulation containing 5 mg of the compound disclosed in Reference Example 9 per tablet was prepared in accordance with the method disclosed in Preparation Example 8.

### Tablet formulation:

| | Composition | Amount (mg) |
|---|---|---|
| (1) | Compound of Example 9 | 5.0 |
| (2) | Lactose | 230.0 |
| (3) | Corn starch | 37.1 |
| (4) | Croscarmellose sodium | 15.0 |
| (5) | Hydroxypropylcellulose | 8.4 |
| (6) | Magnesium stearate | 4.5 |
| | Total (Core tablet) | 300.0 |

### Film coated tablet formulation:

| | | |
|---|---|---|
| (1) | Core tablet | 300.0 |
| (Film ingredients) | | |
| (2) | Hydroxypropylmethylcellulose 2910 | 7.485 |
| (3) | Macrogol 6000 | 1.5 |
| (4) | Titanium oxide | 1.0 |
| (5) | Ferric oxide | 0.015 |
| | Total | 310.0 |

### Preparation Example 11

About 13500 film coated tablets of the following formulation containing 1 mg of the compound disclosed in Reference Example 9 per tablet was prepared in accordance with the method disclosed in Preparation Example 8.

### Tablet formulation:

| | Composition | Amount (mg) |
|---|---|---|
| (1) | Compound of Reference Example 9 | 1.0 |
| (2) | Lactose | 234.0 |
| (3) | Corn starch | 37.1 |
| (4) | Croscarmellose sodium | 15.0 |
| (5) | Hydroxypropylcellulose | 8.4 |
| (6) | Magnesium stearate | 4.5 |
| | Total (Core tablet) | 300.0 |

### Film coated tablet formulation:

| | | |
|---|---|---|
| (1) | Core tablet | 300.0 |
| (Film ingredients) | | |
| (2) | Hydroxypropylmethylcellulose 2910 | 7.485 |
| (3) | Macrogol 6000 | 1.5 |
| (4) | Titanium oxide | 1.0 |
| (5) | Ferric oxide | 0.015 |
| | Total | 310.0 |

### Preparation Example 12

| | |
|---|---|
| White vaseline | 40 g |
| Cetanol | 10 g |
| Bleached bees wax | 5 g |
| Sorbitan sesquioleate | 5 g |
| Lauromocrogold | 0.5 g |
| Methyl para-hydroxybenzoate | 0.1 g |
| Propyl para-hydroxybenzoate | 0.1 g |
| Purified water | adequate amount |

An official absorption ointment (100 g) of the above formulation was heated to 70°C in advance, and to its solution was added a solution prepared by dissolving under heating 1 g of the compound prepared in Reference Example 9 in 20 mL of methanol. After heating and mixing at that temperature for 10 minutes, the remaining methanol was removed and the residue was cooled to room temperature to provide an absorption ointment.

### Experimental Example 1

### Inhibiting Effect on the Increase in the Amount of TNF-α in Tissue of IgE-Passively Sensitized Mouse after Antigen Stimulation

Female BALB/c mice were passively sensitized systemically by intravenous administration of 0.25 ml of a 20-fold diluted anti-DNP monoclonal antibody. On the following day, skin reactions were induced by applying 10 µl of a 0.15% dinitrofluorobenzene solution (DNFB diluted with a 4:1 mixture of acetone and olive oil) to each side of both ears of mice. Eight hours after the application of DNFB, both ears were cut off and homogenized in 400 µl of PBS using Polytron and then centrifuged (15,000 rpm, 15 minutes, 4°C). The resulting supernatant was used as a sample. The amount of TNF-α was determined using a commercially available ELISA kit. The amount of proteins in the sample was also determined, and the amount of TNF-α was calculated per mg of protein. A preliminary test was performed in which an auricle sample was taken at certain intervals after the application of the antigen, and a time course of the amount of TNF-α was observed. The drug was orally administered one hour before the application of DNFB.

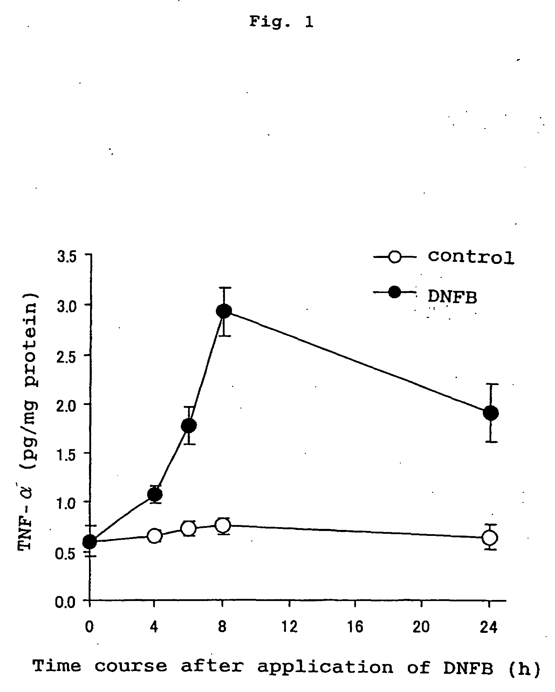

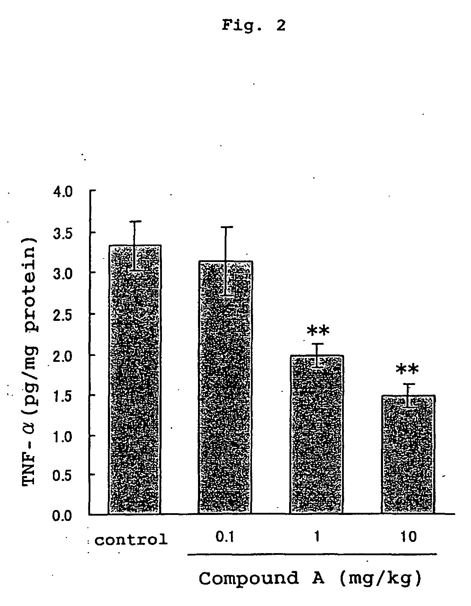

Result: The amount of TNF-α in the ear tissue was found to increase four hours after the application of the antigen and reached the maximum after eight hours (Fig. 1). The amount of TNF-α in the control group was 3.34±0.31 pg/mg protein eight hours after the application of the antigen. The compound of Reference Example 9 (hereinafter abbreviated as Compound A) inhibited the increase in the amount of TNF-α in a dose-dependent manner (0.1 to 10 mg/kg, p.o.), and a dose of 1 mg/kg or more was significantly (Fig. 2).

### Experimental Example 2

### Inhibiting Effect on the Antigen Stimulation-Induced Release of TNF-α from Mast Cells

Female Wistar rats were intraperitoneally administered 2 ml of an anti-DNP-IgE antibody (diluted 250-fold), and killed by exsanguination after 48 hours. 20 ml of a physiological mast cells medium (MCM) was injected into the abdominal cavity, and the lavage was recovered. The lavage was centrifuged at 50xg for seven minutes at 4°C and then washed twice with MCM. Cells were suspended in 1 ml of a heparin-free MCM at a concentration of 1.25x10⁵ cells/mL, and then CaCl₂ was added (at a final concentration of 1 mmol/L). The resulting mixture was incubated with Compound A or DMSO (at a final concentration of 0.1%) at 37°C for 60 minutes, and then DNP-BSA (at a final concentration of 30 ng/mL) was added, and further incubation was performed for two hours. Thereafter, the supernatant was harvested and stored at -40°C until the amount of TNF-α was measured. The amount of TNF-α was determined using a commercially available ELISA kit.

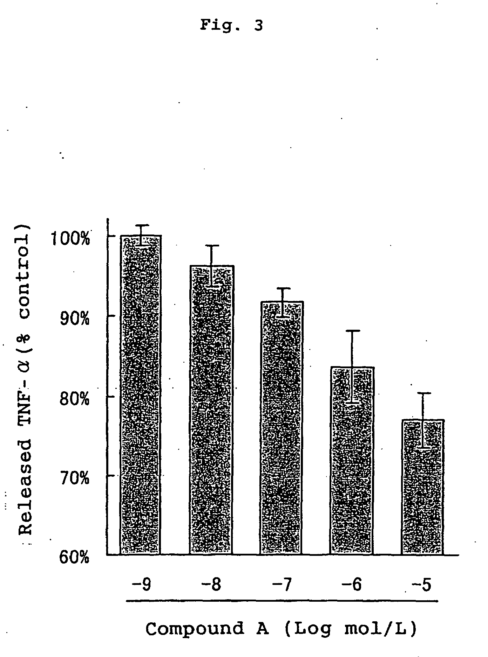

Result: Two hours after the addition of the antigen (DNP-BSA), TNF-α was released at 27.46±1.40 µg/10⁶ cells of peritoneal mast cells from the passively sensitized rat. Compound A (10⁻⁹ to 10⁻⁵ mol/l) inhibited the antigen stimulation-induced release of TNF-α in a concentration-dependent manner (Fig. 3).

### Experimental Example 3

### Influence on Intracellular Signal-Transmitting System in RBL-2H3 Cell

Rat basophilic leukemia 2H3 (RBL-2H3) cells (JCRB0023 from Health Science Research Resources Bank) were seeded in a 6-well plate or a 60 mm dish (0.5x10⁶ cells/ml) and sensitized with the anti-DNP-IgE antibody (1:2000) at 37°C for 90 minutes, and then washed twice with a glucose/PIPES buffer. After the RBL-2H3 cells were incubated at 37°C for 15 minutes in the presence of Compound A or DMSO (at a final concentration of 0.1%), DNP-BSA (at a final concentration of 3 ng/ml) was added, and further incubation was performed for 10 minutes. The cells were then washed twice with PBS on ice, the cells were harvested with a scraper in a cell extraction buffer for western blot analysis or kinase assay, and incubated on ice for 15 minutes, and centrifuged (14,500 rpm, 4°C, 15 minutes). The resulting supernatant was recovered.

Western Blot Analysis: The supernatant of the total protein extracted from the cells was separated by 10% SDS-PAGE. The proteins and phosphorylated proteins were detected by a chemiluminescence technique using an anti-PLCγ-1[pY783] phosphospecific antibody, an anti-PLCγ antibody, an anti-active JNK (pTPpY) antibody, an anti-phospho-MAPK (ERK1/2)(Thr202/Tyr204) antibody, an anti-phospho-p38MAPK (Thr180/Tyr182) antibody, an anti-p44/42MAP kinase antibody, and an anti-JNK antibody.

JNK Kinase Assay: The total protein extracted from the cells (250 µg) was incubated for 12 hours with gentle rocking with GST-c-Jun(1-89) sepharose beads (2 µg) at 4°C. After washing, a kinase buffer was used for replacement. ATP (100 µmol) was added to start the kinase reaction. After incubation was performed at 30°C for 30 minutes, 10% SDS-PAGE was used for separation. The phosphorylated GST-c-Jun(1-89) was detected by a chemiluminescence technique using an anti-phospho-c-Jun antibody.

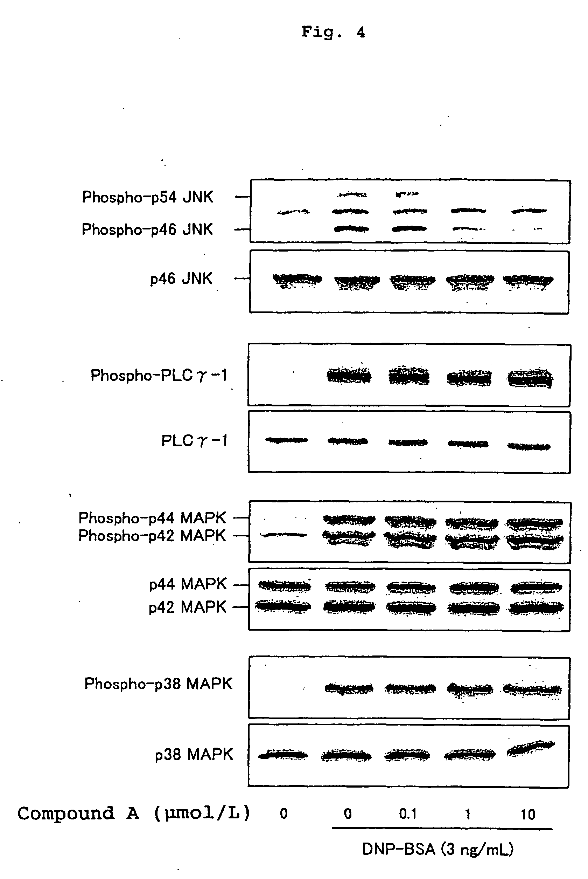

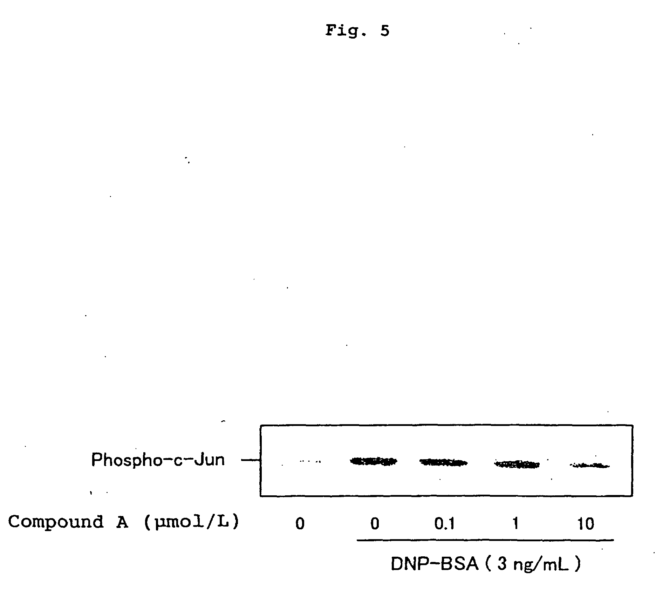

Result: Ten minutes after the antigen stimulation, activated site-specific phosphorylation of JNKs, PLCγ, ERKs, and p38MAPK was observed in the passively sensitized RBL-2H3 cells. Compound A (10⁻⁵ to 10⁻⁷ mol/l) inhibited the specific phosphorylation of JNK (JNK1 and JNK2) in a dose-dependent manner (Fig. 4). However; Compound A had no influence on the activated site-specific phosphorylation of PLCγ, ERKs or p38MAPK. The expression amount of any other protein was not influenced by the antigen stimulation or the treatment with Compound A (Fig. 4). The protein extract, obtained from the cells treated with Compound A using c-Jun(1-89) as a substrate, was examined for the JNK activity. As a result, it was demonstrated that the JNK activity was inhibited depending on the concentration of Compound A (20⁻⁵ to 10⁻⁷ mol/l) (Fig. 5). From the above results, it has been apparent that Compound A selectively inhibits the activation of JNK among various cascades that are activated depending on IgE.

### Industrial Applicability

Compounds (A), (I) and (II) or salt thereof have an excellent JNK activation inhibitory effect, therefore they can be used as a drug for preventing and/or treating JNK derived disease.

## Claims

1. A c-Jun N-terminal kinase activation inhibitor which comprises a compound represented by the formula: 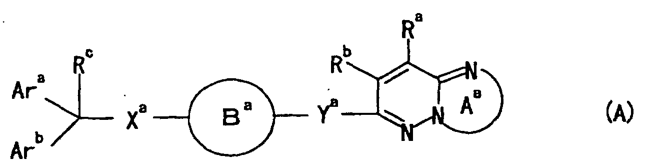 wherein each of Ar^{a} and Ar^{b} is an aromatic group optionally having substituents, Ar^{a} and Ar^{b} optionally form a condensed cyclic group together with the adjacent carbon atom; ring B^{a} is a nitrogen-containing heterocycle optionally having substituents; X^{a} and Y^{a} are the same or different and each is (1) a bond, (2) an oxygen atom, (3) S(O)ₚ (wherein p is an integer of 0 to 2), (4) NR^{d} (wherein R^{d} is a hydrogen atom or a lower alkyl group) or (5) a divalent linear lower hydrocarbon group optionally having substituents and containing 1 to 3 hetero atom(s); ring A^{a} is a 5-membered ring optionally having substituents; R^{a} and R^{b} are the same or different and each is (1) a hydrogen atom, (2) a halogen atom, (3) a hydrocarbon group optionally having substituents, (4) an acyl group or (5) a hydroxy group optionally having a substituent; R^{c} is (1) a hydrogen atom, (2) a hydroxy group optionally substituted by a lower alkyl group or (3) a carboxyl group or a salt thereof, or a prodrug thereof.

2. A TNF-α inhibitor which comprises a compound represented by the formula: 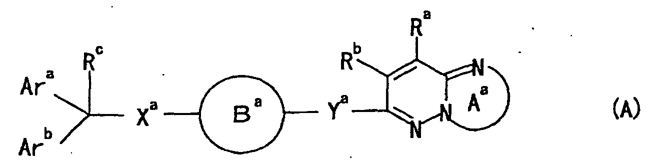 wherein each of Ar^{a} and Ar^{b} is an aromatic group optionally having substituents, Ar^{a} and Ar^{b} optionally form a condensed cyclic group together with the adjacent carbon atom; ring B^{a} is a nitrogen-containing heterocycle optionally having substituents; X^{a} and Y^{a} are the same or different and each is (1) a bond, (2) an oxygen atom, (3) S(O)ₚ (wherein p is an integer of 0 to 2), (4) NR^{d} (wherein R^{d} is a hydrogen atom or a lower alkyl group) or (5) a divalent linear lower hydrocarbon group optionally having substituents and containing 1 to 3 hetero atom(s); ring A^{a} is a 5-membered ring optionally having substituents; R^{a} and R^{b} are the same or different and each is (1) a hydrogen atom, (2) a halogen atom, (3) a hydrocarbon group optionally having substituents, (4) an acyl group or (5) a hydroxy group optionally having a substituent; R^{c} is (1) a hydrogen atom, (2) a hydroxy group optionally substituted by a lower alkyl group or (3) a carboxyl group or a salt thereof, or a prodrug thereof.

3. A c-Jun N-terminal kinase activation inhibitor which comprises a compound represented by the formula: 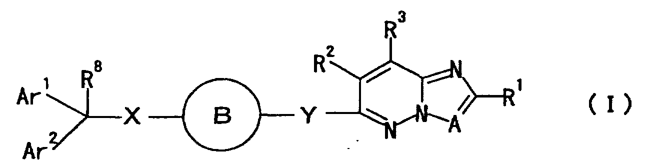 wherein each of Ar¹ and Ar² is an aromatic group optionally having substituents, Ar¹ and Ar² optionally form a condensed cyclic group together with the adjacent carbon atom; ring B is a nitrogen-containing heterocycle optionally having substituents; X and Y are the same or different and each is (1) a bond, (2) an oxygen atom, (3) S(O)ₚ (wherein p is an integer of 0 to 2), (4) NR⁴ (wherein R⁴ is a hydrogen atom or a lower alkyl group) or (5) a divalent linear lower hydrocarbon group optionally having substituents and containing 1 to 3 hetero atom(s); A is (1) a nitrogen atom or (2) CR⁷ (wherein R⁷ is a hydrogen atom, a halogen atom, a hydrocarbon group optionally having substituents, an acyl group or a hydroxy group optionally having a substituent); R¹, R² and R³ are the' same or different and each is (1) a hydrogen atom, (2) a halogen atom, (3) a hydrocarbon group optionally having substituents, (4) an acyl group or (5) a hydroxy group optionally having a substituent, R⁸ is (1) a hydrogen atom, (2) a hydroxy group optionally substituted by a lower alkyl group or (3) a carboxyl group or a salt thereof, or a prodrug thereof.

4. A TNF-α inhibitor which comprises a compound represented by the formula: 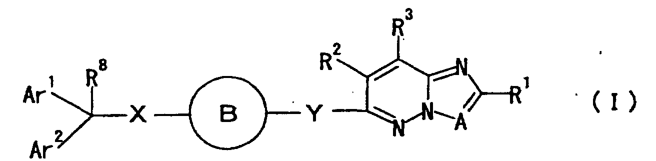 wherein each of Ar¹ and Ar² is an aromatic group optionally having substituents, Ar¹ and Ar² optionally form a condensed cyclic group together with the adjacent carbon atom; ring B is a nitrogen-containing heterocycle optionally having substituents; X and Y are the same or different and each is (1) a bond, (2) an oxygen atom, (3) S(O)ₚ (wherein p is an integer of 0 to 2) , (4) NR⁴ (wherein R⁴ is a hydrogen atom or a lower alkyl group) or (5) a divalent linear lower hydrocarbon group optionally having substituents and containing 1 to 3 hetero atom(s); A is (1) a nitrogen atom or (2) CR⁷ (wherein R⁷ is a hydrogen atom, a halogen atom, a hydrocarbon group optionally having substituents, an acyl group or a hydroxy group optionally having a substituent); R¹, R² and R³ are the same or different and each is (1) a hydrogen atom, (2) a halogen atom, (3) a hydrocarbon group optionally having substituents, (4) an acyl group or (5) a hydroxy group optionally having a substituent, R⁸ is (1) a hydrogen atom, (2) a hydroxy group optionally substituted by a lower alkyl group or (3) a carboxyl group or a salt thereof, or a prodrug thereof.

5. The inhibitor according to claim 3 or 4, wherein each of Ar¹ and Ar² is (1) a C₆₋₁₄ aromatic hydrocarbon group, (2) a 5 to 8 membered aromatic heterocyclic group containing 1 to 4 hetero atoms selected from a nitrogen atom, a sulfur atom and an oxygen atom other than carbon atoms or (3) (3) a monovalent group obtained by removing one optional hydrogen atom from a ring formed by condensation of said aromatic heterocyclic ring with the C₆₋₁₄ aromatic hydrocarbon ring, wherein the C₆₋₁₄ aromatic hydrocarbon group, the 5 to 8 membered aromatic heterocyclic group and the monovalent group may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy and (xxviii) C₇₋₁₆ aralkyloxy; and Ar¹ and Ar² may form a condensed cyclic group with the adjacent carbon atom represented by the formula: 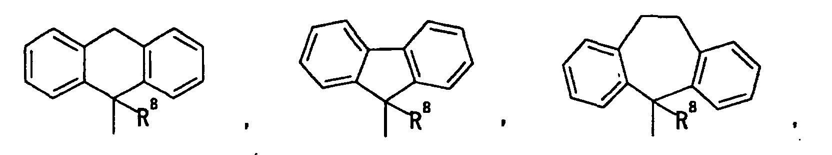 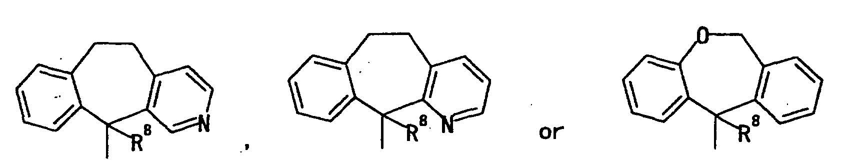 wherein R⁸ is a hydrogen atom, a hydroxy group which may be substituted by C₁₋₆ alkyl or a carboxyl group, and the condensed cyclic group may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo;
the ring B is a 3 to 13 membered nitrogen-containing heterocycle containing at least one nitrogen atom which may contain 1 to 3 hetero atoms selected from a nitrogen atom, an oxygen atom and a sulfur atom, and the 3 to 13 membered nitrogen-containing heterocycle may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo;
X and Y are same or different (1) a bond, (2) an oxygen atom, (3) S(O)ₚ wherein p is an integer of 0 to 2, (4) NR⁴ wherein R⁴ is a hydrogen atom or a linear or branched C₁₋₆ alkyl group or (5) a bivalent linear C₁₋₆ hydrocarbon group which may contain 1 to 3 hetero atoms selected from an oxygen atom, NR^{4'} (wherein R⁴' is a hydrogen atom or or a linear or branched C₁₋₆ alkyl group) and a sulfur atom, and the bivalent linear C₁₋₆ hydrocarbon group may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo;
A is a nitrogen atom or CR⁷ wherein R⁷ is
(1) a hydrogen atom,
(2) a halogen atom,
(3) a C₁₋₆ alkyl group, a C₂₋₆ alkenyl group, a C₂₋₆ alkynyl group, a C₃₋₆ cycloalkyl group, a condensed group formed by a C₃₋₆ cycloalkyl group and a benzene ring optionally having 1 to 3 C₁₋₆ alkoxy, a C₆₋₁₄ aryl group or a C₇₋₁₆ aralkyl group, which may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo,
(4) an acyl group represented by the formula: - (C=O)-R⁹, -SO₂-R⁹, -SO-R⁹, -(C=O)NR¹⁰R⁹, -(C=O)O-R⁹ , -(C=S)O-R⁹ or -(C=S)NR¹⁰R⁹ wherein R⁹ is (a) a hydrogen atom, (b) a C₁₋₆ alkyl group, a C₂₋₆ alkenyl group, a C₂₋₆ alkynyl group, a C₃₋₆ cycloalkyl group, a condensed group formed by a C₃₋₆ cycloalkyl group and a benzene ring optionally having 1 to 3 C₁₋₆ alkoxy, a C₆₋₁₄ aryl group or a C₇₋₁₆ aralkyl group, which may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo or (c) -OR¹¹ wherein R¹¹ is a hydrogen atom or a C₁₋₆ alkyl group, a C₂₋₆ alkenyl group, a C₂₋₆ alkynyl group, a C₃₋₆ cycloalkyl group, a condensed group formed by a C₃₋₆ cycloalkyl group and a benzene ring optionally having 1 to 3 C₁₋₆ alkoxy, a C₆₋₁₄ aryl group or a C₇₋₁₆ aralkyl group, which may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo, R¹⁰ is a hydrogen atom or a C₁₋₆ alkyl group, or
(5) -OR¹² wherein R¹² is a hydrogen atom, or a C₁₋₆ alkyl group, a C₂₋₆ alkenyl group, a C₂₋₆ alkynyl group, a C₃₋₆ cycloalkyl group, a condensed group formed by a C₃₋₆ cycloalkyl group and a benzene ring optionally having 1 to 3 C₁₋₆ alkoxy, a C₆₋₁₄ aryl group or a C₇₋₁₆ aralkyl group, which may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo;
R¹, R² and R³ are the same or different and are independently
(1) a hydrogen atom,
(2) a halogen atom,
(3) a C₁₋₆ alkyl group, a C₂₋₆ alkenyl group, a C₂₋₆ alkynyl group, a C₃₋₆ cycloalkyl group, a condensed group formed by a C₃₋₆ cycloalkyl group and a benzene ring optionally having 1 to 3 C₁₋₆ alkoxy, a C₆₋₁₄ aryl group or a C₇₋₁₆ aralkyl group, which may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo,
(4) an acyl group represented by the formula: -(C=O)-R¹³, -SO₂-R¹³, -SO-R¹³, -(C=O)NR¹⁴R¹³, -(C=O)O-R¹³, -(C=S)O-R¹³ or -(C=S)NR¹⁴R¹³ wherein R¹³ is (a) a hydrogen atom, (b) a C₁₋₆ alkyl group, a C₂₋₆ alkenyl group, a C₂₋₆ alkynyl group, a C₃₋₆ cycloalkyl group, a condensed group formed by a C₃₋₆ cycloalkyl group and a benzene ring optionally having 1 to 3 C₁₋₆ alkoxy, a C₆₋₁₄ aryl group or a C₇₋₁₆ aralkyl group, which may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo or (c) -OR¹⁵ wherein R¹⁵ is a hydrogen atom, or a C₁₋₆ alkyl group, a C₂₋₆ alkenyl group, a C₂₋₆ alkynyl group, a C₃₋₆ cycloalkyl group, a condensed group formed by a C₃₋₆ cycloalkyl group and a benzene ring optionally having 1 to 3 C₁₋₆ alkoxy, a C₆₋₁₄ aryl group or a C₇₋₁₆ aralkyl group, which may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo, R¹⁴ is a hydrogen atom or a C₁₋₆ alkyl group; or (5) -OR¹⁶ wherein R¹⁶ is a hydrogen atom, or a C₁₋₆ alkyl group, a C₂₋₆ alkenyl group, a C₂₋₆ alkynyl group, a C₃₋₆ cycloalkyl group, a condensed group formed by a C₃₋₆ cycloalkyl group and a benzene ring optionally having 1 to 3 C₁₋₆ alkoxy, a C₆₋₁₄ aryl group or a C₇₋₁₆ aralkyl group, which may be substituted by a group selected from the group consisting of (i) a halogen atom, (ii) C₁₋₆ alkylenedioxy, (iii) nitro, (iv) cyano, (v) optionally halogenated C₁₋₆ alkyl, (vi) optionally halogenated C₂₋₆ alkenyl, (vii) optionally halogenated C₂₋₆ alkynyl, (viii) C₃₋₆ cycloalkyl, (ix) C₁₋₆ alkoxy optionally having 1 to 3 halogen atoms, mono- or di-C₁₋₆ alkylamino or C₁₋₆ alkoxy-carbonyl, (x) optionally halogenated C₁₋₆ alkylthio, (xi) hydroxy, (xii) amino, (xiii) mono-C₁₋₆ alkylamino, (xiv) di-C₁₋₆ alkylamino, (xv) 5 or 6 membered cyclic amino, (xvi) C₁₋₆ alkyl-carbonyl, (xvii) carboxyl, (xviii) C₁₋₆ alkoxy-carbonyl, (xix) carbamoyl, (xx) thiocarbamoyl, (xxi) mono-C₁₋₆ alkyl-carbamoyl, (xxii) di-C₁₋₆ alkyl-carbamoyl, (xxiii) C₆₋₁₀ aryl-carbamoyl, (xxiv) sulfo, (xxv) C₁₋₆ alkylsulfonyl, (xxvi) C₆₋₁₀ aryl, (xxvii) C₆₋₁₀ aryloxy, (xxviii) C₇₋₁₆ aralkyloxy and (xxix) oxo;
R⁸ is a hydrogen atom, a hydroxy group which may be substituted by C₁₋₆ alkyl or a carboxyl group.

6. The inhibitor according to claim 3 or 4, wherein Ar¹ and Ar² are a phenyl group; the ring B is a ring represented by the formula: 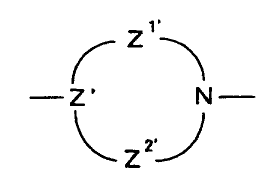 wherein Z' is a methine group; Z^{1'} and Z^{2'} are a methylene group or an ethylene group; X is a bond or an oxygen atom; Y is -(CH₂)ₚ₁NH- wherein ₚ₁ is an integer of 1 to 6; A is CR^{7'} wherein R^{7'} is a hydrogen atom or a C₁₋₆ alkyl group; R¹ is (1) a hydrogen atom, (2) a C₁₋₆ alkyl group which may be substituted by carboxyl or C₁₋₆ alkoxy-carbonyl or (3) a carbamoyl group which may be substituted by a C₁₋₆ alkyl group optionally having C₁₋₆ alkoxy-carbonyl; R² is a hydrogen atom; R³ is a hydrogen atom; R^{B} is a hydrogen atom.

7. The inhibitor according to claim 3 or 4, wherein the compound is 2-[6-[3-[4-(diphenylmethoxy)piperidino]propylamino]imidazo[1,2-b]pyridazin-2-yl]-2-methylpropionic acid or a salt thereof.

8. The inhibitor according to claim 3 or 4, wherein the compound is 2-[6-[3-[4-(diphenylmethoxy)piperidino]propylamino]imidazo[1,2-b]pyridazin-2-yl]-2-methylpropionic acid dihydrate.

9. The inhibitor according to claim 1, which is an agent for preventing and/or treating c-Jun derived disease.

10. The inhibitor according to claim 1, which is an agent for preventing and/or treating c-Jun N-terminal kinase derived disease.

11. The inhibitor according to claim 1, which is an agent for preventing and/or treating acute pancreatitis, chronic pancreatitis, adult dyspnea syndrome, pachyderma, lupus erythematosus profundus, chronic thyroid gland, Graves' disease, autoimmune gastritis, autoimmune neutropenia, thrombocytopenia, myasthenia gravis, multiple myeloma, acute myeloblastic leukemia, chronic sarcoma, chronic myelocytic leukemia, metastatic melanoma, Kaposi's sarcoma, marasmic disease, Huntington's chorea, disease derived from ischemia and/or reperfusion of cerebral apoplexy, myocardial ischemia, ischemic heart disease, kidney ischemia, neovascular glaucoma, infantile angiosarcoma, vascularization, hypercardia, abnormal immune response, fever, cellular aging or apoptosis derived disease.

12. The inhibitor according to claim 1, which is an agent for improving snuff.

13. A method for inhibiting c-Jun N-terminal kinase activation, which comprises administering an effective amount of a compound represented by the formula: 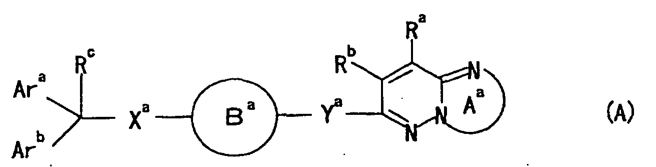 wherein each of Ar^{a} and Ar^{b} is an aromatic group optionally having substituents, Ar^{a} and Ar^{b} optionally form a condensed cyclic group together with the adjacent carbon atom; ring B^{a} is a nitrogen-containing heterocycle optionally having substituents; X^{a} and Y^{a} are the same or different and each is (1) a bond, (2) an oxygen atom, (3) S(O)ₚ (wherein p is an integer of 0 to 2) , (4) NR^{d} (wherein R^{d} is a hydrogen atom or a lower alkyl group) or (5) a divalent linear lower hydrocarbon group optionally having substituents and containing 1 to 3 hetero atom(s); ring A^{a} is a 5-membered ring optionally having substituents; R^{a} and R^{b} are the same or different and each is (1) a hydrogen atom, (2) a halogen atom, (3) a hydrocarbon group optionally having substituents, (4) an acyl group or (5) a hydroxy group optionally having a substituent; R^{c} is (1) a hydrogen atom, (2) a hydroxy group optionally substituted by a lower alkyl group or (3) a carboxyl group or a salt thereof, or a prodrug thereof to a mammal.

14. A method for preventing and/or treating acute pancreatitis, chronic pancreatitis, adult dyspnea syndrome, pachyderma, lupus erythematosus profundus, chronic thyroid gland, Graves' disease, autoimmune gastritis, autoimmune neutropenia, thrombocytopenia, myasthenia gravis, multiple myeloma, acute myeloblastic leukemia, chronic sarcoma, chronic myelocytic leukemia, metastatic melanoma, Kaposi's sarcoma, marasmic disease, Huntington's chorea, disease derived from ischemia and/or reperfusion of cerebral apoplexy, myocardial ischemia, ischemic heart disease, kidney ischemia, neovascular glaucoma, infantile angiosarcoma, vascularization, hypercardia, abnormal immune response, fever, cellular aging or apoptosis derived disease and improving snuff, which comprises administering an effective amount of a compound represented by the formula: 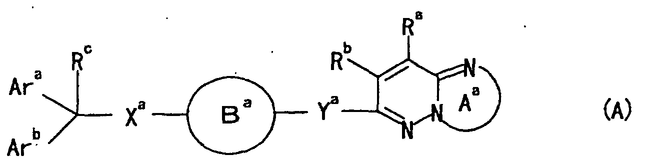 wherein each of Ar^{a} and Ar^{b} is an aromatic group optionally having substituents, Ar^{a} and Ar^{b} optionally form a condensed cyclic group together with the adjacent carbon atom; ring B^{a} is a nitrogen-containing heterocycle optionally having substituents; X^{a} and Y^{a} are the same or different and each is (1) a bond, (2) an oxygen atom, (3) S(O)ₚ (wherein p is an integer of 0 to 2) , (4) NR^{d} (wherein R^{d} is a hydrogen atom or a lower alkyl group) or (5) a divalent linear lower hydrocarbon group optionally having substituents and containing 1 to 3 hetero atom(s); ring A^{a} is a 5-membered ring optionally having substituents; R^{a} and R^{b} are the same or different and each is (1) a hydrogen atom, (2) a halogen atom, (3) a hydrocarbon group optionally having substituents, (4) an acyl group or (5) a hydroxy group optionally having a substituent; R^{c} is (1) a hydrogen atom, (2) a hydroxy group optionally substituted by a lower alkyl group or (3) a carboxyl group or a salt thereof, or a prodrug thereof to a mammal.

15. Use of a compound represented by the formula: 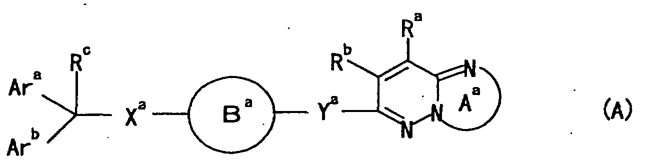 wherein each of Ar^{a} and Ar^{b} is an aromatic group optionally having substituents, Ar^{a} and Ar^{b} optionally form a condensed cyclic group together with the adjacent carbon atom; ring B^{a} is a nitrogen-containing heterocycle optionally having substituents; X^{a} and Y^{a} are the same or different and each is (1) a bond, (2) an oxygen atom, (3) S(O)ₚ (wherein p is an integer of 0 to 2) , (4) NR^{d} (wherein R^{d} is a hydrogen atom or a lower alkyl group) or (5) a divalent linear lower hydrocarbon group optionally having substituents and containing 1 to 3 hetero atom(s); ring A^{a} is a 5-membered ring optionally having substituents; R^{a} and R^{b} are the same or different and each is (1) a hydrogen atom, (2) a halogen atom, (3) a hydrocarbon group optionally having substituents, (4) an acyl group or (5) a hydroxy group optionally having a substituent; R^{c} is (1) a hydrogen atom, (2) a hydroxy group optionally substituted by a lower alkyl group or (3) a carboxyl group or a salt thereof, or a prodrug thereof for producing a c-Jun N-terminal kinase activation inhibitor.

16. Use of a compound represented by the formula: 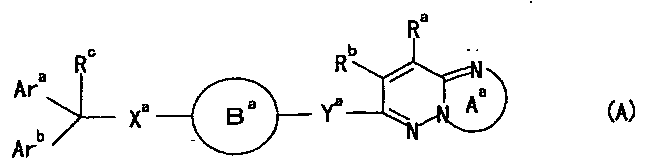 wherein each of Ar^{a} and Ar^{b} is an aromatic group optionally having substituents, Ar^{a} and Ar^{b} optionally form a condensed cyclic group together with the adjacent carbon atom; ring B^{a} is a nitrogen-containing heterocycle optionally having substituents; X^{a} and Y^{a} are the same or different and each is (1) a bond, (2) an oxygen atom, (3) S(O)ₚ (wherein p is an integer of 0 to 2), (4) NR^{d} (wherein R^{d} is a hydrogen atom or a lower alkyl group) or (5) a divalent linear lower hydrocarbon group optionally having substituents and containing 1 to 3 hetero atom(s); ring A^{a} is a 5-membered ring optionally having substituents; R^{a} and R^{b} are the same or different and each is (1) a hydrogen atom, (2) a halogen atom, (3) a hydrocarbon group optionally having substituents, (4) an acyl group or (5) a hydroxy group optionally having a substituent; R^{c} is (1) a hydrogen atom, (2) a hydroxy group optionally substituted by a lower alkyl group or (3) a carboxyl group or a salt thereof, or a prodrug thereof for producing an agent for preventing and/or treating acute pancreatitis, chronic pancreatitis, adult dyspnea syndrome, pachyderma, lupus erythematosus profundus, chronic thyroid gland, Graves' disease, autoimmune gastritis, autoimmune neutropenia, thrombocytopenia, myasthenia gravis, multiple myeloma, acute myeloblastic leukemia, chronic sarcoma, chronic myelocytic leukemia, metastatic melanoma, Kaposi's sarcoma, marasmic disease, Huntington's chorea, disease derived from ischemia and/or reperfusion of cerebral apoplexy, myocardial ischemia, ischemic heart disease, kidney ischemia, neovascular glaucoma, infantile angiosarcoma, vascularization, hypercardia, abnormal immune response, fever, cellular aging or apoptosis derived disease and improving snuff.

17. A c-Jun N-terminal kinase activation inhibitor, which comprises a compound having (1) anti-histamine activity and/or eosinophile chemotaxis inhibiting activity and (2) c-Jun N-terminal kinase activation inhibiting activity or a prodrug thereof.

18. A TNF-α inhibitor, which comprises a compound having (1) anti-histamine activity and/or eosinophile chemotaxis inhibiting activity and (2) c-Jun N-terminal kinase activation inhibiting activity or a prodrug thereof.

19. An agent for preventing and/or treating acute pancreatitis, chronic pancreatitis, adult dyspnea syndrome, pachyderma, lupus erythematosus profundus, chronic thyroid gland, Graves' disease, autoimmune gastritis, autoimmune neutropenia, thrombocytopenia, myasthenia gravis, multiple myeloma, acute myeloblastic leukemia, chronic sarcoma, chronic myelocytic 'leukemia, metastatic melanoma, Kaposi's sarcoma, marasmic disease, Huntington's chorea, disease derived from ischemia and/or reperfusion of cerebral apoplexy, myocardial ischemia, ischemic heart disease, kidney ischemia, neovascular glaucoma, infantile angiosarcoma, vascularization, hypercardia, abnormal immune response, fever, cellular aging or apoptosis derived disease and improving snuff, which comprises a compound having (1) anti-histamine activity and/or eosinophile chemotaxis inhibiting activity and (2) c-Jun N-terminal kinase activation inhibiting activity or a prodrug thereof.

20. An agent for preventing and/or treating acute pancreatitis, chronic pancreatitis, adult dyspnea syndrome, pachyderma, lupus erythematosus profundus, chronic thyroid gland, Graves' disease, autoimmune gastritis, autoimmune neutropenia, thrombocytopenia, myasthenia gravis, multiple myeloma, acute myeloblastic leukemia, chronic sarcoma, chronic myelocytic leukemia, metastatic melanoma, Kaposi's sarcoma, marasmic disease, Huntington's chorea, disease derived from ischemia and/or reperfusion of cerebral apoplexy, myocardial ischemia, ischemic heart disease, kidney ischemia, neovascular glaucoma, infantile angiosarcoma, vascularization, hypercardia, abnormal immune response, fever, cellular aging or apoptosis derived disease and improving snuff, which comprises a compound having (1) anti-histamine activity and/or eosinophile chemotaxis inhibiting activity, (2) c-Jun N-terminal kinase activation inhibiting activity and (3) TNF-α inhibiting activity or a prodrug thereof.

21. A method for inhibiting c-Jun N-terminal kinase activation, which comprises administering an effective amount of a compound having (1) anti-histamine activity and/or eosinophile chemotaxis inhibiting activity and (2) c-Jun N-terminal kinase activation inhibiting activity or a prodrug thereof to a mammal.

22. A method for preventing and/or treating acute pancreatitis, chronic pancreatitis, adult dyspnea syndrome, pachyderma, lupus erythematosus profundus, chronic thyroid gland, Graves' disease, autoimmune gastritis, autoimmune neutropenia, thrombocytopenia, myasthenia gravis, multiple myeloma, acute myeloblastic leukemia, chronic sarcoma, chronic myelocytic leukemia, metastatic melanoma, Kaposi's sarcoma, marasmic disease, Huntington's chorea, disease derived from ischemia and/or reperfusion of cerebral apoplexy, myocardial ischemia, ischemic heart disease, kidney ischemia, neovascular glaucoma, infantile angiosarcoma, vascularization, hypercardia, abnormal immune response, fever, cellular aging or apoptosis derived disease and improving snuff, which comprises administering an effective amount of a compound having (1) anti-histamine activity and/or eosinophile chemotaxis inhibiting activity and (2) c-Jun N-terminal kinase activation inhibiting activity or a prodrug thereof to a mammal.

23. Use of a compound having (1) anti-histamine activity and/or eosinophile chemotaxis inhibiting activity and (2) c-Jun N-terminal kinase activation inhibiting activity or a prodrug thereof for producing an agent for c-Jun N-terminal kinase activation inhibitor.

24. Use of a compound having (1) anti-histamine activity and/or eosinophile chemotaxis inhibiting activity and (2) c-Jun N-terminal kinase activation inhibiting activity or a prodrug thereof for producing an agent for preventing and/or treating acute pancreatitis, chronic pancreatitis, adult dyspnea syndrome, pachyderma, lupus erythematosus profundus, chronic thyroid gland, Graves' disease, autoimmune gastritis, autoimmune neutropenia, thrombocytopenia, myasthenia gravis, multiple myeloma, acute myeloblastic leukemia, chronic sarcoma, chronic myelocytic leukemia, metastatic melanoma, Kaposi's sarcoma, marasmic disease, Huntington's chorea, disease derived from ischemia and/or reperfusion of cerebral apoplexy, myocardial ischemia, ischemic heart disease, kidney ischemia, neovascular glaucoma, infantile angiosarcoma, vascularization, hypercardia, abnormal immune response, fever, cellular aging or apoptosis derived disease and improving snuff.

25. A pharmaceutical, which comprises combining anti-histamic agent and c-Jun N-terminal kinase activation inhibitor and/or TNF-α inhibitor.

26. An anti-allergy pharmaceutical, which comprises combining anti-histamic agent and c-Jun N-terminal kinase activation inhibitor and/or TNF-α inhibitor.

27. A pharmaceutical for preventing and/or treating chronic urticaria, atopic dermatitis, allergic dermatitis, allergic conjunctivitis, hypersensitivity pneumonitis, eczema, dermatitis herpetiformis, psoriasis, eosinophilic pneumonia (PIE syndrome), chronic obstructive pulmonary disease (COPD) or asthma and improving snuff, which comprises combining anti-histamic agent and c-Jun N-terminal kinase activation inhibitor.

28. A method for preventing and/or treating allergy, which comprises administering the combination of an effective amount of anti-histamic agent and an effective amount of c-Jun N-terminal kinase activation inhibitor to a mammal.

29. A method for preventing and/or treating chronic urticaria, atopic dermatitis, allergic dermatitis, allergic conjunctivitis, hypersensitivity pneumonitis, eczema, dermatitis herpetiformis, psoriasis, eosinophilic pneumonia (PIE syndrome), chronic obstructive pulmonary disease (COPD) or asthma and improving snuff, which comprising administering the combination of an effective amount of anti-histamic agent and an effective amount of c-Jun N-terminal kinase activation inhibitor to a mammal.

30. Use of anti-histamic agent and c-Jun N-terminal kinase activation inhibitor for producing anti-allergy agent.

31. Use of anti-histamic agent and c-Jun N-terminal kinase activation inhibitor for producing an agent for preventing and/or treating chronic urticaria, atopic dermatitis, allergic dermatitis, allergic conjunctivitis, hypersensitivity pneumonitis, eczema, dermatitis herpetiformis, psoriasis, eosinophilic pneumonia (PIE syndrome), chronic obstructive pulmonary disease (COPD) or asthma and improving snuff.
